# EUROPEAN PATENT SPECIFICATION

(11) **EP 1 835 907 B1**
(45) Date of publication and mention of the grant of the patent: **22.04.2009**
(21) Application number: 05823903.9
(22) Date of filing: 21.12.2005
(51) Int. Cl.: A61K 31/404, A61P 43/00

(54) **MEDICAMENTS FOR THE TREATMENT OR PREVENTION OF FIBROTIC DISEASES**
MEDIKAMENTE ZUR BEHANDLUNG ODER PRÄVENTION FIBROTISCHER KRANKHEITEN
MEDICAMENTS DESTINES AU TRAITEMENT OU A LA PREVENTION DE MALADIES FIBROTIQUES

(30) Priority: 24.12.2004 EP 04030769
(43) Date of publication of application: 26.09.2007
(73) Proprietor: Boehringer Ingelheim International GmbH, 55216 Ingelheim am Rhein (DE); Boehringer Ingelheim Pharma GmbH & Co.KG, 55216 Ingelheim am Rhein (DE)
(72) Inventor: PARK, John, Edward, 88447 Warthausen (DE); CHAUDHARY, Nveed, 88400 Biberach (DE); ROTH, Gerald, Jürgen, 88400 Biberach (DE); HECKEL, Armin, 88400 Biberach (DE); LEHMANN-LINTZ, Thorsten, 88416 Ochsenhausen (DE); KLEY, Jörg, 88441 Mittelbiberach (DE)
(74) Representative: Hammann, Heinz
(86) International application number: PCT/EP2005/057013
(87) International publication number: WO 2006/067175

(56) References cited:
- WO-A-20/04009547

## Description

The present invention relates to a new use of indolinones of general formula 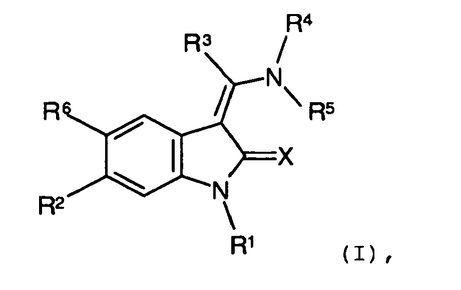 substituted in the 6 position, the tautomers, the diastereomers, the enantiomers, the mixtures thereof and the salts thereof, particularly the physiologically acceptable salts thereof.

### BACKGROUND

Compounds of the above general formula I, the tautomers, the diastereomers, the enantiomers, the mixtures thereof and the salts thereof, particularly the physiologically acceptable salts thereof, have been described in WO 04/009547 as having valuable pharmacological properties, in particular an inhibiting effect on various kinases, especially receptor tyrosine kinases such as VEGFR1, VEGFR2, VEGFR3, PDGFRα, PDGFRβ, FGFR1, FGFR3, EGFR, HER2, c-Kit, IGF1R and HGFR, Flt-3, and on the proliferation of cultivated human cells, in particular endothelial cells, e.g. in angiogenesis, but also on the proliferation of other cells, in particular tumour cells.

However, none of these compounds have been described for their use in the treatment or prevention of the fibrotic diseases referred to in the present invention.

Remodeling is a normal response to tissue injury and inflammation that is observed in many tissues throughout the body. After resolution of the inflammation and repair of tissue damage, the tissue is generally returned to its original condition. Excessive uncontrolled tissue repair or the failure to stop remodeling when it is no longer required leads to condition known as fibrosis. Fibrosis is characterized by excessive deposition of extracellular matrix components and overgrowth of fibroblasts. Fibrosis can occur in all tissues but is especially prevalent in organs with frequent exposure to chemical and biological insults including the lung, skin, digestive tract, kidney, and liver (Eddy, 1996, J Am Soc Nephrol, 7(12):2495-503; Dacic et al., 2003, Am J Respir Cell Mol Biol, 29S: S5-9; Wynn, 2004, Nat Rev Immunol, 4(8):583-94). Fibrosis often severely compromises the normal function(s) of the organ and many fibrotic diseases are, in fact, life-threatening or severely disfiguring, such as idiopathic pulmonary fibrosis (IPF), liver cirrhosis, scleroderma, or renal fibrosis. Treatment options for these diseases are often limited to organ transplantation, a risky and expensive procedure.

A large body of literature implicates the platelet-derived growth factor (PDGF), fibroblast growth factor (FGF), vascular endothelial growth factor (VEGF), epidermal growth factor (EGF), and transforming growth factor beta (TGFb) growth factor families in the induction or persistence of fibrosis (Levitzki, Cytokine Growth Factor Rev, 2004, 15(4):229-35; Strutz et al., Kidney Intl, 2000, 57:1521-38; Strutz et al., 2003, Springer Semin Immunopathol, 24:459-76; Rice et al., 1999, Amer J Pathol, 155 (1) :213-221; Broekelmann et al., 1991, Proc Nat Acad Sci, 88:6642-6; Wynn, 2004, Nat Rev Immunol, 4(8):583-94).

PDGF, EGF and FGF family members are potent mitogens for mesenchymal cells such as smooth muscle cells, myofibroblasts and fibroblasts (Benito et al., 1993, Growth Regul 3 (3) :172-9; Simm et al, 1998, Basic Res Cardiol, 93(S3) :40-3; Klagsburn, Prog Growth Factor Res, 1989, 1(4):207-35; Kirkland et al., 1998, J Am Soc Nephrol, 9(8) :1464-73), the very cells which supplant normal tissue in fibrosis and are believed to play a role in tissue remodeling (Abboud, 1995, Annu Rev Physiol., 57-:297-309; Jinnin et al., 2004, J Cell Physiol, online; Martinet et al., 1996, Arch Toxicol 18:127-39; Desmouliere, Cell Biology International, 1995, 19:471-6; Jelaska et al., Springer Semin Immunopathol, 2000, 21:385-95).

Inhibition of PDGF attenuates both liver fibrosis and lung fibrosis in experimental models, suggesting fibrosis in different organs may have a common origin (Borkham-Kamphorst et al., 2004, Biochem Biophys Res Commun; Rice et al., 1999, Amer J Pathol, 155(1):213-221). An EGF receptor kinase inhibitor was also active in this lung fibrosis model. Three-fold overexpression of an EGF family member, HB-EGF, in mouse pancreas islets was sufficient to cause development of fibrosis in both the exocrine and endocrine compartments (Means et al., 2003, Gastroenterology, 124 (4) :1020-36).

Similarly, FGF1/FGF2-deficient mice show dramatically decreased liver fibrosis after chronic carbon tetrachloride (CCl4) exposure (Yu et al., 2003, Am J Pathol, 163(4):1653-62). FGF expression is increased in human renal interstitial fibrosis where it strongly correlates with interstitial scarring (Strutz et al., 2000, Kidney Intl, 57:1521-38) as well as in a model of experimental lung fibrosis (Barrios et al., 1997, Am J Physiol, 273 (2 Pt 1):L451-8), again lending credence to the idea that fibrosis in various tissues has a common basis.

In addition, elevated levels of VEGF have been observed in several studies in persons with asthma (Hoshino et al., 2001, J Allergy Clin Immunol 107:1034-39; Hoshino et al., 2001, J Allergy Clin Immunol 107:295-301; Kanazawa et al., 2002, Thorax 57:885-8; Asai et al., J Allergy Clin Immunol 110:571-5, 2002; Kanazawa et al., 2004, Am J Respir Crit Care Med, 169:1125-30). Inducible expression of VEGF in a transgenic mouse model induces an asthma-like phenotype, edema, angiogenesis and smooth muscle hyperplasia (Lee et al., 2004, Nature Med 10:1095-1103).

Finally, TGFb stimulates production of extracellular matrix proteins including fibronectin and collagens and is believed to play an important role in fibrosis in many tissues (Leask et al., 2004, FASEB J 18(7):816-27; Bartram et al., 2004, Chest 125(2):754-65; Strutz et al., 2003, Springer Semin Immunopathol, 24:459-76; Wynn, 2004, Nat Rev Immunol, 4(8):583-94). Inhibitors of TGFb production and signaling pathways are active in a number of fibrosis animal models (Wang et al., 2002, Exp Lung Res, 28:405-17; Laping, 2003, Curr Opin Pharmacol, 3(2):204-8).

As summarized above, several growth factors are upregulated in fibrosis and the inhibition of a single factor seems to reduce the severity of fibrosis in the fibrosis models.

### SUMMARY OF THE INVENTION

Surprisingly, we found that the compounds of above general formula I are effective in the treatment or prevention of specific fibrotic diseases.

The present invention thus relates to the use of the compounds of above general formula I for the preparation of a medicament for the treatment or prevention of specific fibrotic diseases.

The present invention also relates to a method for the treatment or prevention of specific fibrotic diseases, by administration to a patient in need thereof of a pharmaceutical composition comprising a compound of above general formula I, together with a pharmaceutically suitable carrier. The expression "patient" is meant to comprise the mammalian animal body, preferably the human body.

The present invention further relates to a pharmaceutical composition for the treatment or prevention of specific fibrotic diseases which comprises a compound of above general formula I alone or in combination with one or more further therapeutic agents.

### DETAILLED DESCRIPTION OF THE INVENTION

In accordance with the present invention, the compounds of general formula I are the compounds 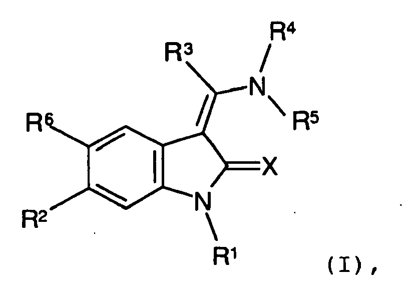 in which
I. In the above formula I,
   X is an oxygen atom,
   R¹ is a hydrogen atom,
   R² is a fluorine, chlorine or bromine atom or a cyano group,
   R³ is a phenyl group or a phenyl group which is monosubstituted by a fluorine, chlorine, bromine or iodine atom or by a C₁₋₃-alkoxy group, where the abovementioned unsubstituted and the monosubstituted phenyl groups may additionally be substituted in the 3- or 4-position
   by a fluorine, chlorine or bromine atom,
   by a cyano group,
   by a C₁₋₃-alkoxy or C₁₋₂-alkyl-carbonyl-amino group,
   by a cyano-C₁₋₃-alkyl, carboxy-C₁₋₃-alkyl, carboxy-C₁₋₄-alkoxy, carboxy-C₁₋₃-alkylamino, carboxy-C₁₋₃-alkyl-N-(C₁₋₃-alkyl)-amino, C₁₋₄-alkoxy-carbonyl-C₁₋₃-alkyl, C₁₋₄-alkoxy-carbonyl-C₁₋₃-alkoxy, C₁₋₄-alkoxy-carbonyl-C₁₋₃-alkylamino, C₁₋₄-alkoxycarbonyl-C₁₋₃-alkyl-N-(C₁₋₃-alkyl) -amino, amino-C₁₋₃-alkyl, aminocarbonyl-C₁₋₃-alkyl, (C₁₋₂-alkylamino)-carbonyl-C₁₋₃-alkyl, di-(C₁₋₂-alkyl)-amino-carbonyl-C₁₋₃-alkyl, (C₁₋₂-alkyl-carbonyl) -amino-C₁₋₃-alkyl, (C₁₋₄-alkoxy-carbonyl) -amino-C₁₋₃-alkyl, (C₃₋₆-alkyl-carbonyl) -amino-C₁₋₃-alkyl, (phenyl-carbonyl) -amino-C₁₋₃-alkyl, (C₃₋₆-cycloalkylcarbonyl) -amino-C₁₋₃-alkyl, (C₃₋₆-cycloalkyl-C₁₋₃-alkyl-carbonyl) -amino-C₁₋₃-alkyl, (thiophen-2-yl-carbonyl)-amino-C₁₋₃-alkyl, (furan-2-yl-carbonyl) - amino-C₁₋₃-alkyl, (phenyl-C₁₋₃-alkyl-carbonyl) - amino-C₁₋₃-alkyl, (2- (C₁₋₄-alkoxy) -benzoyl-carbonyl)-amino-C₁₋₃-alkyl, (pyridin-2-yl-carbonyl) -amino-C₁₋₃-alkyl, (pyridin-3-yl-carbonyl)-amino-C₁₋₃-alkyl-, (pyridin-4-yl-carbonyl) -amino-C₁₋₃-alkyl- or C₁₋₃-alkyl-piperazin-1-yl-carbonyl-C₁₋₃-alkyl group,
   by a carboxy-C₂₋₃-alkenyl, aminocarbonyl-C₂₋₃-alkenyl, (C₁₋₃-alkylamino) -carbonyl-C₂₋₃-alkenyl, di- (C₁₋₃-alkyl) -amino-carbonyl-C₂₋₃-alkenyl or C₁₋₄-alkoxy-carbonyl-C₂₋₃-alkenyl group,
   where the substituents may be identical or different,
   R⁴ is a phenyl group or a phenyl group which is monosubstituted
   by a C₁₋₃-alkyl group which is terminally substituted by an amino, guanidino, mono- or di-(C₁₋₂-alkyl)-amino-, N-[ω-di-(C₁₋₃-alkyl)-amino-C₂-₃-alkyl)-N- (C₁₋₃-alkyl) -amino, N-methyl-(C₃₋₄-alkyl)-amino, N-(C₁₋₃-alkyl)-N-benzylamino, N- (C₁₋₄-alkoxycarbonyl) -amino, N- (C₁₋₄-alkoxycarbonyl)-C₁₋₄-alkylamino, 4-(C₁₋₃-alkyl)-piperazin-1-yl, imidazol-1-yl, pyrrolidin-1-yl, azetidin-1-yl, morpholin-4-yl, piperazin-1-yl, thiomorpholin-4-yl group,
   by a di-(C₁₋₃-alkyl)-amino-(C₁₋₃-alkyl)-sulphonyl, 2- [di-(C₁₋₃-alkyl) -amino]-ethoxy, 4- (C₁₋₃-alkyl)-piperazin-1-yl-carbonyl, {ω [di-(C₁₋₃-alkyl) - amino]- (C₂₋₃-alkyl)}-N- (C₁₋₃-alkyl)-amino-carbonyl, 1- (C₁₋₃-alkyl) imidazol-2-yl, (C₁₋₃-alkyl)-sulphonyl group, or
   by a group of the formula 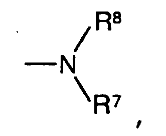 in which
   R⁷ is a C₁₋₂-alkyl, C₁₋₂-alkyl-carbonyl, di-(C₁₋₂-alkyl) -amino-carbonyl-C₁₋₃-alkyl or C₁₋₃-alkylsulphonyl group and
   R⁸ is C₁₋₃-alkyl, ω-[di-(C₁₋₂-alkyl)-amino]-C₂-₃-alkyl, ω-[mono- (C₁₋₂-alkyl) -amino] -C₂₋₃-alkyl group, or
   a (C₁₋₃-alkyl)-carbonyl, (C₄₋₆-alkyl)-carbonyl or carbonyl- (C₁₋₃-alkyl ) group which is terminally substituted by a di-(C₁₋₂-alkyl)-amino, piperazin-1-yl or 4- (C₁₋₃-alkyl) - piperazin-1-yl group,
   where all dialkylamino groups present in the radical R⁴ may also be present in quaternized form, for example as an N-methyl-(N,N-dialkyl)-ammonium group, where the counterion is preferably selected from the group consisting of iodide, chloride, bromide, methylsulphonate, para-toluenesulphonate and trifluoroacetate,
   R⁵ is a hydrogen atom and
   R⁶ is a hydrogen atom,
   where the abovementioned alkyl groups include linear and branched alkyl groups in which additionally one to 3 hydrogen atoms may be replaced by fluorine atoms, where additionally a carboxyl, amino or imino group present may be substituted by an in vivo cleavable radical or may be present in the form of a prodrug radical, for example in the form of a group which can be converted in vivo into a carboxyl group or in the form of a group which can be converted in vivo into an imino or amino group,
   their tautomers, enantiomers, diastereomers, their mixtures and their salts.
II. Particularly preferred compounds of the above formula I are those compounds in which X, R¹, R⁵ and R⁶ are as defined under I. and:
   II.i. R² and R⁴ are as defined under I. and
      R³ is a phenyl group or a phenyl group which is monosubstituted by a fluorine, chlorine, bromine or iodine atom or by a C₁₋₃-alkoxy group, where the abovementioned unsubstituted and the monosubstituted phenyl groups may additionally be substituted in the 3- or 4-position
      by a fluorine, chlorine or bromine atom,
      by a cyano group,
      by a -C₁₋₃-alkoxy or C₁₋₂-alkyl-carbonyl-amino group,
      by a cyano-C₁₋₃-alkyl, carboxy-C₁₋₃-alkyl, carboxy-C₁₋₄-alkoxy, carboxy-C₁₋₃-alkylamino, carboxy-C₁₋₃-alkyl-N-(C₁₋₃-alkyl)-amino, C₁₋₄-alkoxy-carbonyl-C₁₋₃-alkyl, C₁₋₄-alkoxy-carbonyl-C₁₋₃-alkoxy, C₁₋₄ alkoxy-carbonyl-C₁₋₃-alkylamino, C₁₋₄-alkoxycarbonyl-C₁₋₃-alkyl-N- (C₁₋₃-alkyl) -amino, amino-C₁₋₃-alkyl, aminocarbonyl-C₁₋₃-alkyl, (C₁₋₂-alkylamino)-carbonyl-C₁₋₃-alkyl, di- (C₁₋₂-alkyl)-amino-carbonyl-C₁₋₃-alkyl, (C₁₋₂-alkyl-carbonyl) -amino-C₁₋₃-alkyl, (C₁₋₄-alkoxy-carbonyl) -amino-C₁₋₃-alkyl, (C₃₋₆-alkyl-carbonyl) -amino-C₁₋₃-alkyl, (phenyl-carbonyl) -amino-C₁₋₃-alkyl, (C₃₋₆-cycloalkylcarbonyl) -amino-C₁₋₃-alkyl, (C₃₋₆-cycloalkyl-C₁-₃-alkyl-carbonyl)-amino-C₁₋₃-alkyl, (thiophen-2-yl-carbonyl)-amino-C₁₋₃-alkyl, (furan-2-yl-carbonyl)-amino-C₁₋₃-alkyl, (phenyl-C₁₋₃-alkyl-carbonyl) - amino-C₁₋₃-alkyl, (2-(C₁₋₄-alkoxy)-benzoyl-carbonyl)-amino-C₁₋₃-alkyl, (pyridin-2-yl-carbonyl)-amino-C₁₋₃-alkyl, (pyridin-3-yl-carbonyl)-amino-C₁₋₃-alkyl, (pyridin-4-yl-carbonyl)-amino-C₁₋₃-alkyl or C₁₋₃-alkyl-piperazin-1-yl-carbonyl-C₁₋₃-alkyl group,
      by a carboxy-C₂₋₃-alkenyl, aminocarbonyl-C₂₋₃-alkenyl-, (C₁₋₃-alkylamino)-carbonyl-C₂₋₃-alkenyl-, di-(C₁₋₃-alkyl)-amino-carbonyl-C₂₋₃-alkenyl or C₁₋₄-alkoxy-carbonyl-C₂₋₃-alkenyl group,
      where the substituents may be identical or different;
   II.ii. R² and R⁴ are as defined under I. and
      R³ is a phenyl group which is substituted
      by a C₁₋₂-alkyl-carbonyl-amino group,
      by a carboxy-C₁₋₃-alkyl, carboxy-C₁₋₄-alkoxy, C₁₋₄-alkoxy-carbonyl-C₁₋₃-alkyl, C₁₋₄-alkoxy-carbonyl-C₁₋₃-alkoxy, aminocarbonyl-C₁₋₃-alkyl, (C₁₋₂-alkylamino)-carbonyl-C₁₋₃-alkyl, di- (C₁₋₂-alkyl)-amino-carbonyl-C₁₋₃-alkyl, (C₁₋₂-alkyl-carbonyl) -amino-C₁₋₃-alkyl, (C₁₋₄-alkoxy-carbonyl) -amino-C₁₋₃-alkyl, (phenyl-carbonyl) -amino-C₁₋₃-alkyl, (C₃₋₆-cycloalkyl-carbonyl) -amino-C₁₋₃-alkyl, (C₃₋₆-cycloalkyl-C₁₋₃-alkyl-carbonyl)-amino-C₁₋₃-alkyl, (thiophen-2-yl-carbonyl)-amino-C₁₋₃-alkyl, (furan-2-yl-carbonyl)-amino-C₁₋₃₋alkyl, (phenyl-C₁₋₃-alkyl-carbonyl)-amino-C₁₋₃-alkyl, (2-(C₁₋₄-alkoxy)-benzoyl-carbonyl)-amino-C₁₋₃-alkyl, (pyridin-2-yl-carbonyl) -amino-C₁₋₃-alkyl, (pyridin-3-yl-carbonyl) -amino-C₁₋₃-alkyl, (pyridin-4-yl-carbonyl) -amino-C₁₋₃-alkyl or C₁₋₃-alkyl-piperazin-1-yl-carbonyl-C₁₋₃-alkyl group,
      by an aminocarbonyl-C₂₋₃-alkenyl, (C₁₋₃-alkylamino) -carbonyl-C₂₋₃-alkenyl, di- (C₁₋₃-alkyl) - amino-carbonyl-C₂₋₃-alkenyl or C₁₋₄-alkoxycarbonyl-C₂₋₃-alkenyl group;
   II.iii. R² and R⁴ are as defined under I. and
      R³ is a phenyl group substituted by a carboxy-C₁₋₃-alkyl or C₁₋₄-alkoxy-carbonyl-C₁₋₃-alkyl group;
   II.iv. R³ and R⁴ are as defined under I. and
      R² is a fluorine or chlorine atom;
   II.v. R² and R³ are as defined under I. and
      R⁴ is a phenyl group or a phenyl group which is monosubstituted
      by a C₁₋₃-alkyl group which is terminally substituted by an amino, guanidino, mono- or di-(C₁₋₂-alkyl)-amino-, N- [ω-di- (C₁₋₃-alkyl) -amino-C₂-₃-alkyl]-N- (C₁₋₃-alkyl) -amino, N-methyl- (C₃₋₄-alkyl)-amino, N-(C₁₋₃-alkyl)-N-benzylamino, N- (C₁₋₄-alkoxycarbonyl) -amino, N- (C₁₋₄-alkoxycarbonyl) -C₁₋₄-alkylamino, 4-(C₁₋₃-alkyl)-piperazin-1-yl, imidazol-1-yl, pyrrolidin-1-yl, azetidin-1-yl, morpholin-4-yl, piperazin-1-yl, thiomorpholin-4-yl group,
      by a di-(C₁₋₃-alkyl)-amino-(C₁₋₃-alkyl)-sulphonyl, 2- [di-(C₁₋₃-alkyl) -amino] -ethoxy, 4- (C₁₋₃-alkyl) - piperazin-1-yl-carbonyl, {ω- [di- (C₁₋₃-alkyl) - amino]- (C₂₋₃-alkyl) }-N- (C₁₋₃-alkyl) -amino-carbonyl, 1- (C₁₋₃-alkyl) imidazol-2-yl, (C₁₋₃-alkyl)-sulphonyl group, or
      by a group of the formula 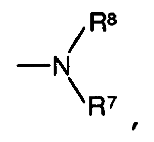 in which
      R⁷ is a C₁₋₂-alkyl, C₁₋₂-alkyl-carbonyl, di-(C₁₋₂-alkyl)-amino-carbonyl-C₁₋₃-alkyl or C₁₋₃-alkylsulphonyl group and
      R⁸ is C₁₋₃-alkyl, ω-[di-(C₁₋₂-alkyl)-amino]-C₂-₃-alkyl, ω- [mono- (C₁₋₂-alkyl) -amino] -C₂₋₃-alkyl group, or
      a (C₁₋₃-alkyl)-carbonyl, (C₄₋₆-alkyl)-carbonyl or carbonyl-(C₁₋₃-alkyl) group which is terminally substituted by a di-(C₁₋₂-alkyl)-amino, piperazin-1-yl or 4-(C₁₋₃-alkyl)-piperazin-1-yl group,
      where all dialkylamino groups present in the radical R⁴ may also be present in quaternized form, for example as an N-methyl-(N,N-dialkyl)-ammonium group, where the counterion is preferably selected from the group consisting of iodide, chloride, bromide, methylsulphonate, para-toluenesulphonate and trifluoroacetate.
III. Subgroups of particularly preferred compounds of the above formula I which are to be mentioned in particular are those in which:
   III.i. X, R¹, R², R⁵ and R⁶ are as defined under I., R³ is as defined under II.i. and R⁴ is as defined under II.v.;
   III. ii. X, R¹, R², R⁵ and R⁶ are as defined under I., R³ is as defined under II.ii. and R⁴ is as defined under II.v.;
   III.iii. X, R¹, R², R⁵ and R⁶ are as defined under I., R³ is as defined under II.iii. and R⁴ is as defined under II.v.;
   III.iv. X, R¹, R⁵ and R⁶ are as defined under I., R² is as defined under II.iv., R³ is as defined under II.i., II.ii. or II.iii. and R⁴ is as defined under II.v.

A further preferred group of compounds of the above formula I are those in which
X is an oxygen atom,
R¹ is a hydrogen atom,
R² is a fluorine, chlorine or bromine atom or a cyano group,
R³ is a phenyl group or a phenyl group which is monosubstituted by a fluorine, chlorine, bromine or iodine atom or by a C₁₋₃-alkoxy group, where the abovementioned unsubstituted and the monosubstituted phenyl groups may additionally be substituted in the 3- or 4-position
by a fluorine, chlorine or bromine atom,
by a C₁₋₃-alkoxy or C₁₋₂-alkyl-carbonyl-amino group, by a carboxy-C₁₋₃-alkyl, aminocarbonyl-C₁₋₃-alkyl, (C₁₋₂-alkylamino)-carbonyl-C₁₋₃-alkyl, di-(C₁₋₂-alkyl)-amino-carbonyl-C₁₋₃-alkyl, (C₁₋₂-alkyl-carbonyl) -amino-C₁₋₃-alkyl or (phenyl-carbonyl)-amino-C₁₋₃-alkyl group,
where the substituents may be identical or different,
R⁴ is a phenyl group which is substituted
by a C₁-₃-alkyl group terminally substituted by a di-(C₁₋₂-alkyl)-amino group, or
by a group of the formula 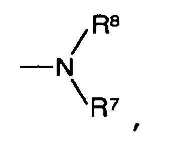 in which
R⁷ is a C₁₋₂-alkyl, C₁₋₂-alkyl-carbonyl, di- (C₁₋₂-alkyl)-amino-carbonyl-C₁₋₃-alkyl or C₁₋₃-alkylsulphonyl group and
R⁸ is a C₁₋₃-alkyl or ω-[di-(C₁₋₂-alkyl) -amino] -C₂₋₃-alkyl group, or
a C₁₋₃-alkyl-carbonyl group terminally substituted by a di-(C₁₋₂-alkyl)-amino, piperazino or 4-(C₁₋₃-alkyl)-piperazin-1-yl group,
R⁵ is a hydrogen atom and
R⁶ is a hydrogen atom,
where the abovementioned alkyl groups include linear and branched alkyl groups in which additionally one to 3 hydrogen atoms may be replaced by fluorine atoms,
where additionally a carboxyl, amino or imino group present may be substituted by an in vivo cleavable radical,
their tautomers, enantiomers, diastereomers, their mixtures and their salts.

The following compounds of the formula I are particularly preferred:
(a) 3-Z-[1-(4-dimethylaminomethylanilino)-1-(3-(2-carboxyethyl)phenyl)methylene]-6-chloro-2-indolinone
(b) 3-Z-[1-(4-dimethylaminomethylanilino)-1-(4-(2-carboxyethyl)phenyl)methylene]-6-fluoro-2-indolinone
(c) 3-Z-[1-(4-dimethylaminomethylanilino)-1-(3-(2-carboxyethyl)phenyl)methylene]-6-fluoro-2-indolinone
(d) 3-Z-[1-(4-(N-(4-methylpiperazin-1-ylmethylcarbonyl)-N-methylamino)anilino)-1-(4-(2-carboxyethyl)phenyl)methylene]-6-fluoro-2-indolinone
(e) 3-Z-[1-(4-(N-(2-dimethylaminoethyl)-N-methylsulphonylamino)anilino)-1-(4-(2-carboxyethyl)phenyl)methylene]-6-fluoro-2-indolinone
(f) 3-Z-[1-(4-(N-(3-dimethylaminopropyl)-N-acetylamino) anilino)-1- (4- (2-carboxyethyl)phenyl)methylene]-6-fluoro-2-indolinone
(g) 3-Z-[1-(4-(1-methylimidazol-2-yl)anilino)-1-(4-(2-carboxyethyl)phenyl)methylene]-6-fluoro-2-indolinone
(h) 3-Z-[1-(4-(N-(dimethylaminomethylcarbonyl)-N-methylamino)anilino)-1-(4-(2-carboxyethyl)phenyl)methylene]-6-fluoro-2-indolinone
(i) 3-Z-[1-(4-(N-(2-dimethylaminoethylcarbonyl)-N-methylamino)anilino)-1-(4- (2-carboxyethyl)phenyl)methylene]-6-fluoro-2-indolinone
(j) 3-Z-[1-(4-(pyrrolidin-1-ylmethyl) anilino)-1-(4-(2-carboxyethyl)phenyl)methylene]-6-fluoro-2-indolinone
(k) 3-Z-[1-(4-(diethylaminomethyl) anilino)-1-(4-(2-carboxyethyl)phenyl)methylene]-6-fluoro-2-indolinone.
(l) 3-Z-[1-(4-(2-dimethylaminoethyl)anilino)-1-(4-(2-carboxyethyl)phenyl)methylene]-6-chloro-2-indolinone
(m) 3-Z-[1-(4-dimethylaminomethylanilino)-1-(4-(2-carboxyethyl)phenyl)methylene]-6-chloro-2-indolinone
(n) 3-Z-[1-(4-(pyrrolidin-1-ylmethyl) anilino)-1-(4-(2-carboxyethyl)phenyl)methylene]-6-chloro-2-indolinone
(o) 3-Z-[1-(4-(pyrrolidin-1-ylmethyl)anilino)-1-(4-(2-carboxyethyl)phenyl)methylene]-6-bromo-2-indolinone
(p) 3-Z-[1-(4-(dimethylaminomethyl)anilino)-1-(4-(2-carboxyethyl)phenyl)methylene]-6-bromo-2-indolinone
(q) 3-Z-[1-(4-(diethylaminomethyl) anilino) -1- (4- (2-carboxyethyl)-methylene]-6-bromo-2-indolinone
where additionally a carboxyl, amino or imino group present may be substituted by an in vivo cleavable radical or may be present in the form of a prodrug radical, for example in the form of a group which can be converted in vivo into a carboxyl group or in the form of a group which can be converted in vivo into an imino or amino group,
their tautomers, enantiomers, diastereomers, their mixtures and their salts.

A group which can be converted in vivo into a carboxyl group is to be understood as meaning, for example, a hydroxymethyl group, a carboxyl group which is esterified with an alcohol in which the alcoholic moiety is preferably a C₁₋₆-alkanol, a phenyl-C₁₋₃-alkanol, a C₃₋₉-cycloalkanol, where a C₅₋₈-cycloalkanol may additionally be substitituted by one or two C₁₋₃-alkyl groups, a C₅₋₈-cycloalkanol in which one methylene group in the 3- or 4-position is replaced by an oxygen atom or by an imino group optionally substituted by a C₁₋₃-alkyl, phenyl-C₁₋₃-alkyl, phenyl-C₁₋₃-alkoxy-carbonyl or C₁₋₆-alkyl-carbonyl group and in which the cycloalkanol moiety may additionally be substituted by one or two C₁₋₃-alkyl groups, a C₄₋₇-cycloalkenol, a C₃₋₅-alkenol, a phenyl-C₃₋₅-alkenol, a C₃₋₅-alkynol or a phenyl-C₃₋₅-alkynol, with the proviso that no bond to the oxygen atom originates from a carbon atom which carries a double or triple bond, a C₃₋₈-cycloalkyl-C₁₋₃-alkanol, a bicycloalkanol having a total of 8 to 10 carbon atoms which may additionally be substituted in the bicycloalkyl moiety by one or two C₁₋₃-alkyl groups, a 1,3-dihydro-3-oxo-1-isobenzofuranol or an alcohol of the formula

Rₐ-CO-O- (R_{b}CR_{c}) -OH,

in which
Rₐ is a C₁₋₈-alkyl, C₅₋₇-cycloalkyl, phenyl or phenyl-C₁₋₃-alkyl group, .
R_{b} is a hydrogen atom, a C₁₋₃-alkyl, C₅₋₇-cycloalkyl or phenyl group, and
R_{c} is a hydrogen atom or a C₁₋₃-alkyl group,
and a radical cleavable in vivo from an imino or amino group is to be understood as meaning, for example, a hydroxyl group, an acyl group, such as the benzoyl or pyridinoyl group, or a C₁₋₁₆-alkylcarbonyl group, such as the formyl, acetyl, propionyl, butanoyl, pentanoyl or hexanoyl group, an allyloxycarbonyl group, a C₁₋₁₆-alkoxycarbonyl group, such as the methoxycarbonyl, ethoxycarbonyl, propoxycarbonyl, isopropoxycarbonyl, butoxycarbonyl, tert-butoxycarbonyl, pentoxycarbonyl, hexyloxycarbonyl, octyloxycarbonyl, nonyloxycarbonyl, decyloxycarbonyl, undecyloxycarbonyl, dodecyloxycarbonyl or hexadecyloxycarbonyl group, a phenyl-C₁₋₆-alkoxy-carbonyl group, such as the benzyloxycarbonyl, phenylethoxycarbonyl or phenylpropoxycarbonyl group, a C₁₋₃-alkylsulphonyl-C₁₋₄-alkoxy-carbonyl, C₁₋₃-alkoxy-C₂₋₄-alkoxy-C₂₋₄-alkoxy-carbonyl or RₐCO-O- (R_{b}CR_{c}) -O-CO- group, in which
Rₐ is a C₁₋₈-alkyl, C₅₋₇-cycloalkyl, phenyl or phenyl-C₁₋₃-alkyl group,
R_{b} is a hydrogen atom, a C₁₋₃-alkyl, C₅₋₇-cycloalkyl or phenyl group and
R_{c} is a hydrogen atom, a C₁₋₃-alkyl or RₐCO-O- (R_{b}CR_{c}) -O-group, in which Rₐ to R_{c} are as defined above,
and additionally, for an amino group, the phthalimido group, where the ester radicals mentioned above can also be used as a group which can be converted in vivo into a carboxyl group.

Preferred prodrug radicals for a carboxyl group are a C₁₋₆-alkoxy-carbonyl group, such as the methoxycarbonyl, ethoxycarbonyl, n-propyloxycarbonyl, isopropyloxycarbonyl, n-butyloxycarbonyl, n-pentyloxycarbonyl, n-hexyloxycarbonyl or cyclohexyloxycarbonyl group, or a phenyl-C₁₋₃-alkoxycarbonyl group, such as the benzyloxycarbonyl group, and, for an imino or amino group, a C₁₋₉-alkoxy-carbonyl group, such as the methoxycarbonyl, ethoxycarbonyl, n-propyloxycarbonyl, isopropyloxycarbonyl, n-butyloxycarbonyl, n-pentyloxycarbonyl, n-hexyloxycarbonyl, cyclohexyloxycarbonyl, n-heptyloxycarbonyl, n-octyloxycarbonyl or n-nonyloxycarbonyl group, a phenyl-C₁₋₃-alkoxy-carbonyl group, such as the benzyloxycarbonyl group, a phenylcarbonyl group optionally substituted by a C₁₋₃-alkyl group, such as the benzoyl or 4-ethyl-benzoyl group, a pyridinoyl group, such as the nicotinoyl group, a C₁₋₃-alkylsulphonyl-n-C₂₋₃-alkoxy-carbonyl or C₁₋₃-alkoxy-C₂₋₃-alkoxy-C₁₋₄-alkoxy-carbonyl group, such as the 2-methylsulphonylethoxycarbonyl or 2-(2-ethoxy)-ethoxycarbonyl group.

The above exemplified compounds, their tautomers, their stereoisomers or the physiologically acceptable salts thereof, as well as their manufacturing process, have been described in WO 04/009547, the content of which is incorporated herein by reference.

The following list of specific compounds is illustrative of the present invention, without constituting any limitation of its scope:
(1) 3-Z-[1-(4-(N-methyl-N-methylsulphonylamino)anilino)-1-(3-iodophenyl)-methylene]-6-chloro-2-indolinone
(2) 3-Z-[1-(4-(dimethylaminomethyl)anilino)-1-(3-iodophenyl)methylene]-6-chloro-2-indolinone
(3) 3-Z-[1-(4-(N-(dimethylaminomethylcarbonyl)-N-methylamino)aniline)-1-(4-chlorophenyl)methylene]-6-chloro-2-indolinone
(4) 3-Z-[1-(4-(N-(2-dimethylaminoethyl) -N-acetylamino)anilino)-1-(4-chlorophenyl)methylene]-6-chloro-2-indolinone
(5) 3-Z-[1-(4-(N-(4-methylpiperazin-1-ylmethylcarbonyl)-N-methylamino)anilino)-1-(4-chlorophenyl)methylene]-6-chloro-2-indolinone
(6) 3-Z-[1-(4-(N-(3-dimethylaminopropyl)-N-acetylamino)anilino)-1-(4-chlorophenyl)methylene]-6-chloro-2-indolinone
(7) 3-Z-[1-(4-(dimethylaminomethyl)anilino)-1-(4-chlorophenyl)methylene]-6-chloro-2-indolinone
(8) 3-Z-[1-(4-(N-(2-dimethylaminoethyl)-N-acetylamino) anilino)-1-(3,4-dimethoxyphenyl) methylene]-6-chloro-2-indolinone
(9) 3-Z-[1-(4-(N-(4-methylpiperazin-1-ylmethylcarbonyl)-N-methylamino)anilino)-1-(3,4-dimethoxyphenyl)methylene]-6-chloro-2-indolinone
(10) 3-Z-[1-(4-(N-(2-dimethylaminoethyl)-N-methylsulphonylamino)anilino)-1-(3,4-dimethoxyphenyl)methylene]-6-chloro-2-indolinone
(11) 3-Z-[1-(4-(dimethylaminomethyl)anilino)-1-(3,4-dimethoxyphenyl)-methylene]-6-chloro-2-indolinone
(12) 3-Z-[1-(4-(N-(2-dimethylaminoethyl)-N-methylcarbamoyl)anilino)-1-(3,4-dimethoxyphenyl)methylene]-6-chloro-2-indolinone
(13) 3-Z-[1-(4-(dimethylaminomethyl)anilino)-1-(4-cyanophenyl)-methylene]-6-chloro-2-indolinone
(14) 3-Z-[1-(4-(dimethylaminomethyl)anilino)-1-(4-iodophenyl)methylene]-6-fluoro-2-indolinone
(15) 3-Z-[1-(4-(dimethylaminomethyl)anilino)-1-(3-fluorophenyl)methylene]-6-fluoro-2-indolinone
(16) 3-Z-[1-(4-(N-(3-dimethylaminopropyl)-N-acetylamino)anilino)-1-(3-fluorophenyl)methylene]-6-fluoro-2-indolinone
(17) 3-Z-[1-(4-(N-(4-methylpiperazin-1-ylmethylcarbonyl)-N-methylamino)anilino)-1-(3-fluorophenyl)methylene]-6-fluoro-2-indolinone
(18) 3-Z-[1-(4-(dimethylaminomethyl)anilino)-1-(4-(2-acetylaminoethyl)phenyl)methylene]-6-fluoro-2-indolinone
(19) 3-Z-[1-(4-(N-(3-dimethylaminopropyl)-N-acetylamino)anilino)-1-(4-(2-acetylaminoethyl)phenyl)methylene]-6-fluoro-2-indolinone
(20) 3-Z-[1-(4-(N-(4-methylpiperazin-1-ylmethylcarbonyl)-N-methylamino) anilino)-1-(4-(2-acetylaminoethyl)phenyl)methylene]-6-fluoro-2-indolinone
(21) 3-Z-[1-(4-(dimethylaminomethyl)anilino)-1-(4-methoxycarbonylmethylphenyl)methylene]-6-fluoro-2-indolinone
(22) 3-Z-[1-(4-(dimethylaminomethyl)anilino)-1-(3-iodophenyl)methylene]-6-fluoro-2-indolinone
(23) 3-Z-[1-(4-(dimethylaminomethyl)anilino)-1-(3-methoxycarbonylmethylphenyl)methylene]-6-fluoro-2-indolinone
(24) 3-Z-[1-(4-(dimethylaminomethyl) anilino)-1-(3-(N-tert-butoxycarbonyl-aminomethyl)phenyl)methylene]-6-fluoro-2-indolinone
(25) 3-Z-[1-(4-(N-(2-dimethylaminoethyl)-N-methylsulphonylamino)anilino)-1-(4-methoxycarbonylmethylphenyl)methylene]-6-fluoro-2-indolinone
(26) 3-Z-[1-(4-(N-(4-methylpiperazin-1-ylmethylcarbonyl)-N-methylamino) anilino)-1-(4-methoxycarbonylmethylphenyl)methylene]-6-fluoro-2-indolinone
(27) 3-Z-[1-(4-(dimethylaminomethyl)anilino)-1-(3-cyanomethylphenyl)-methylene]-6-fluoro-2-indolinone
(28) 3-Z-[1-(4-(N-(4-methylpiperazin-1-ylmethylcarbonyl)-N-methylamino)anilino)-1-(4-(N-tert-butoxycarbonylaminomethyl)phenyl)methylene]-6-fluoro-2-indolinone
(29) 3-Z-[1-(4-(dimethylaminomethyl)anilino)-1-(4-(N-tert-butoxycarbonyl-aminomethyl)phenyl)methylene]-6-fluoro-2-indolinone
(30) 3-Z-[1-(4-(dimethylaminomethyl) anilino)-1-(3-(N-tert-butoxycarbonyl-2-aminoethyl)phenyl)methylene]-6-fluoro-2-indolinone
(31) 3-Z-[1-(4-(N-Acetyl-N-methylamino) anilino) -1-(4-(2-methoxycarbonylethyl)phenyl)methylene]-6-fluoro-2-indolinone
(32) 3-Z-[1-(4-(N-(4-methylpiperazin-1-ylmethylcarbonyl)-N-methylamino) anilino)-1-(4-(2-methoxycarbonylethyl)phenyl)methylene]-6-fluoro-2-indolinone
(33) 3-Z-[1-(4-(N-(2-dimethylaminoethyl) -N-methylsulphonylamino)anilino)-1-(4-methoxycarbonylmethylphenyl)methylene]-6-fluoro-2-indolinone
(34) 3-Z-[1-(4-(N-(3-dimethylaminopropyl)-N-acetylamino)anilino)-1-(4-(2-methoxycarbonylethyl)phenyl)methylene]-6-fluoro-2-indolinone
(35) 3-Z-[1-(4-(N-tert-butoxycarbonylmethylaminomethyl)anilino)-1-(4-(2-methoxycarbonylethyl)phenyl)methylene]-6-fluoro-2-indolinone
(36) 3-Z-[1-(4-(4-methylpiperazin-1-yl-carbonyl)anilino)-1-(4-(2-methoxycarbonylethyl)phenyl)methylene]-6-fluoro-2-indolinone
(37) 3-Z-[1-(4-(1-methylimidazol-2-yl)anilino)-1-(4-(2-methoxycarbonylethyl)phenyl)methylene]-6-fluoro-2-indolinone
(38) 3-Z-[1-(4-methylsulphonylanilino)-1-(4-(2-methoxycarbonylethyl)phenyl)methylene]-6-fluoro-2-indolinone
(39) 3-Z-[1-(4-(N-(4-methylpiperazin-1-ylmethylcarbonyl)-N-methylamino)anilino)-1-(3-methoxycarbonylmethylphenyl)methylene)-6-fluoro-2-indolinone
(40) 3-Z-[1-(4-(N-(2-dimethylaminoethyl)-N-methylsulphonylamino)anilino)-1-(3-methoxycarbonylmethylphenyl)methylene]-6-fluoro-2-indolinone
(41) 3-Z-[1-(4-(4-methylpiperazin-1-yl-carbonyl)anilino)-1-(3-methoxycarbonylmethylphenyl)methylene]-6-fluoro-2-indolinone
(42) 3-Z-[1-(4-(N-(dimethylaminomethylcarbonyl)-N-methylamino)anilino)-1-(3-methoxycarbonylmethylphenyl)methylene]-6-fluoro-2-indolinone
(43) 3-Z-[1-(4-(N-(3-dimethylaminopropyl)-N-acetylamino) anilino) -1- (3-methoxycarbonylmethylphenyl)methylene]-6-fluoro-2-indolinone
(44) 3-Z-[1-(4-(N-(2-dimethylaminoethyl)-N-acetylamino) anilino)-1-(3-methoxycarbonylmethylphenyl)methylene]-6-fluoro-2-indolinone
(45) 3-Z-[1-(4-(N-(4-dimethylamino-butylcarbonyl)-N-methylamino)anilino)-1-(3-methoxycarbonylmethylphenyl)methylene]-6-fluoro-2-indolinone
(46) 3-Z-[1-Anilino-1-(4-methoxycarbonylmethylphenyl)methylene]-6-fluoro-2-indolinone
(47) 3-Z-[1-(4-(1-methylimidazol-2-yl) anilino)-1-(4-methoxycarbonylmethylphenyl)methylene]-6-fluoro-2-indolinone
(48) 3-Z-[1-(4-(4-methylpiperazin-1-ylcarbonyl) anilino) -1-(4-methoxycarbonylmethylphenyl)methylene]-6-fluoro-2-indolinone
(49) 3-Z-[1-(4-(N-(dimethylaminocarbonylmethyl) -N-methylsulphonylamino)anilino)-1-(4-methoxycarbonylmethylphenyl)-methylene]-6-fluoro-2-indolinone
(50) 3-Z-[1-(4-(N-(dimethylaminomethylcarbonyl)-N-methylamino)anilino)-1-(4-methoxycarbonylmethylphenyl)methylene]-6-fluoro-2-indolinone

(51) 3-Z-[1-(4-(N-methyl-N-acetylamino) anilino)-1-(4-methoxycarbonylmethylphenyl)methylene]-6-fluoro-2-indolinone
(52) 3-Z-[1-(4-(N-(2-dimethylaminoethylcarbonyl)-N-methylamino)anilino)-1-(4-methoxycarbonylmethylphenyl)methylene]-6-fluoro-2-indolinone
(53) 3-Z-[1-(4-methylsulphonylanilino)-1-(4-methoxycarbonylmethylphenyl)methylene]-6-fluoro-2-indolinone
(54) 3-Z-[1-(4-(N-(3-dimethylaminopropylcarbonyl)-N-methylamino)anilino)-1-(4-methoxycarbonylmethylphenyl)methylene]-6-fluoro-2-indolinone
(55) 3-Z-[1-(4-(N-(3-dimethylaminopropyl)-N-acetylamino) anilino) -1- (4-methoxycarbonylmethylphenyl)methylene]-6-fluoro-2-indolinone
(56) 3-Z-[1-Anilino-1-(3-methoxycarbonylmethylphenyl)methylene]-6-fluoro-2-indolinone
(57) 3-Z-[1-(4-methylsulphonylanilino)-1-(3-methoxycarbonylmethylphenyl)-methylene]-6-fluoro-2-indolinone
(58) 3-Z-[1-(4-(1-methylimidazol-2-yl)anilino)-1-(3-methoxycarbonylmethylphenyl)methylene]-6-fluoro-2-indolinone
(59) 3-Z-[1-(4-(N-(dimethylaminocarbonylmethyl) -N-methylsulphonylamino)anilino)-1-(3-methoxycarbonylmethylphenyl)-methylene]-6-fluoro-2-indolinone
(60) 3-Z-[1-(4-(N-(3-dimethylaminopropylcarbonyl)-N-methylamino)anilino)-1-(3-methoxycarbonylmethylphenyl)methylene]-6-fluoro-2-indolinone
(61) 3-Z-[1-(4-(N-(2-dimethylaminoethylcarbonyl) -N-methylamino)anilino)-1-(3-methoxycarbonylmethylphenyl)methylene]-6-fluoro-2-indolinone
(62) 3-Z-[1-(4-(N-(4-methylpiperazin-1-ylmethylcarbonyl)-N-methylamino)anilino)-1-(3-(2-methoxycarbonylethyl)phenyl)methylene]-6-fluoro-2-indolinone
(63) 3-Z-[1-(4-(N-(2-dimethylaminoethyl)-N-methylsulphonylamino)anilino)-1-(3-(2-methoxycarbonylethyl)phenyl)methylene]-6-fluoro-2-indolinone
(64) 3-Z-[1-(4-(N-(dimethylaminomethylcarbonyl)-N-methylamino)anilino)-1-(3-(2-methoxycarbonylethyl)phenyl)methylene]-6-fluoro-2-indolinone
(65) 3-Z-[1-(4-(N-(3-dimethylaminopropyl)-N-acetylamino) anilino)-1-(3- (2-methoxycarbonylethyl)phenyl)methylene]-6-fluoro-2-indolinone
(66) 3-Z-[1-(4-(N-(4-methylpiperazin-1-ylmethylcarbonyl)-N-methylamino)anilino)-1-(3-(N-tert-butoxycarbonylaminomethyl)phenyl)methylene]-6-fluoro-2-indolinone
(67) 3-Z-[1-(4-(N-methyl-N-acetylamino) anilino)-1-(3-acetylaminomethylphenyl)methylene]-6-chloro-2-indolinone
(68) 3-Z-[1-(4-(N-(3-dimethylaminopropyl)-N-acetylamino)anilino)-1-(3-acetylaminomethylphenyl)methylene]-6-chloro-2-indolinone
(69) 3-Z-[1-(4-(N-(2-dimethylaminoethyl)-N-methylsulphonylamino)anilino)-1-(3-acetylaminomethylphenyl)methylene]-6-chloro-2-indolinone
(70) 3-Z-[1-(4-(N-(dimethylaminomethylcarbonyl)-N-methylamino)anilino)-1-(3-acetylaminomethylphenyl)methylene]-6-chloro-2-indolinone
(71) 3-Z-[1-(4-(2-dimethylaminoethyl) anilino) -1- (4- (2-methoxycarbonylethyl)phenyl)methylene]-6-fluoro-2-indolinone
(72) 3-Z-[1-(4-(N-(2-dimethylaminoethylcarbonyl) -N-methylamino)anilino)-1-(4-(2-methoxycarbonylethyl)phenyl)methylene]-6-fluoro-2-indolinone
(73) 3-Z-[1-(4-(N-(dimethylaminomethylcarbonyl)-N-methylamino)anilino)-1-(4-(2-methoxycarbonylethyl)phenyl)methylene]-6-fluoro-2-indolinone
(74) 3-Z-[1-(4-(2-dimethylaminoethyl)anilino)-1-(3-(2-ethoxycarbonylethyl)phenyl)methylene]-6-fluoro-2-indolinone
(75) 3-Z-[1-(4-(N-(2-dimethylaminoethyl) -N-acetylamino) anilino) -1- (4- (2-methoxycarbonylethyl)phenyl)methylene]-6-fluoro-2-indolinone
(76) 3-Z-[1-(4-(N-(3-dimethylaminopropylcarbonyl)-N-methylamino)anilino)-1-(3-(2-ethoxycarbonylethyl)phenyl)methylene]-6-fluoro-2-indolinone
(77) 3-Z-[1-(4-(N-(4-dimethylaminobutylcarbonyl)-N-methylamino)anilino)-1-(3-(2-ethoxycarbonylethyl)phenyl)methylene]-6-fluoro-2-indolinone
(78) 3-Z-[1-(4-(1-methylimidazol-2-yl)anilino)-1-(3-(2-ethoxycarbonylethyl)phenyl)methylene]-6-fluoro-2-indolinone
(79) 3-Z-[1-(4-(N-(4-dimethylaminobutylcarbonyl)-N-methylamino)anilino)-1-(4-(2-methoxycarbonylethyl)phenyl)methylene]-6-fluoro-2-indolinone
(80) 3-Z-[lanilino-1-(4-(2-methoxycarbonylethyl)phenyl)methylene]-6-fluoro-2-indolinone
(81) 3-Z-[1-(4-(pyrrolidin-1-ylmethyl)anilino)-1-(4-(2-methoxycarbonylethyl)phenyl)methylene]-6-fluoro-2-indolinone
(82) 3-Z-[1-(4-(diethylaminomethyl)anilino)-1-(4-(2-methoxycarbonylethyl)phenyl)methylene]-6-fluoro-2-indolinone
(83) 3-Z-[1-(4-(N-tert-butoxycarbonylaminomethyl) anilino)-1-(4-(2-methoxycarbonylethyl)phenyl)methylene]-6-fluoro-2-indolinone
(84) 3-Z-[1-(4-(2-dimethylaminoethyl) anilino) -1- (3-methoxycarbonylmethylphenyl)methylene]-6-fluoro-2-indolinone
(85) 3-Z-[1-(4-(2-dimethylaminoethyl) anilino) -1- (4-methoxycarbonylmethylphenyl)methylene]-6-fluoro-2-indolinone
(86) 3-Z-[1-(4-(dimethylaminomethyl) anilino)-1-(3-(2-ethoxycarbonylethyl)phenyl)methylene]-6-fluoro-2-indolinone
(87) 3-Z-[1-(4-(2-dimethylaminoethyl) anilino)-1-(4- (2-methoxycarbonylethyl)phenyl)methylene]-6-chloro-2-indolinone
(88) 3-Z-[1-(4-(1-methylimidazol-2-yl)anilino)-1-(4- (2-ethoxycarbonylethyl)phenyl)methylene]-6-chloro-2-indolinone
(89) 3-Z-[1-(4-dimethylaminomethylanilino)-1-(4- (2-methoxycarbonylethyl)phenyl)methylene]-6-chloro-2-indolinone
(90) 3-Z-[1-(4-dimethylaminomethylanilino)-1-(4-(2-ethoxycarbonylethyl)phenyl)methylene]-6-fluoro-2-indolinone
(91) 3-Z-[1-(4-((4-methylpiperazin-1-yl)methyl)anilino)-1-(3-(2-methoxycarbonylethyl)phenyl)methylene]-6-fluoro-2-indolinone
(92) 3-Z-[1-(4-(imidazol-1-ylmethyl) anilino)-1-(3-(2-methoxycarbonylethyl)phenyl)methylene]-6-fluoro-2-indolinone
(93) 3-Z-[1-(4-((4-methylpiperazin-1-yl)methyl) anilino) -1-(4-(2-methoxycarbonylethyl)phenyl)methylene]-6-fluoro-2-indolinone
(94) 3-Z-[1-(4-(imidazol-1-ylmethyl) anilino)-1- (4- (2-methoxycarbonylethyl)phenyl)methylene]-6-fluoro-2-indolinone
(95) 3-Z-[1-(4-(N-(2-dimethylaminoethyl) -N-methylaminomethyl)anilino)-1-(4-(2-methoxycarbonylethyl)phenyl)methylene]-6-fluoro-2-indolinone
(96) 3-Z-[1-(4-(N-(2-dimethylaminoethyl) -N-methylaminomethyl)anilino)-1-(3-(2-ethoxycarbonylethyl)phenyl)methylene]-6-fluoro-2-indolinone
(97) 3-Z-[1-(4-(pyrrolidin-1-ylmethyl)anilino)-1-(4-(2-methoxycarbonylethyl)phenyl)methylene]-6-chloro-2-indolinone
(98) 3-Z-[lanilino-1-(3-(2-ethoxycarbonylethyl)phenyl)methylene]-6-fluoro-2-indolinone
(99) 3-Z-[1-(4-(N-tert-butoxycarbonylaminomethyl) anilino)-1-(3-(2-ethoxycarbonylethyl)phenyl)methylene]-6-fluoro-2-indolinone
(100) 3-Z-[1-(4-(N-tert-butoxycarbonylmethylaminomethyl)anilino)-1-(3-(2-ethoxycarbonylethyl)phenyl)methylene]-6-fluoro-2-indolinone

(101) 3-Z- [1- (4-dimethylaminomethylanilino) -1- (3-methoxycarbonylmethoxy-phenyl)methylene]-6-fluoro-2-indolinone
(102) 3-Z-[1-(4-dimethylaminomethylanilino)-1-(4-methoxycarbonylmethoxy-phenyl)methylene]-6-fluoro-2-indolinone
(103) 3-Z-[1-(4-dimethylaminomethylanilino)-1-(3-(2-ethoxycarbonyl-ethoxy)phenyl)methylene]-6-fluoro-2-indolinone
(104) 3-Z-[1-(4-(pyrrolidin-1-ylmethyl)anilino)-1-(4-(2-methoxycarbonylethyl)phenyl)methylene]-6-bromo-2-indolinone
(105) 3-Z-[1-(4-(dimethylaminomethyl) anilino)-1-(4-(2-methoxycarbonylethyl)phenyl)methylene]-6-bromo-2-indolinone
(106) 3-Z-[1-(4-(diethylaminomethyl)anilino)-1-(4-(2-methoxycarbonylethyl)phenyl)methylene]-6-bromo-2-indolinone
(107) 3-Z-[1-(3-dimethylaminomethylanilino)-1-(4- (2-methoxycarbonylethyl)phenyl)methylene]-6-fluoro-2-indolinone
(108) 3-Z-[1-(3-dimethylaminomethylanilino)-1-(3-(2-ethoxycarbonylethyl)phenyl)methylene]-6-fluoro-2-indolinone
(109) 3-Z-[1-(3-dimethylaminomethylanilino)-1-(4- (2-methoxycarbonylethyl)phenyl)methylene]-6-chloro-2-indolinone
(110) 3-Z-[1-(4-(dimethylaminomethyl)anilino)-1-(3,4-dimethoxyphenyl)-methylene]-6-cyano-2-indolinone
(111) 3-Z-[1-(4-(N-methyl-N-methylsulphonylamino)anilino)-1-(3-(2-methoxycarbonylvinyl)phenyl)methylene]-6-chloro-2-indolinone
(112) 3-Z-[1-(4-(dimethylaminomethyl) anilino)-1- (4- (2-methoxycarbonyl-vinyl)phenyl)methylene]-6-chloro-2-indolinone
(113) 3-Z-[1-(4-(dimethylaminomethyl) anilino)-1- (4- (2-carbamoyl-vinyl)phenyl)methylene]-6-fluoro-2-indolinone
(114) 3-Z-[1-(4-(dimethylaminomethyl)anilino)-1-(4-(2-methoxycarbonyl-vinyl)phenyl)methylene]-6-fluoro-2-indolinone
(115) 3-Z-[1-(4-(dimethylaminomethyl) anilino)-1-(3-(2-methoxycarbonyl-vinyl)phenyl)methylene]-6-fluoro-2-indolinone
(116) 3-Z-[1-(4-dimethylaminomethylanilino)-1-(3- (2-methoxycarbonylethyl)phenyl)methylene]-6-chloro-2-indolinone
(117) 3-Z-[1-(4-(N-methyl-N-methylsulphonylamino)anilino)-1-(3-(2-methoxycarbonylethyl)phenyl)methylene]-6-chloro-2-indolinone
(118) 3-Z-[1-(4-dimethylaminomethylanilino)-1-(4- (2-carbamoyl-ethyl)phenyl)methylene]-6-fluoro-2-indolinone
(119) 3-Z-[1-(4-dimethylaminomethylanilino)-1-(4-(2-methoxycarbonylethyl)phenyl)methylene]-6-fluoro-2-indolinone
(120) 3-Z-[1-(4-dimethylaminomethylanilino)-1-(3- (2-methoxycarbonylethyl)phenyl)methylene]-6-fluoro-2-indolinone
(121) 3-Z-[1-(4-dimethylaminomethylanilino)-1-(4-aminomethylphenyl)-methylene]-6-chloro-2-indolinone
(122) 3-Z-[1-(4-(N-((4-methylpiperazin-1-yl)methylcarbonyl)-N-methylamino)anilino)-1-(4-aminomethylphenyl)methylene]-6-chloro-2-indolinone
(123) 3-Z-[1-(4-(dimethylaminomethyl)anilino)-1-(3-aminomethylphenyl)-methylene]-6-fluoro-2-indolinone
(124) 3-Z-[1-(4-(dimethylaminomethyl)anilino)-1-(3-(2-aminoethyl)phenyl)methylene]-6-fluoro-2-indolinone
(125) 3-Z-[1-(4-(dimethylaminomethyl)anilino)-1-(4-aminomethylphenyl)-methylene]-6-fluoro-2-indolinone
(126) 3-Z-[1-(4-(N-(4-methylpiperazin-1-ylmethylcarbonyl)-N-methylamino)anilino)-1-(4-aminomethylphenyl)methylene]-6-fluoro-2-indolinone
(127) 3-Z-[1-(4-(methylaminomethyl) anilino)-1-(4-(2-carboxyethyl)phenyl)methylene]-6-fluoro-2-indolinone
(128) 3-Z-[1-(4-(methylaminomethyl) anilino)-1-(4-(2-methylcarbamoyl-ethyl)phenyl)methylene]-6-fluoro-2-indolinone
(129) 3-Z-[1-(4-(N-(4-methylpiperazin-1-ylmethylcarbonyl)-N-methylamino) anilino)-1-(3-aminomethylphenyl)methylene]-6-fluoro-2-indolinone
(130) 3-Z-[1-(4-(aminomethyl)anilino)-1-(4-(2-methoxycarbonylethyl)phenyl)methylene]-6-fluoro-2-indolinone
(131) 3-Z-[1-(4-(aminomethyl)anilino)-1-(3-(2-ethoxycarbonylethyl)phenyl)methylene]-6-fluoro-2-indolinone
(132) 3-Z-[1-(4-(methylaminomethyl) anilino)-1-(3-(2-ethoxycarbonylethyl)phenyl)methylene]-6-fluoro-2-indolinone
(133) 3-Z-[1-(4-dimethylaminomethylanilino)-1-(3-(2-carboxyethyl)phenyl)methylene]-6-chloro-2-indolinone
(134) 3-Z-[1-(4-dimethylaminomethylanilino)-1-(4-(2-carboxyethyl)phenyl)methylene]-6-fluoro-2-indolinone
(135) 3-Z-[1-(4-dimethylaminomethylanilino)-1-(3-carboxymethylphenyl)-methylene]-6-fluoro-2-indolinone
(136) 3-Z-[1-(4-dimethylaminomethylanilino)-1-(3- (2-carboxyethyl)phenyl)methylene]-6-fluoro-2-indolinone
(137) 3-Z-[1-(4-dimethylaminomethylanilino)-1-(4-carboxymethylphenyl)-methylene]-6-fluoro-2-indolinone
(138) 3-Z-[1-(4-(N-(4-methylpiperazin-1-ylmethylcarbonyl)-N-methylamino)anilino)-1-(4-carboxymethylphenyl)methylene]-6-fluoro-2-indolinone
(139) 3-Z-[1-(4-(N-(2-dimethylaminoethyl)-N-methylsulphonylamino)anilino)-1-(4-carboxymethylphenyl)methylene]-6-fluoro-2-indolinone
(140) 3-Z-[1-(4-(N-methyl-N-acetylamino)anilino)-1-(4-(2-carboxyethyl)phenyl)methylene]-6-fluoro-2-indolinone
(141) 3-Z-[1-(4-(N-(4-methylpiperazin-1-ylmethylcarbonyl)-N-methylamino)anilino)-1-(4-(2-carboxyethyl)phenyl)methylene]-6-fluoro-2-indolinone
(142) 3-Z-[1-(4-(N-(2-dimethylaminoethyl)-N-methylsulphonylamino)anilino)-1-(4-(2-carboxyethyl)phenyl)methylene]-6-fluoro-2-indolinone
(143) 3-Z-[1-(4-(N-(3-dimethylaminopropyl)-N-acetylamino) anilino) -1- (4- (2-carboxyethyl)phenyl)methylene]-6-fluoro-2-indolinone
(144) 3-Z-[1-(4-(N-tert-butoxycarbonylmethylaminomethyl)anilino)-1-(4-(2-carboxyethyl)phenyl)methylene]-6-fluoro-2-indolinone
(145) 3-Z-[1-(4-(4-methylpiperazin-1-ylcarbonyl)anilino)-1-(4-(2-carboxyethyl)phenyl)methylene]-6-fluoro-2-indolinone
(146) 3-Z-[1-(4-(1-methylimidazol-2-yl)anilino)-1-(4-(2-carboxyethyl)phenyl)methylene]-6-fluoro-2-indolinone
(147) 3-Z-[1-(4-methylsulphonylanilino)-1-(4-(2-carboxyethyl)phenyl)methylene]-6-fluoro-2-indolinone
(148) 3-Z-[1-(4-(4-methylpiperazin-1-ylcarbonyl)anilino)-1-(3-carboxymethylphenyl)methylene]-6-fluoro-2-indolinone
(149) 3-Z-[1-(4-(N-(4-methylpiperazin-1-ylmethylcarbonyl)-N-methylamino)anilino)-1-(3-carboxymethylphenyl)methylene]-6-fluoro-2-indolinone
(150) 3-Z-[1-(4-(N-(dimethylaminomethylcarbonyl)-N-methylamino)anilino)-1-(3-carboxymethylphenyl)methylene]-6-fluoro-2-indolinone

(151) 3-Z-[1-(4-(N-(4-dimethylaminobutylcarbonyl)-N-methylamino)anilino)-1-(3-carboxymethylphenyl)methylene]-6-fluoro-2-indolinone
(152) 3-Z-[1-Anilino-1-(3-carboxymethylphenyl)-methylene]-6-fluoro-2-indolinone
(153) 3-Z-[1-(4-methylsulphonylanilino)-1-(3-carboxymethylphenyl)-methylene]-6-fluoro-2-indolinone
(154) 3-Z-[1-(4-(1-methylimidazol-2-yl)anilino)-1-(3-carboxymethylphenyl)-methylene]-6-fluoro-2-indolinone
(155) 3-Z-[1-(4-(N-(dimethylaminocarbonylmethyl)-N-methylsulphonylamino)anilino)-1-(3-carboxymethylphenyl)-methylene]-6-fluoro-2-indolinone
(156) 3-Z-[lanilino-1-(4-carboxymethylphenyl)methylene]-6-fluoro-2-indolinone
(157) 3-Z-[1-(4-(1-methylimidazol-2-yl)anilino)-1-(4-carboxymethylphenyl)-methylene]-6-
(158) fluoro-2-indolinone
(159) 3-Z-[1-(4-(N-(3-dimethylaminopropylcarbonyl)-N-methylamino)aniline)-1-(4-carboxymethylphenyl)methylene]-6-fluoro-2-indolinone
(160) 3-Z-[1-(4-(N-(dimethylaminomethylcarbonyl)-N-methylamino)aniline)-1-(4-carboxymethylphenyl)methylene]-6-fluoro-2-indolinone
(161) 3-Z-[1-(4-(4-methylpiperazin-1-yl-carbonyl)aniline)-1-(4-carboxymethylphenyl)methylene]-6-fluoro-2-indolinone
(162) 3-Z-[1-(4-methylsulphonylanilino)-1-(4-carboxymethylphenyl)-methylene]-6-fluoro-2-indolinone
(163) 3-Z-[1-(4-(N-methyl-N-acetylamino)anilino)-1-(4-carboxymethylphenyl)-methylene]-6-fluoro-2-indolinone
(164) 3-Z-[1-(4-(N-(dimethylaminocarbonylmethyl)-N-methylsulphonylamino)anilino)-1-(4-carboxymethylphenyl)methylene]-6-fluoro-2-indolinone
(165) 3-Z-[1-(4-(N-(2-dimethylaminoethylcarbonyl)-N-methylamino)anilino)-1-(4-carboxymethylphenyl)methylene]-6-fluoro-2-indolinone
(166) 3-Z-[1-(4-(N-(3-dimethylaminopropylcarbonyl)-N-methylamino)anilino)-1-(4-carboxymethylphenyl)methylene]-6-fluoro-2-indolinone
(167) 3-Z-[1-(4-(N-(4-methylpiperazin-1-ylmethylcarbonyl)-N-methylamino)anilino)-1-(3-(2-carboxyethyl)phenyl)methylene]-6-fluoro-2-indolinone
(168) 3-Z-[1-(4-(N-(2-dimethylaminoethyl)-N-methylsulphonylamino)anilino)-1-(3-carboxymethylphenyl)methylene]-6-fluoro-2-indolinone
(169) 3-Z-[1-(4-(N-(2-dimethylaminoethyl)-N-methylsulphonylamino)anilino)-1-(3-(2-carboxyethyl)phenyl)methylene]-6-fluoro-2-indolinone
(170) 3-Z-[1-(4-(N-(dimethylaminomethylcarbonyl)-N-methylamino)anilino)-1-(3-(2-carboxyethyl)phenyl)methylene.]-6-fluoro-2-indolinone
(171) 3-Z-[1-(4-(N-(3-dimethylaminopropyl)-N-acetylamino)anilino)-1-(3-(2-carboxyethyl)phenyl)methylene]-6-fluoro-2-indolinone
(172) 3-Z-[1-(4-(N-(dimethylaminomethylcarbonyl)-N-methylamino)anilino)-1-(4-(2-carboxyethyl)phenyl)methylene]-6-fluoro-2-indolinone
(173) 3-Z-[1-(4-(2-dimethylaminoethyl)anilino)-1-(4-(2-carboxyethyl)phenyl)methylene]-6-fluoro-2-indolinone
(174) 3-Z-[1-(4-(N-(2-dimethylaminoethylcarbonyl)-N-methylamino)anilino)-1-(4-(2-carboxyethyl)phenyl)methylene]-6-fluoro-2-indolinone
(175) 3-Z-[1-(4-(2-dimethylaminoethyl)anilino)-1-(3-(2-carboxyethyl)phenyl)methylene]-6-fluoro-2-indolinone
(176) 3-Z-[1-(4-(N-(2-dimethylaminoethyl)-N-acetylamino)anilino)-1-(4-(2-carboxyethyl)phenyl)methylene]-6-fluoro-2-indolinone
(177) 3-Z-[1-(4-(N-(3-dimethylaminopropylcarbonyl)-N-methylamino)anilino)-1-(3-(2-carboxyethyl)phenyl)methylene]-6-fluoro-2-indolinone
(178) 3-Z-[1-(4-(N-(4-dimethylamino-butylcarbonyl)-N-methylamino)anilino)-1-(3-(2-carboxyethyl)phenyl)methylene]-6-fluoro-2-indolinone
(179) 3-Z-[1-(4-(1-methylimidazol-2-yl)anilino)-1-(3-(2-carboxyethyl)phenyl)methylene]-6-fluoro-2-indolinone
(180) 3-Z-[1-(4-(N-(4-dimethylaminobutylcarbonyl)-N-methylamino)anilino)-1-(4-(2-carboxyethyl)phenyl)methylene]-6-fluoro-2-indolinone
(181) 3-Z-[lanilino-1-(4-(2-carboxyethyl)phenyl)methylene]-6-fluoro-2-indolinone
(182) 3-Z-[1-(4-(pyrrolidin-1-ylmethyl)anilino)-1-(4-(2-carboxyethyl)phenyl)methylene]-6-fluoro-2-indolinone
(183) 3-Z-[1-(4-(diethylaminomethyl)anilino)-1-(4-(2-carboxyethyl)phenyl)methylene]-6-fluoro-2-indolinone
(184) 3-Z-[1-(4-aminomethylanilino)-1-(4-(2-carboxyethyl)phenyl)methylene]-6-fluoro-2-indolinone
(185) 3-Z-[1-(4-(2-dimethylaminoethyl)anilino)-1-(3-carboxymethylphenyl)-methylene]-6-fluoro-2-indolinone
(186) 3-Z-[1-(4-(2-dimethylaminoethyl)anilino)-1-(4-carboxymethylphenyl)-methylene]-6-fluoro-2-indolinone
(187) 3-Z-[1-(4-(2-dimethylaminoethyl)anilino)-1-(4-(2-carboxyethyl)phenyl)methylene]-6-chloro-2-indolinone
(188) 3-Z-[1-(4-(1-methylimidazol-2-yl)anilino)-1-(4-(2-carboxyethyl)phenyl)methylene]-6-chloro-2-indolinone
(189) 3-Z-[1-(4-dimethylaminomethylanilino)-1-(4-(2-carboxyethyl)phenyl)methylene]-6-chloro-2-indolinone
(190) 3-Z-[1-(4-((4-methylpiperazin-1-yl)methyl)anilino)-1-(3-(2-carboxyethyl)phenyl)methylene]-6-fluoro-2-indolinone
(191) 3-Z-[1-(4-(imidazol-1-ylmethyl)anilino)-1-(3-(2-carboxyethyl)phenyl)methylene]-6-fluoro-2-indolinone
(192) 3-Z-[1-(4-(N-(2-dimethylaminoethyl)-N-methylaminomethyl)anilino)-1-(3-(2-carboxyethyl)phenyl)methylene]-6-fluoro-2-indolinone
(193) 3-Z-[1-(4-((4-methylpiperazin-1-yl)methyl)anilino)-1-(4-(2-carboxyethyl)phenyl)methylene]-6-fluoro-2-indolinone
(194) 3-Z-[1-(4-(imidazol-1-ylmethyl) anilino)-1- (4- (2-carboxyethyl)phenyl)methylene]-6-fluoro-2-indolinone
(195) 3-Z-[1-(4-(pyrrolidin-1-ylmethyl)anilino)-1-(4-(2-carboxyethyl)phenyl)methylene]-6-chloro-2-indolinone
(196) 3-Z-[1-(4-(N-(2-dimethylaminoethyl)-N-methylaminomethyl)anilino)-1-(4-(2-carboxyethyl)phenyl)methylene]-6-fluoro-2-indolinone
(197) 3-Z-[lanilino-1-(3-(2-carboxyethyl)phenyl)methylene]-6-fluoro-2-indolinone
(198) 3-Z-[1-(4-aminomethylanilino)-1-(3-(2-carboxyethyl)phenyl)methylene]-6-fluoro-2-indolinone
(199) 3-Z-[1-(4-methylaminomethylanilino)-1-(3-(2-carboxyethyl)phenyl)methylene]-6-fluoro-2-indolinone

(200) 3-Z-[1-(4-dimethylaminomethylanilino)-1-(3-carboxymethoxy-phenyl)-methylene]-6-fluoro-2-indolinone
(201) 3-Z-[1-(4-dimethylaminomethylanilino)-1-(4-carboxymethoxy-phenyl)phenyl)methylene]-6-fluoro-2-indolinone
(202) 3-Z-[1-(4-(pyrrolidin-1-ylmethyl)anilino)-1-(4-(2-carboxyethyl)phenyl)methylene]-6-bromo-2-indolinone
(203) 3-Z-[1-(4-(dimethylaminomethyl) anilino)-1-(4-(2-carboxyethyl)phehyl)methylene]-6-bromo-2-indolinone
(204) 3-Z-[1-(4-(diethylaminomethyl)anilino)-1-(4-(2-carboxyethyl)-methylene]-6-bromo-2-indolinone
(205) 3-Z-[1-(3-dimethylaminomethylanilino)-1-(4-(2-carboxyethyl)phenyl)methylene]-6-fluoro-2-indolinone
(206) 3-Z-[1-(3-dimethylaminomethylanilino)-1-(3-(2-carboxyethyl)phenyl)methylene]-6-fluoro-2-indolinone
(207) 3-Z-[1-(3-dimethylaminomethylanilino)-1-(4-(2-carboxyethyl)phenyl)methylene]-6-chloro-2-indolinone
(208) 3-Z-[1-(4-dimethylaminomethylanilino)-1-(3-(2-carbamoyl-ethyl)phenyl)methylene]-6-chloro-2-indolinone
(209) 3-Z-[1-(4-dimethylaminomethylanilino)-1-(3-(2-methylcarbamoyl-ethyl)phenyl)methylene]-6-chloro-2-indolinone
(210) 3-Z-[1-(4-dimethylaminomethylanilino)-1-(4-(2-methylcarbamoyl-ethyl)phenyl)methylene]-6-fluoro-2-indolinone
(211) 3-Z-[1-(4-dimethylaminomethylanilino)-1-(3-dimethylcarbamoylmethylphenyl)methylene]-6-fluoro-2-indolinone
(212) 3-Z-[1-(4-dimethylaminomethylanilino)-1-(3-(2-carbamoyl-ethyl)phenyl)methylene]-6-fluoro-2-indolinone
(213) 3-Z-[1-(4-dimethylaminomethylanilino)-1-(3-(2-methylcarbamoyl-ethyl)phenyl)methylene]-6-fluoro-2-indolinone
(214) 3-Z-[1-(4-dimethylaminomethylanilino)-1-(3-(2-dimethylcarbamoyl-ethyl)phenyl)methylene]-6-fluoro-2-indolinone
(215) 3-Z-[1-(4-dimethylaminomethylanilino)-1-(3-carbamoylmethylphenyl)-methylene]-6-fluoro-2-indolinone
(216) 3-Z-[1-(4-dimethylaminomethylanilino)-1-(3-methylcarbamoylmethylphenyl)methylene]-6-fluoro-2-indolinone
(217) 3-Z-[1-(4-dimethylaminomethylanilino)-1-(4-carbamoylmethylphenyl)-methylene]-6-fluoro-2-indolinone
(218) 3-Z-[1-(4-dimethylaminomethylanilino)-1-(4-(2-dimethylcarbamoyl-ethyl)phenyl)methylene]-6-fluoro-2-indolinone
(219) 3-Z-[1-(4-dimethylaminomethylanilino)-1-(4-(2-(4-methylpiperazin-1-yl-carbonyl)ethyl)phenyl)methylene]-6-fluoro-2-indolinone
(220) 3-Z-[1-(4-(N-(4-methylpiperazin-1-ylmethylcarbonyl)-N-methylamino)anilino)-1-(4-carbamoylmethylphenyl)methylene]-6-fluoro-2-indolinone
(221) 3-Z-[1-(4-(N-(2-dimethylaminoethyl)-N-methylsulphonylamino)anilino)-1-(4-carbamoylmethylphenyl)methylene]-6-fluoro-2-indolinone
(222) 3-Z-[1-(4-dimethylaminomethylanilino)-1-(4-dimethylcarbamoylmethylphenyl)methylene]-6-fluoro-2-indolinone
(223) 3-Z-[1-(4-dimethylaminomethylanilino)-1-(4-methylcarbamoylmethylphenyl)methylene]-6-fluoro-2-indolinone
(224) 3-Z-[1-(4-(N-(2-dimethylaminoethyl)-N-methylsulphonylamino)anilino)-1-(4-methylcarbamoylmethylphenyl)methylene]-6-fluoro-2-indolinone
(225) 3-Z-[1-(4-(N-(2-dimethylaminoethyl)-N-methylsulphonylamino)anilino)-1-(4-dimethylcarbamoylmethylphenyl)methylene]-6-fluoro-2-indolinone
(226) 3-Z-[1-(4-(N-(4-methylpiperazin-1-ylmethylcarbonyl)-N-methylamino)anilino)-1-(4-methylcarbamoylmethylphenyl)methylene]-6-fluoro-2-indolinone
(227) 3-Z-[1-(4-(N-methyl-N-acetylamino)anilino)-1-(4-(2-methylcarbamoyl-ethyl)phenyl)methylene]-6-fluoro-2-indolinone
(228) 3-Z-[1-(4-(N-(4-methylpiperazin-1-ylmethylcarbonyl)-N-methylamino)anilino)-1-(4-(2-methylcarbamoyl-ethyl)phenyl)methylene]-6-fluoro-2-indolinone
(229) 3-Z-[1-(4-(N-(2-dimethylaminoethyl)-N-methylsulphonylamino)anilino)-1-(4-(2-methylcarbamoylethyl)phenyl)methylene]-6-fluoro-2-indolinone
(230) 3-Z-[1-(4-(N-tert-butoxycarbonylmethylaminomethyl)anilino)-1-(4-(2-methylcarbamoylethyl)phenyl)methylene]-6-fluoro-2-indolinone
(231) 3-Z-[1-(4-(1-methylimidazol-2-yl)anilino)-1-(4-(2-methylcarbamoyl-ethyl)phenyl)methylene]-6-fluoro-2-indolinone
(232) 3-Z-[1-(4-methylsulphonylanilino)-1-(4-(2-methylcarbamoyl-ethyl)phenyl)methylene]-6-fluoro-2-indolinone
(233) 3-Z-[1-(4-(4-methylpiperazin-1-ylcarbonyl)anilino)-1-(4-(2-methylcarbamoylethyl)phenyl)methylene]-6-fluoro-2-indolinone
(234) 3-Z-[1-(4-(4-methylpiperazin-1-ylcarbonyl)anilino)-1-(3-methylcarbamoylmethylphenyl)methylene]-6-fluoro-2-indolinone
(235) 3-Z-[1-(4-(N-(4-methylpiperazin-1-ylmethylcarbonyl)-N-methylamino)anilino)-1-(3-methylcarbamoylmethylphenyl)methylene]-6-fluoro-2-indolinone
(236) 3-Z-[1-(4-dimethylaminomethylanilino)-1-(4-acetylaminomethylphenyl)-methylene]-6-chloro-2-indolinone
(237) 3-Z-[1-(4-(N-(4-methylpiperazin-1-ylmethylcarbonyl)-N-methylamino)anilino)-1-(4-acetylaminomethylphenyl)methylene]-6-chloro-2-indolinone
(238) 3-Z-[1-(4-dimethylaminomethylanilino)-1-(4-benzoylaminophenyl)-methylene]-6-chloro-2-indolinone
(239) 3-Z-[1-(4-(N-(4-methylpiperazin-1-ylmethylcarbonyl)-N-methylamino)anilino)-1-(4-benzoylaminomethylphenyl)methylene]-6-chloro-2-indolinone
(240) 3-Z-[1-(4-dimethylaminomethylanilino)-1-(3-acetylaminomethylphenyl)-methylene]-6-fluoro-2-indolinone
(241) 3-Z-[1-(4-dimethylaminomethylanilino)-1-(3-propionylaminomethylphenyl)methylene]-6-fluoro-2-indolinone
(242) 3-Z-[1-(4-dimethylaminomethylanilino)-1-(3-benzoylaminomethylphenyl)methylene]-6-fluoro-2-indolinone
(243) 3-Z-[1-(4-dimethylaminomethylanilino)-1-(3-phenylacetylaminomethylphenyl)methylene]-6-fluoro-2-indolinone
(244) 3-Z-[1-(4-dimethylaminomethylanilino)-1-(3-(2-acetylaminoethyl)phenyl)methylene]-6-fluoro-2-indolinone
(245) 3-Z-[1-(4-dimethylaminomethylanilino)-1-(3-(2-benzoylaminoethyl)phenyl)methylene]-6-fluoro-2-indolinone
(246) 3-Z-[1-(4-dimethylaminomethylanilino)-1-(3-(2-propionylaminoethyl)phenyl)methylene]-6-fluoro-2-indolinone
(247) 3-Z-[1-(4-dimethylaminomethylanilino)-1-(3-(2-phenylacetylaminoethyl)phenyl)methylene]-6-fluoro-2-indolinone
(248) 3-Z-[1-(4-dimethylaminomethylanilino)-1-(4-acetylaminomethylphenyl)-methylene]-6-fluoro-2-indolinone
(249) 3-Z-[1-(4-dimethylaminomethylanilino)-1-(4-propionylaminomethylphenyl)methylene]-6-fluoro-2-indolinone
(250) 3-Z-[1-(4-dimethylaminomethylanilino)-1-(4-phenylacetylaminomethylphenyl)methylene]-6-fluoro-2-indolinone

(251) 3-Z-[1-(4-(N-(4-methylpiperazin-1-ylmethylcarbonyl)-N-methylamino)anilino)-1-(4-acetylaminomethylphenyl)methylene]-6-fluoro-2-indolinone
(252) 3-Z-[1-(4-(N-(4-methylpiperazin-1-ylmethylcarbonyl)-N-methylamino)anilino)-1-(4-propionylaminomethylphenyl)methylene]-6-fluoro-2-indolinone
(253) 3-Z-[1-(4-(N-(4-methylpiperazin-1-ylmethylcarbonyl)-N-methylamino)anilino)-1-(9-phenylacetylaminomethylphenyl)-methylene]-6-fluoro-2-indolinone
(254) 3-Z-[1-(4-(N-(4-methylpiperazin-1-ylmethylcarbonyl)-N-methylamino)anilino)-1-(3-cyclopropylcarbonylaminomethylphenyl)-methylene]-6-fluoro-2-indolinone
(255) 3-Z-[1-(4-(N-(4-methylpiperazin-1-ylmethylcarbonyl)-N-methylamino)anilino)-1-(3-cyclobutylcarbonylaminomethylphenyl)-methylene]-6-fluoro-2-indolinone
(256) 3-Z-[1-(4-(N-(4-methylpiperazin-1-ylmethylcarbonyl)-N-methylamino)anilino)-1-(3-(pyridin-2-yl-carbonylaminomethyl)phenyl)methylene]-6-fluoro-2-indolinone
(257) 3-Z-[1-(4-(N-(4-methylpiperazin-1-ylmethylcarbonyl)-N-methylamino)anilino)-1-(3-cyclohexylcarbonylaminomethylphenyl)-methylene]-6-fluoro-2-indolinone
(258) 3-Z-[1-(4-(N-(4-methylpiperazin-1-ylmethylcarbonyl)-N-methylamino)anilino)-1-(3-(pyridin-3-yl-carbonylaminomethyl)phenyl)methylene]-6-fluoro-2-indolinone
(259) 3-Z-[1-(4-(N-(4-methylpiperazin-1-ylmethylcarbonyl)-N-methylamino)anilino)-1-(3-isobutyrylaminomethylphenyl)methylene]-6-fluoro-2-indolinone
(260) 3-Z-[1-(4-(N-(4-methylpiperazin-1-ylmethylcarbonyl)-N-methylamino)anilino)-1-(3-(3-methylbutyrylaminomethylphenyl)-methylene]-6-fluoro-2-indolinone
(261) 3-Z-[1-(4-(N-(4-methylpiperazin-1-ylmethylcarbonyl)-N-methylamino) aniline)-1-(3-cyclohexylmethylcarbonylaminomethylphenyl)methylene]-6-fluoro-2-indolinone
(262) 3-Z-[1-(4-(N-(4-methylpiperazin-1-ylmethylcarbonyl)-N-methylamino)anilino)-1-(3-methoxyacetylaminomethylphenyl)-methylene]-6-fluoro-2-indolinone
(263) 3-Z-[1-(4-(N-(4-methylpiperazin-1-ylmethylcarbonyl)-N-methylamino)anilino)-1-(3-(2-methoxybenzoyl-aminomethyl)phenyl)methylene]-6-fluoro-2-indolinone
(264) 3-Z-[1-(4-(N-(4-methylpiperazin-1-ylmethylcarbonyl)-N-methylamino)anilino)-1-(3-tert-butylacetylaminomethylphenyl)-methylene]-6-fluoro-2-indolinone
(265) 3-Z-[1-(4-(N-(4-methylpiperazin-1-ylmethylcarbonyl)-N-methylamino)anilino)-1-(3-(2-thiophen-carbonylaminomethyl)phenyl)methylene]-6-fluoro-2-indolinone
(266) 3-Z-[1-(4-(N-(4-methylpiperazin-1-ylmethylcarbonyl)-N-methylamino)anilino)-1-(3-pivaloylaminomethylphenyl)methylene]-6-fluoro-2-indolinone
(267) 3-Z-[1-(4-(N-(4-methylpiperazin-1-ylmethylcarbonyl)-N-methylamino)anilino)-1-(3-(2-furoylaminomethyl)phenyl)methylene]-6-fluoro-2-indolinone
(268) 3-Z-[1-(4-(N-(4-methylpiperazin-1-ylmethylcarbonyl)-N-methylamino)anilino)-1-(3-acetylaminomethylphenyl)methylene]-6-fluoro-2-indolinone
(269) 3-Z-[1-(4-(N-(4-methylpiperazin-1-ylmethylcarbonyl)-N-methylamino)anilino)-1-(3-propionylaminomethylphenyl)methylene]-6-fluoro-2-indolinone
(270) 3-Z-[1-(4-(N-(4-methylpiperazin-1-ylmethylcarbonyl)-N-methylamino)anilino)-1-(3-benzoylaminomethylphenyl)methylene]-6-fluoro-2-indolinone
(271) 3-Z-[1-(4-(N-(4-methylpiperazin-1-ylmethylcarbonyl)-N-methylamino)anilino)-1-(3-phenylacetylaminomethylphenyl)-methylene]-6-fluoro-2-indolinone
(272) 3-Z-[1-(4-dimethylaminomethylanilino)-1-(3-cyclopropylcarbonylaminomethylphenyl)methylene]-6-fluoro-2-indolinone
(273) 3-Z-[1-(4-dimethylaminomethylanilino)-1-(3-cyclobutylcarbonylaminomethylphenyl)methylene]-6-fluoro-2-indolinone
(274) 3-Z-[1-(4-dimethylaminomethylanilino)-1-(3-(pyridin-2-yl-carbonylaminomethyl)phenyl)methylene]-6-fluoro-2-indolinone
(275) 3-Z-[1-(4-dimethylaminomethylanilino)-1-(3-cyclohexylcarbonylaminomethylphenyl)methylene]-6-fluoro-2-indolinone
(276) 3-Z-[1-(4-dimethylaminomethylanilino)-1-(3-(pyridin-3-yl-carbonylaminomethyl)phenyl)methylene]-6-fluoro-2-indolinone
(277) 3-Z-[1-(4-dimethylaminomethylanilino)-1-(3-isobutyrylaminomethylphenyl)methylene]-6-fluoro-2-indolinone
(278) 3-Z-[1-(4-dimethylaminomethylanilino)-1-(3-(3-methylbutyryl-aminomethylphenyl)methylene]-6-fluoro-2-indolinone
(279) 3-Z-[1-(4-dimethylaminomethylanilino)-1-(3-cyclohexylmethylcarbonylaminomethylphenyl)methylene]-6-fluoro-2-indolinone
(280) 3-Z-[1-(4-dimethylaminomethylanilino)-1-(3-methoxyacetylaminomethylphenyl)methylene]-6-fluoro-2-indolinone
(281) 3-Z-[1-(4-dimethylaminomethylanilino)-1-(3-(2-methoxybenzoyl-aminomethyl)phenyl)methylene]-6-fluoro-2-indolinone
(282) 3-Z-[1-(4-dimethylaminomethylanilino)-1-(3-tert-butylacetylaminomethylphenyl)methylene]-6-fluoro-2-indolinone
(283) 3-Z-[1-(4-dimethylaminomethylanilino)-1-(3-(2-thiophenecarbonylaminomethyl)phenyl)methylene]-6-fluoro-2-indolinone
(284) 3-Z-[1-(4-dimethylaminomethylanilino)-1-(3-pivaloylaminomethylphenyl)methylene]-6-fluoro-2-indolinone
(285) 3-Z-[1-(4-dimethylaminomethylanilino)-1-(3-(2-furoylaminomethyl)phenyl)methylene]-6-fluoro-2-indolinone
(286) 3-Z-[1-(4-dimethylaminomethylanilino)-1-(3-(pyridin-4-yl-carbonylaminomethyl)phenyl)methylene]-6-fluoro-2-indolinone
(287) 3-Z-[1-(4-trimethylammoniummethylanilino)-1-(4-(2-carboxyethyl)phenyl)methylene]-6-fluoro-2-indolinone iodide
(288) 3-Z-[1-(4-trimethylammoniummethylanilino)-1-(3-(2-carboxyethyl)phenyl)methylene]-6-fluoro-2-indolinone iodide
(289) 3-Z-[1-(4-guanidinomethylanilino)-1-(4-(2-carboxyethyl)phenyl)methylene]-6-fluoro-2-indolinone
(290) 3-Z-[1-(4-guanidinomethylanilino)-1-(3-(2-carboxyethyl)phenyl)methylene]-6-fluoro-2-indolinone
(291) 3-Z-[1-(4-(N-(2-dimethylaminoethyl)-N-methylsulphonylamino)anilino)-1-(4-(2-carboxyethyl)phenyl)methylene]-6-chloro-2-indolinone
(292) 3-Z-[1-(4-(N-(dimethylaminomethylcarbonyl)-N-methylamino)aniline)-1-(4-(2-carboxyethyl)phenyl)methylene]-6-chloro-2-indolinone
(293) 3-Z-[1-(4-(N-(2-dimethylaminoethyl)-N-acetylamino)anilino)-1-(4-(2-carboxyethyl)phenyl)methylene]-6-chloro-2-indolinone
(294) 3-Z-[1-(4-(N-(2-methylaminoethyl)-N-acetylamino)anilino)-1-(4-(2-carboxyethyl)phenyl)methylene]-6-chloro-2-indolinone
(295) 3-Z-[1-(4-(N-(3-dimethylaminopropyl)-N-acetylamino)anilino)-1-(4-(2-carboxyethyl)phenyl)methylene]-6-chloro-2-indolinone
(296) 3-Z-[1-(4-(N-(3-methylaminopropyl)-N-acetylamino)anilino)-1-(4-(2-carboxyethyl)phenyl)methylene]-6-chloro-2-indolinone
(297) 3-Z-[1-(4-(3-dimethylaminopropyl)anilino)-1-(4-(2-carboxyethyl)phenyl)methylene]-6-chloro-2-indolinone
(298) 3-Z-[1-(4-ethylaminomethylanilino)-1-(4-(2-carboxyethyl)phenyl)methylene]-6-chloro-2-indolinone
(299) 3-Z-[1-(4-methylaminomethylanilino)-1-(4-(2-carboxyethyl)phenyl)methylene]-6-chloro-2-indolinone

(300) 3-Z-[1-(4-(N-(4-methylpiperazin-1-ylmethylcarbonyl)-N-methylamino)anilino)-1-(4-(2-carboxyethyl)phenyl)methylene]-6-chloro-2-indolinone
(301) 3-Z-[1-(4-(4-methylpiperazin-1-ylcarbonyl)anilino)-1-(4-(2-carboxyethyl)phenyl)methylene]-6-chloro-2-indolinone
(302) 3-Z-[1-(4-(N-(3-dimethylaminopropyl)-N-methylsulphonylamino)anilino)-1-(4-(2-carboxyethyl)phenyl)methylene]-6-chloro-2-indolinone
(303) 3-Z-[1-(4-(N-(2-dimethylaminoethyl)-N-propylsulphonylamino)anilino)-1-(4-(2-carboxyethyl)phenyl)methylene]-6-chloro-2-indolinone
(304) 3-Z-[1-(4-aminomethylanilino)-1-(4-(2-carboxyethyl)phenyl)methylene]-6-chloro-2-indolinone
(305) 3-Z-[1-(3-(methylaminomethyl)anilino)-1-(4-(2-carboxyethyl)phenyl)methylene]-6-chloro-2-indolinone
(306) 3-Z-[1-(3-(2-dimethylaminoethyl)anilino)-1-(4-(2-carboxyethyl)phenyl)methylene]-6-chloro-2-indolinone
(307) 3-Z-[1-(3-(3-dimethylaminopropyl)anilino)-1-(4-(2-carboxyethyl)phenyl)methylene]-6-chloro-2-indolinone
(308) 3-Z-[1-(4-(N-(dimethylamino-carbonylmethyl)-N-methylsulphonylamino)anilino)-1-(4-(2-carboxyethyl)phenyl)methylene]-6-chloro-2-indolinone
(309) 3-Z-[1-(4-(N-methyl-N-methylsulphonylamino)anilino)-1-(4-(2-carboxyethyl)phenyl)methylene]-6-chloro-2-indolinone
(310) 3-Z-[1-(4-(N-methyl-N-acetylamino)anilino)-1-(4-(2-carboxyethyl)phenyl)methylene]-6-chloro-2-indolinone
(311) 3-Z-[1-(4-(N-(N-(2-dimethylaminoethyl)-N-methylaminomethylcarbonyl)-N-methylamino)anilino)-1-(4-(2-carboxyethyl)phenyl)methylene]-6-chloro-2-indolinone
(312) 3-Z-[1-(4-(2-diethylaminoethylsulphonyl)anilino)-1-(4-(2-carboxyethyl)phenyl)methylene]-6-chloro-2-indolinone
(313) 3-Z-[1-(4-(N-(2-dimethylaminoethyl-carbonyl)-N-methylamino)aniline)-1-(4-(2-carboxyethyl)phenyl)methylene]-6-chloro-2-indolinone
(314) 3-Z-[1-(4-(N-(2-dimethylaminoethyl)-N-methylaminomethyl)anilino)-1-(4-(2-carboxyethyl)-phenyl)methylene]-6-chloro-2-indolinone
(315) 3-Z-[1-(4-(2-dimethylaminoethoxy)anilino)-1-(4-(2-carboxyethyl)phenyl)methylene]-6-chloro-2-indolinone
(316) 3-Z-[1-(4-(N-(4-dimethylaminobutylcarbonyl)-N-methylamino)anilino)-1-(4-(2-carboxyethyl)phenyl)methylene]-6-chloro-2-indolinone
(317) 3-Z-[1-(4-(N-(3-dimethylaminopropylcarbonyl)-N-methylamino)anilino)-1-(4-(2-carboxyethyl)phenyl)methylene]-6-chloro-2-indolinone
(318) 3-Z-[1-(4-(methylethylaminomethyl)anilino)-1-(4-(2-carboxyethyl)phenyl)methylene]-6-chloro-2-indolinone
(319) 3-Z-[1-(4-(methylpropylaminomethyl)anilino)-1-(4-(2-carboxyethyl)phenyl)methylene]-6-chloro-2-indolinone
(320) 3-Z-[1-(4-(methylbenzylaminomethyl)anilino)-1-(4-(2-carboxyethyl)phenyl)methylene]-6-chloro-2-indolinone
(321) 3-Z-[1-(4-(N-(2-dimethylaminoethyl)-N-methylaminomethyl)anilino)-1-(4-(2-carboxyethyl)phenylmethylene]-6-chloro-2-indolinone
(322) 3-Z-[1-(4-(azetidin-1-ylmethyl)anilino)-1-(4-(2-carboxyethyl)phenyl)methylene]-6-chloro-2-indolinone
(323) 3-Z-[1-(4-((4-methylpiperazin-1-yl)methyl)anilino)-1-(4-(2-carboxyethyl)phenyl)methylene]-6-chloro-2-indolinone
(324) 3-Z-[1-(4-(piperazin-1-ylmethyl)anilino)-1-(4-(2-carboxyethyl)phenyl)methylene]-6-chloro-2-indolinone
(325) 3-Z-[1-(4-(morpholin-4-ylmethyl)anilino)-1-(4-(2-carboxyethyl)phenyl)methylene]-6-chloro-2-indolinone
(326) 3-Z-[1-(4-(thiomorpholin-4-ylmethyl)anilino)-1-(4-(2-carboxyethyl)phenyl)methylene]-6-chloro-2-indolinone
(327) 3-Z-[1-(4-(imidazol-1-ylmethyl)anilino)-1-(4-(2-carboxyethyl)phenyl)methylene]-6-chloro-2-indolinone
(328) 3-Z-[1-(4-(N-(2-dimethylaminoethyl)-N-methylsulphonylamino)anilino)-1-(3-(2-carboxyethyl)phenyl)methylene]-6-chloro-2-indolinone
(329) 3-Z-[1-(4-(N-(dimethylaminomethylcarbonyl)-N-methylamino)anilino)-1-(3-(2-carboxyethyl)phenyl)methylene]-6-chloro-2-indolinone
(330) 3-Z-[1-(4-(N-(2-dimethylaminoethyl)-N-acetylamino)anilino)-1-(3-(2-carboxyethyl)phenyl)methylene]-6-chloro-2-indolinone
(331) 3-Z-[1-(4-(N-(2-methylaminoethyl)-N-acetylamino)anilino)-1-(3-(2-carboxyethyl)phenyl)methylene]-6-chloro-2-indolinone
(332) 3-Z-[1-(4-(N-(3-dimethylaminopropyl)-N-acetylamino) anilino) -1- (3- (2-carboxyethyl)phenyl)methylene]-6-chloro-2-indolinone
(333) 3-Z-[1-(4-(N-(3-methylaminopropyl)-N-acetylamino)anilino)-1-(3-(2-carboxyethyl)phenyl)methylene]-6-chloro-2-indolinone
(334) 3-Z-[1-(4-(3-dimethylaminopropyl)anilino)-1-(3-(2-carboxyethyl)phenyl)methylene]-6-chloro-2-indolinone
(335) 3-Z-[1-(4-ethylaminomethylanilino)-1-(3-(2-carboxyethyl)phenyl)methylene]-6-chloro-2-indolinone
(336) 3-Z-[1-(4-methylaminomethylanilino)-1-(3-(2-carboxyethyl)phenyl)methylene]-6-chloro-2-indolinone
(337) 3-Z-[1-(4-(N-(4-methylpiperazin-1-ylmethylcarbonyl)-N-methylamino)anilino)-1-(3-(2-carboxyethyl)phenyl)methylene]-6-chloro-2-indolinone
(338) 3-Z-[1-(4-(4-methylpiperazin-1-ylcarbonyl)anilino)-1-(3-(2-carboxyethyl)phenyl)methylene]-6-chloro-2-indolinone
(339) 3-Z-[1-(4-(N-(3-dimethylaminopropyl)-N-methylsulphonylamino)anilino)-1-(3-(2-carboxyethyl)phenyl)methylene]-6-chloro-2-indolinone
(340) 3-Z-[1-(4-(N-(2-dimethylaminoethyl)-N-propylsulphonylamino)anilino)-1-(3-(2-carboxyethyl)phenyl)methylene]-6-chloro-2-indolinone
(341) 3-Z-[1-(4-aminomethylanilino)-1-(3-(2-carboxyethyl)phenyl)methylene]-6-chloro-2-indolinone
(342) 3-Z-[1-(3-(dimethylaminomethyl)anilino)-1-(3-(2-carboxyethyl)phenyl)methylene]-6-chloro-2-indolinone
(343) 3-Z-[1-(3-(methylaminomethyl)anilino)-1-(3-(2-carboxyethyl)phenyl)methylene]-6-chloro-2-indolinone
(344) 3-Z-[1-(3-(2-dimethylaminoethyl)anilino)-1-(3-(2-carboxyethyl)phenyl)methylene]-6-chloro-2-indolinone
(345) 3-Z-[1-(3-(3-dimethylaminopropyl)anilino)-1-(3-(2-carboxyethyl)phenyl)methylene]-6-chloro-2-indolinone
(346) 3-Z-[1-(4-(2-dimethylaminoethyl)anilino)-1-(3-(2-carboxyethyl)phenyl)methylene]-6-chloro-2-indolinone
(347) 3-Z-[1-(4-(N-(dimethylaminocarbonylmethyl)-N-methylsulphonylamino)anilino)-1-(3-(2-carboxyethyl)phenyl)methylene]-6-chloro-2-indolinone
(348) 3-Z-[1-(4-(N-methyl-N-methylsulphonylamino)anilino)-1-(3-(2-carboxyethyl)phenyl)methylene]-6-chloro-2-indolinone
(349) 3-Z-[1-(4-(N-methyl-N-acetylamino)anilino)-1-(3-(2-carboxyethyl)phenyl)methylene]-6-chloro-2-indolinone
(350) 3-Z-[1-(4-(1-methylimidazol-2-yl)anilino)-1-(3-(2-carboxyethyl)phenyl)methylene]-6-chloro-2-indolinone

(351) 3-Z-[1-(4-(N-(N-(2-dimethylaminoethyl)-N-methylaminomethylcarbonyl)-N-methylamino)anilino)-1-(3-(2-carboxyethyl)phenyl)methylene]-6-chloro-2-indolinone
(352) 3-Z-[1-(4-(2-diethylaminoethylsulphonyl)anilino)-1-(3-(2-carboxyethyl)phenyl)methylene]-6-chloro-2-indolinone
(353) 3-Z-[1-(4-(N-(2-dimethylaminoethylcarbonyl)-N-methylamino)anilino)-1-(3-(2-carboxyethyl)phenyl)methylene]-6-chloro-2-indolinone
(354) 3-Z-[1-(4-(N-(2-dimethylaminoethyl)-N-methylaminomethyl)anilino)-1-(3-(2-carboxyethyl)phenyl)methylene]-6-chloro-2-indolinone
(355) 3-Z-[1-(4-(2-dimethylaminoethoxy)anilino)-1-(3-(2-carboxyethyl)phenyl)methylene]-6-chloro-2-indolinone
(356) 3-Z-[1-(4-(N-(4-dimethylaminobutylcarbonyl)-N-methylamino)anilino)-1-(3-(2-carboxyethyl)phenyl)methylene]-6-chloro-2-indolinone
(357) 3-Z-[1-(4-(N-(3-dimethylaminopropylcarbonyl)-N-methylamino)anilino)-1-(3-(2-carboxyethyl)phenyl)methylene]-6-chloro-2-indolinone
(358) 3-Z-[1-(4-(methylethylaminomethyl)anilino)-1-(3-(2-carboxyethyl)phenyl)methylene]-6-chloro-2-indolinone
(359) 3-Z-[1-(4-(methylpropylaminomethyl)anilino)-1-(3-(2-carboxyethyl)phenyl)methylene]-6-chloro-2-indolinone
(360) 3-Z-[1-(4-(methylbenzylaminomethyl)anilino)-1-(3-(2-carboxyethyl)phenyl)methylene]-6-chloro-2-indolinone
(361) 3-Z-[1-(4-(diethylaminomethyl)anilino)-1-(3-(2-carboxyethyl)phenyl)methylene]-6-chloro-2-indolinone
(362) 3-Z-[1-(4-(N-(2-dimethylaminoethyl)*-*N-methylaminomethyl)anilino)-1-(3-(2-carboxyethyl)phenylmethylene]-6-chloro-2-indolinone
(363) 3-Z-[1-(4-(pyrrolidin-1-ylmethyl)anilino)-1-(3-(2-carboxyethyl)phenyl)methylene]-6-chloro-2-indolinone
(364) 3-Z-[1-(4-(azetidin-1-ylmethyl)anilino)-1-(3-(2-carboxyethyl)phenyl)methylene]-6-chloro-2-indolinone
(365) 3-Z-[1-(4-((4-methylpiperazin-1-yl)methyl)anilino)-1-(3-(2-carboxyethyl)phenyl)methylene]-6-chloro-2-indolinone
(366) 3-Z-[1-(4-(piperazin-1-ylmethyl)anilino)-1-(3-(2-carboxyethyl)phenyl)methylene]-6-chloro-2-indolinone
(367) 3-Z-[1-(4-(morpholin-4-ylmethyl)anilino)-1-(3-(2-carboxyethyl)phenyl)methylene]-6-chloro-2-indolinone
(368) 3-Z-[1-(4-(thiomorpholin-4-ylmethyl)anilino)-1-(3-(2-carboxyethyl)phenyl)methylene]-6-chloro-2-indolinone
(369) 3-Z-[1-(4-(imidazol-1-ylmethyl)anilino)-1-(3-(2-carboxyethyl)phenyl)methylene]-6-chloro-2-indolinone
(370) 3-Z-[1-(4-(N-(2-methylaminoethyl)*-*N-acetylamino)anilino)-1-(4-(2-carboxyethyl)phenyl)methylene]-6-fluoro-2-indolinone
(371) 3-Z-[1-(4-(N-(3-methylaminopropyl)-N-acetylamino)anilino)-1-(4-(2-carboxyethyl)phenyl)methylene]-6-fluoro-2-indolinone
(372) 3-Z-[1-(4-(3-dimethylaminopropyl)anilino)-1-(4-(2-carboxyethyl)phenyl)methylene]-6-fluoro-2-indolinone
(373) 3-Z-[1-(4-ethylaminomethylanilino)-1-(4-(2-carboxyethyl)phenyl)methylene]-6-fluoro-2-indolinone
(374) 3-Z-[1-(4-(N-(3-dimethylaminopropyl)-N-methylsulphonylamino)anilino)-1-(4-(2-carboxyethyl)phenyl)methylene]-6-fluoro-2-indolinone
(375) 3-Z-[1-(4-(N-(2-dimethylaminoethyl)-N-propylsulphonylamino)anilino)-1-(4-(2-carboxyethyl)phenyl)methylene]-6-fluoro-2-indolinone
(376) 3-Z-[1-(3-(methylaminomethyl)anilino)-1-(4-(2-carboxyethyl)phenyl)methylene]-6-fluoro-2-indolinone
(377) 3-Z-[1-(3-(2-dimethylaminoethyl)amino)-1-(4-(2-carboxyethyl)phenyl)methylene]-6-fluoro-2-indolinone
(378) 3-Z-[1-(3-(3-dimethylaminopropyl)anilino)-1-(4-(2-carboxyethyl)phenyl)methylene]-6-fluoro-2-indolinone
(379) 3-Z-[1-(4-(N-(dimethylaminocarbonylmethyl)-N-methylsulphonylamino)anilino)-1-(4-(2-carboxyethyl)phenyl)methylene]-6-fluoro-2-indolinone
(380) 3-Z-[1-(4-(N-methyl-N-methylsulphonylamino)anilino)-1-(4-(2-carboxyethyl)phenyl)methylene]-6-fluoro-2-indolinone
(381) 3-Z-[1-(4-(N-(N-(2-dimethylaminoethyl)-N-methylaminomethylcarbonyl)-N-methylamino)anilino)-1-(4-(2-carboxyethyl)phenyl)methylene]-6-fluoro-2-indolinone
(382) 3-Z-[1-(4-(2-diethylaminoethylsulphonyl)aniline)-1-(4-(2-carboxyethyl)phenyl)methylene]-6-fluoro-2-indolinone
(383) 3-Z-[1-(4-(2-dimethylaminoethoxy)anilino)-1-(4-(2-carboxyethyl)phenyl)methylene]-6-fluoro-2-indolinone
(384) 3-Z-[1-(4-(N-(3-dimethylaminopropylcarbonyl)-N-methylamino)anilino)-1-(4-(2-carboxyethyl)phenyl)methylene]-6-fluoro-2-indolinone
(385) 3-Z-[1-(4-(methylethylaminomethyl)anilino)-1-(4-(2-carboxyethyl)phenyl)methylene]-6-fluoro-2-indolinone
(386) 3-Z-[1-(4-(methylpropylaminomethyl)anilino)-1-(4-(2-carboxyethyl)phenyl)methylene]-6-fluoro-2-indolinone
(387) 3-Z-[1-(4-(methylbenzylaminomethyl)anilino)-1-(4-(2-carboxyethyl)phenyl)methylene]-6-fluoro-2-indolinone
(388) 3-Z-[1-(4-(azetidin-1-ylmethyl)anilino)-1-(4-(2-carboxyethyl)phenyl)methylene]-6-fluoro-2-indolinone
(389) 3-Z-[1-(4-(piperazin-1-ylmethyl)anilino)-1-(4-(2-carboxyethyl)phenyl)methylene]-6-fluoro-2-indolinone
(390) 3-Z-[1-(4-(morpholin-4-ylmethyl)anilino)-1-(4-(2-carboxyethyl)phenyl)methylene]-6-fluoro-2-indolinone
(391) 3-Z-[1-(4-(thiomorpholin-4-ylmethyl)anilino)-1-(4-(2-carboxyethyl)phenyl)methylene]-6-fluoro-2-indolinone
(392) 3-Z-[1-(4-(N-(2-dimethylaminoethyl)-N-acetylamino)anilino)-1-(3-(2-carboxyethyl)phenyl)methylene]-6-fluoro-2-indolinone
(393) 3-Z-[1-(4-(N-(2-methylaminoethyl)-N-acetylamino) anilino) -1- (3-(2-carboxyethyl)phenyl)methylene]-6-fluoro-2-indolinone
(394) 3-Z-[1-(4-(N-(3-methylaminopropyl)-N-acetylamino)anilino)-1-(3-(2-carboxyethyl)phenyl)methylene]-6-fluoro-2-indolinone
(395) 3-Z-[1-(4-(3-dimethylaminopropyl)anilino)-1-(3-(2-carboxyethyl)phenyl)methylene]-6-fluoro-2-indolinone
(396) 3-Z-[1-(4-ethylaminomethylanilino)-1-(3-(2-carboxyethyl)phenyl)methylene]-6-fluoro-2-indolinone
(397) 3-Z-[1-(4-(4-methylpiperazin-1-ylcarbonyl)anilino)-1-(3-(2-carboxyethyl)phenyl)methylene]-6-fluoro-2-indolinone
(398) 3-Z-[1-(4-(N-(3-dimethylaminopropyl)-N-methylsulphonylamino)anilino)-1-(3-(2-carboxyethyl)phenyl)methylene]-6-fluoro-2-indolinone
(399) 3-Z-[1-(4-(N-(2-dimethylaminoethyl)-N-propylsulphonylamino)anilino)-1-(3-(2-carboxyethyl)phenyl)methylene]-6-fluoro-2-indolinone

(400) 3-Z-[1-(3-(methylaminomethyl)anilino)-1-(3-(2-carboxyethyl)phenyl)methylene]-6-fluoro-2-indolinone
(401) 3-Z-[1-(3-(2-dimethylaminoethyl)anilino)-1-(3-(2-carboxyethyl)phenyl)methylene]-6-fluoro-2-indolinone
(402) 3-Z-[1-(3-(3-dimethylaminopropyl)anilino)-1-(3-(2-carboxyethyl)phenyl)methylene]-6-fluoro-2-indolinone
(403) 3-Z-[1-(4-(N-(dimethylaminocarbonylmethyl)-N-methylsulphonylamino)anilino)-1-(3-(2-carboxyethyl)phenyl)methylene]-6-fluoro-2-indolinone
(404) 3-Z-[1-(4-(N-methyl-N-methylsulphonylamino)anilino)-1-(3-(2-carboxyethyl)phenyl)methylene]-6-fluoro-2-indolinone
(405) 3-Z-[1-(4-(N-methyl-N-acetylamino)anilino)-1-(3-(2-carboxyethyl)phenyl)methylene]-6-fluoro-2-indolinone
(406) 3-Z-[1-(4-(N-(N-(2-dimethylaminoethyl)-N-methylaminomethylcarbonyl)-N-methylamino)anilino)-1-(3-(2-carboxyethyl)phenyl)methylene]-6-fluoro-2-indolinone
(407) 3-Z-[1-(4-(2-diethylaminoethylsulphonyl)anilino)-1-(3-(2-carboxyethyl)phenyl)methylene]-6-fluoro-2-indolinone
(408) 3-Z-[1-(4-(N-(2-dimethylaminoethylcarbonyl)-N-methylamino)anilino)-1-(3-(2-carboxyethyl)phenyl)methylene]-6-fluoro-2-indolinone
(409) 3-Z-[1-(4-(N-(2-dimethylaminoethyl)-N-methylaminomethyl)anilino)-1-(3-(2-carboxyethyl)phenyl)methylene]-6-fluoro-2-indolinone
(410) 3-Z-[1-(4-(2-dimethylaminoethoxy)anilino)-1-(3-(2-carboxyethyl)phenyl)methylene]-6-fluoro-2-indolinone
(411) 3-Z-[1-(4-(methylethylaminomethyl)anilino)-1-(3-(2-carboxyethyl)phenyl)methylene]-6-fluoro-2-indolinone
(412) 3-Z-[1-(4-(methylpropylaminomethyl)anilino)-1-(3-(2-carboxyethyl)phenyl)methylene]-6-fluoro-2-indolinone
(413) 3-Z-[1-(4-(methylbenzylaminomethyl)anilino)-1-(3-(2-carboxyethyl)phenyl)methylene]-6-fluoro-2-indolinone
(414) 3-Z-[1-(4-(diethylaminomethyl)anilino)-1-(3-(2-carboxyethyl)phenyl)methylene]-6-fluoro-2-indolinone
(415) 3-Z-[1-(4-(pyrrolidin-1-ylmethyl)anilino)-1-(3-(2-carboxyethyl)phenyl)methylene]-6-fluoro-2-indolinone
(416) 3-Z-[1-(4-(azetidin-1-ylmethyl)anilino)-1-(3-(2-carboxyethyl)phenyl)methylene]-6-fluoro-2-indolinone
(417) 3-Z-[1-(4-(piperazin-1-ylmethyl)anilino)-1-(3-(2-carboxyethyl)phenyl)methylene]-6-fluoro-2-indolinone
(418) 3-Z-[1-(4-(morpholin-4-ylmethyl)anilino)-1-(3-(2-carboxyethyl)phenyl)methylene]-6-fluoro-2-indolinone
(419) 3-Z-[1-(4-(thiomorpholin-4-ylmethyl)anilino)-1-(3-(2-carboxyethyl)phenyl)methylene]-6-fluoro-2-indolinone
(420) 3-Z-[1-(4-(N-(2-dimethylaminoethyl)-N-methylsulphonylamino)anilino)-1-(4-(2-carboxyethyl)phenyl)methylene]-6-bromo-2-indolinone
(421) 3-Z-[1-(4-(N-(dimethylaminomethylcarbonyl)-N-methylamino)anilino)-1-(4-(2-carboxyethyl)phenyl)methylene]-6-bromo-2-indolinone
(422) 3-Z-[1-(4-(N-(2-dimethylaminoethyl)-N-acetylamino)anilino)-1-(4-(2-carboxyethyl)phenyl)methylene]-6-bromo-2-indolinone
(423) 3-Z-[1-(4-(N-(2-methylaminoethyl)-N-acetylamino)anilino)-1-(4-(2-carboxyethyl)phenyl)methylene]-6-bromo-2-indolinone
(424) 3-Z-[1-(4-(N-(3-dimethylaminopropyl)-N-acetylamino)anilino)-1-(4-(2-carboxyethyl)phenyl)methylene]-6-bromo-2-indolinone
(425) 3-Z-[1-(4-(N-(3-methylaminopropyl)-N-acetylamino)anilino)-1-(4-(2-carboxyethyl)phenyl)methylene]-6-bromo-2-indolinone
(426) 3-Z-[1-(4-(3-dimethylaminopropyl)anilino)-1-(4-(2-carboxyethyl)phenyl)methylene]-6-bromo-2-indolinone
(427) 3-Z-[1-(4-ethylaminomethylanilino)-1-(4-(2-carboxyethyl)phenyl)methylene]-6-bromo-2-indolinone
(428) 3-Z-[1-(4-methylaminomethylanilino)-1-(4-(2-carboxyethyl)phenyl)methylene]-6-bromo-2-indolinone
(429) 3-Z-[1-(4-(N-(4-methylpiperazin-1-ylmethylcarbonyl)-N-methylamino)anilino)-1-(4-(2-carboxyethyl)phenyl)methylene]-6-bromo-2-indolinone
(430) 3-Z-[1-(4-(4-methylpiperazin-1-ylcarbonyl)anilino)-1-(4-(2-carboxyethyl)phenyl)methylene]-6-bromo-2-indolinone
(431) 3-Z-[1-(4-(N-(3-dimethylaminopropyl)-N-methylsulphonylamino)anilino)-1-(4-(2-carboxyethyl)phenyl)methylene]-6-bromo-2-indolinone
(432) 3-Z-[1-(4-(N-(2-dimethylaminoethyl)-N-propylsulphonylamino)anilino)-1-(4-(2-carboxyethyl)phenyl)methylene]-6-bromo-2-indolinone
(433) 3-Z-[1-(4-aminomethylanilino)-1-(4-(2-carboxyethyl)phenyl)methylene]-6-bromo-2-indolinone
(434) 3-Z-[1-(3-(dimethylaminomethyl)anilino)-1-(4-(2-carboxyethyl)phenyl)methylene]-6-bromo-2-indolinone
(435) 3-Z-[1-(3-(methylaminomethyl)anilino)-1-(4-(2-carboxyethyl)phenyl)methylene]-6-bromo-2-indolinone
(436) 3-Z-[1-(3-(2-dimethylaminoethyl)anilino)-1-(4-(2-carboxyethyl)phenyl)methylene]-6-bromo-2-indolinone
(437) 3-Z-[1-(3-(3-dimethylaminopropyl)anilino)-1-(4-(2-carboxyethyl)phenyl)methylene]-6-bromo-2-indolinone
(438) 3-Z-[1-(4-(2-dimethylaminoethyl)anilino)-1-(4-(2-carboxyethyl)phenyl)methylene]-6-bromo-2-indolinone
(439) 3-Z-[1-(4-(N-(dimethylaminocarbonylmethyl)-N-methylsulphonylamino)anilino)-1-(4-(2-carboxyethyl)phenyl)methylene]-6-bromo-2-indolinone
(440) 3-Z-[1-(4-(N-methyl-N-methylsulphonylamino)anilino)-1-(4-(2-carboxyethyl)phenyl)methylene]-6-bromo-2-indolinone
(441) 3-Z-[1-(4-(N-methyl-N-acetylamino)anilino)-1-(4-(2-carboxyethyl)phenyl)methylene]-6-bromo-2-indolinone
(442) 3-Z-[1-(4-(1-methylimidazol-2-yl)anilino)-1-(4-(2-carboxyethyl)phenyl)methylene]-6-bromo-2-indolinone
(443) 3-Z-[1-(4-(N-(N-(2-dimethylaminoethyl)-N-methylaminomethylcarbonyl)-N-methylamino)anilino)-1-(4-(2-carboxyethyl)phenyl)methylene]-6-bromo-2-indolinone
(444) 3-Z-[1-(4-(2-diethylaminoethylsulphonyl)anilino)-1-(4-(2-carboxyethyl)phenyl)methylene]-6-bromo-2-indolinone
(445) 3-Z-[1-(4-(N-(2-dimethylaminoethylcarbonyl)-N-methylamino)aniline)-1-(4-(2-carboxyethyl)phenyl)methylene]-6-bromo-2-indolinone
(446) 3-Z-[1-(4-(N-(2-dimethylaminoethyl)-N-methylaminomethyl)anilino)-1-(4-(2-carboxyethyl)phenyl)methylene]-6-bromo-2-indolinone
(447) 3-Z-[1-(4-(2-dimethylaminoethoxy)anilino)-1-(4-(2-carboxyethyl)phenyl)methylene]-6-bromo-2-indolinone
(448) 3-Z-[1-(4-(N-(4-dimethylaminobutylcarbonyl)-N-methylamino)aniline)-1-(4-(2-carboxyethyl)phenyl)methylene]-6-bromo-2-indolinone
(449) 3-Z-[1-(4-(N-(3-dimethylaminopropylcarbonyl)-N-methylamino)anilino)-1-(4-(2-carboxyethyl)phenyl)methylene]-6-bromo-2-indolinone
(450) 3-Z-(1-(4-(methylethylaminomethyl)anilino)-1-(4-(2-carboxyethyl)phenyl)methylene]-6-bromo-2-indolinone

(451) 3-Z-[1-(4-(methylpropylaminomethyl)anilino)-1-(4-(2-carboxyethyl)phenyl)methylene]-6-bromo-2-indolinone
(452) 3-Z-[1-(4-(methylbenzylaminomethyl)anilino)-1-(4-(2-carboxyethyl)phenyl)methylene]-6-bromo-2-indolinone
(453) 3-Z-[1-(4-(diethylaminomethyl)anilino)-1-(4-(2-carboxyethyl)phenyl)methylene]-6-bromo-2-indolinone
(454) 3-Z-[1-(4-(N-(2-dimethylaminoethyl)-N-methylaminomethyl)anilino)-1-(4-(2-carboxyethyl)-phenylmethylene]-6-bromo-2-indolinone
(455) 3-Z-[1-(4-(pyrrolidin-1-ylmethyl)anilino)-1-(4-(2-carboxyethyl)phenyl)methylene]-6-bromo-2-indolinone
(456) 3-Z-[1-(4-(azetidin-1-ylmethyl)anilino)-1-(4-(2-carboxyethyl)phenyl)methylene]-6-bromo-2-indolinone
(457) 3-Z-[1-(4-((4-methylpiperazin-1-yl)methyl)anilino)-1-(4-(2-carboxyethyl)phenyl)methylene]-6-bromo-2-indolinone
(458) 3-Z-[1-(4-(piperazin-1-ylmethyl)anilino)-1-(4-(2-carboxyethyl)phenyl)methylene]-6-bromo-2-indolinone
(459) 3-Z-[1-(4-(morpholin-4-ylmethyl)anilino)-1-(4-(2-carboxyethyl)phenyl)methylene]-6-bromo-2-indolinone
(460) 3-Z-[1-(4-(thiomorpholin-4-ylmethyl)anilino)-1-(4-(2-carboxyethyl)phenyl)methylene]-6-bromo-2-indolinone
(461) 3-Z-[1-(4-(imidazol-1-ylmethyl)anilino)-1-(4-(2-carboxyethyl)phenyl)methylene]-6-bromo-2-indolinone
(462) 3-Z-[1-(4-dimethylaminomethylanilino)-1-(3-(2-carboxyethyl)phenyl)methylene]-6-bromo-2-indolinone
(463) 3-Z-[1-(4-(N-(2-dimethylaminoethyl)-N-methylsulphonylamino)anilino)-1-(3-(2-carboxyethyl)phenyl)methylene]-6-bromo-2-indolinone
(464) 3-Z-[1-(4-(N-(dimethylaminomethylcarbonyl)-N-methylamino)anilino)-1-(3-(2-carboxyethyl)phenyl)methylene]-6-bromo-2-indolinone
(465) 3-Z-[1-(4-(N-(2-dimethylaminoethyl)-N-acetylamino)anilino)-1-(3-(2-carboxyethyl)phenyl)methylene]-6-bromo-2-indolinone
(466) 3-Z-[1-(4-(N-(2-methylaminoethyl)-N-acetylamino)anilino)-1-(3-(2-carboxyethyl)phenyl)methylene]-6-bromo-2-indolinone
(467) 3-Z-[1-(4-(N-(3-dimethylaminopropyl)-N-acetylamino)anilino)-1-(3-(2-carboxyethyl)phenyl)methylene]-6-bromo-2-indolinone
(468) 3-Z-[1-(4-(N-(3-methylaminopropyl)-N-acetylamino)aniline)-1-(3-(2-carboxyethyl)phenyl)methylene]-6-bromo-2-indolinone
(469) 3-Z-[1-(4-(3-dimethylaminopropyl)anilino)-1-(3-(2-carboxyethyl)phenyl)methylene]-6-bromo-2-indolinone
(470) 3-Z-[1-(4-ethylaminomethylanilino)-1-(3-(2-carboxyethyl)phenyl)methylene]-6-bromo-2-indolinone
(471) 3-Z-[1-(4-methylaminomethylanilino)-1-(3-(2-carboxyethyl)phenyl)methylene]-6-bromo-2-indolinone
(472) 3-Z-[1-(4-(N-(4-methylpiperazin-1-ylmethylcarbonyl)-N-methylamino)anilino)-1-(3-(2-carboxyethyl)phenyl)methylene]-6-bromo-2-indolinone
(473) 3-Z-[1-(4-(4-methylpiperazin-1-ylcarbonyl)anilino)-1-(3-(2-carboxyethyl)phenyl)methylene]-6-bromo-2-indolinone
(474) 3-Z-[1-(4-(N-(3-dimethylaminopropyl)-N-methylsulphonylamino)anilino)-1-(3-(2-carboxyethyl)phenyl)methylene]-6-bromo-2-indolinone
(475) 3-Z-[1-(4-(N-(2-dimethylaminoethyl)-N-propylsulphonylamino)anilino)-1-(3-(2-carboxyethyl)phenyl)methylene]-6-bromo-2-indolinone
(476) 3-Z-[1-(4-aminomethylanilino)-1-(3-(2-carboxyethyl)phenyl)methylene]-6-bromo-2-indolinone
(477) 3-Z-[1-(3-(dimethylaminomethyl)anilino)-1-(3-(2-carboxyethyl)phenyl)methylene]-6-bromo-2-indolinone
(478) 3-Z-[1-(3-(methylaminomethyl)anilino)-1-(3-(2-carboxyethyl)phenyl)methylene]-6-bromo-2-indolinone
(479) 3-Z-[1-(3-(2-dimethylaminoethyl)anilino)-1-(3-(2-carboxyethyl)phenyl)methylene]-6-bromo-2-indolinone
(480) 3-Z-[1-(3-(3-dimethylaminopropyl)anilino)-1-(3-(2-carboxyethyl)phenyl)methylene]-6-bromo-2-indolinone
(481) 3-Z-[1-(4-(2-dimethylaminoethyl)anilino)-1-(3-(2-carboxyethyl)phenyl)methylene]-6-bromo-2-indolinone
(482) 3-Z-[1-(4-(N-(dimethylaminocarbonylmethyl)-N-methylsulphonylamino)anilino)-1-(3-(2-carboxyethyl)phenyl)methylene]-6-bromo-2-indolinone
(483) 3-Z-[1-(4-(N-methyl-N-methylsulphonylamino)anilino)-1-(3-(2-carboxyethyl)phenyl)methylene]-6-bromo-2-indolinone
(484) 3-Z-[1-(4-(N-methyl-N-acetylamino)anilino)-1-(3-(2-carboxyethyl)phenyl)methylene]-6-bromo-2-indolinone
(485) 3-Z-[1-(4-(1-methylimidazol-2-yl)anilino)-1-(3-(2-carboxyethyl)phenyl)methylene]-6-bromo-2-indolinone
(486) 3-Z-[1-(4-(N-(N-(2-dimethylaminoethyl)-N-methylaminomethylcarbonyl)-N-methylamino)anilino)-1-(3-(2-carboxyethyl)phenyl)methylene]-6-bromo-2-indolinone
(487) 3-Z-[1-(4-(2-diethylaminoethylsulphonyl)aniline)-1-(3-(2-carboxyethyl)phenyl)methylene]-6-bromo-2-indolinone
(488) 3-Z-[1-(4-(N-(2-dimethylaminoethylcarbonyl)-N-methylamino)anilino)-1-(3-(2-carboxyethyl)phenyl)methylene]-6-bromo-2-indolinone
(489) 3-Z-[1-(4-(N-(2-dimethylaminoethyl)-N-methylaminomethyl)anilino)-1-(3-(2-carboxyethyl)phenyl)methylene]-6-bromo-2-indolinone
(490) 3-Z-[1-(4-(2-dimethylaminoethoxy)anilino)-1-(3-(2-carboxyethyl)phenyl)methylene]-6-bromo-2-indolinone
(491) 3-Z-[1-(4-(N-(4-dimethylaminobutylcarbonyl)-N-methylamino)anilino)-1-(3-(2-carboxyethyl)phenyl)methylene]-6-bromo-2-indolinone
(492) 3-Z-[1-(4-(N-(3-dimethylaminopropylcarbonyl)-N-methylamino)anilino)-1-(3-(2-carboxyethyl)phenyl)methylene]-6-bromo-2-indolinone
(493) 3-Z-[1-(4-(methylethylaminomethyl)anilino)-1-(3-(2-carboxyethyl)phenyl)methylene]-6-bromo-2-indolinone
(494) 3-Z-[1-(4-(methylpropylaminomethyl)anilino)-1-(3-(2-carboxyethyl)phenyl)methylene]-6-bromo-2-indolinone
(495) 3-Z-[1-(4-(methylbenzylaminomethyl)anilino)-1-(3-(2-carboxyethyl)phenyl)methylene]-6-bromo-2-indolinone
(496) 3-Z-[1-(4-(diethylaminomethyl)anilino)-1-(3-(2-carboxyethyl)phenyl)methylene]-6-bromo-2-indolinone
(497) 3-Z-[1-(4-(N-(2-dimethylaminoethyl)-N-methylaminomethyl)anilino)-1-(3-(2-carboxyethyl)phenylmethylene]-6-bromo-2-indolinone
(498) 3-Z-[1-(4-(pyrrolidin-1-ylmethyl)anilino)-1-(3-(2-carboxyethyl)phenyl)methylene]-6-bromo-2-indolinone
(499) 3-Z-[1-(4-(azetidin-1-ylmethyl)anilino)-1-(3-(2-carboxyethyl)phenyl)methylene]-6-bromo-2-indolinone

(500) 3-Z-[1-(4-((4-methylpiperazin-1-yl)methyl)anilino)-1-(3-(2-carboxyethyl)phenyl)methylene]-6-bromo-2-indolinone
(501) 3-Z-[1-(4-(piperazin-1-ylmethyl)anilino)-1-(3-(2-carboxyethyl)phenyl)methylene]-6-bromo-2-indolinone
(502) 3-Z-[1-(4-(morpholin-4-ylmethyl)anilino)-1-(3-(2-carboxyethyl)phenyl)methylene]-6-bromo-2-indolinone
(503) 3-Z-[1-(4-(thiomorpholin-4-ylmethyl)anilino)-1-(3-(2-carboxyethyl)phenyl)methylene]-6-bromo-2-indolinone
(504) 3-Z-[1-(4-(imidazol-1-ylmethyl)anilino)-1-(3-(2-carboxyethyl)phenyl)methylene]-6-bromo-2-indolinone
(505) 3-Z-[1-(4-dimethylaminomethylanilino)-1-(4-carboxymethylaminophenyl)-methylene]-6-fluoro-2-indolinone
(506) 3-Z-[1-(4-dimethylaminomethylanilino)-1-(3-carboxymethylamino-phenyl)-methylene]-6-fluoro-2-indolinone
(507) 3-Z-[1-(4-dimethylaminomethylanilino)-1-(4-(N-methyl-carboxymethylamino)phenyl)methylene]-6-fluoro-2-indolinone
(508) 3-Z-[1-(4-dimethylaminomethylanilino)-1-(3-(N-methyl-carboxymethylamino)phenyl)methylene]-6-fluoro-2-indolinone
(509) 3-Z-[1-(4-dimethylaminomethylanilino)-1-(4-carboxymethoxyphenyl)-methylene]-6-chloro-2-indolinone
(510) 3-Z-[1-(4-dimethylaminomethylanilino)-1-(3-carboxymethoxyphenyl)-methylene]-6-chloro-2-indolinone
(511) 3-Z-[1-(4-dimethylaminomethylanilino)-1-(4-carboxymethylaminophenyl)-methylene]-6-chloro-2-indolinone
(512) 3-Z-[1-(4-dimethylaminomethylanilino)-1-(3-carboxymethylaminophenyl)-methylene]-6-chloro-2-indolinone
(513) 3-Z-[1-(4-dimethylaminomethylanilino)-1-(4-(N-methyl-carboxymethylamino)phenyl)methylene]-6-chloro-2-indolinone
(514) 3-Z-[1-(4-dimethylaminomethylanilino)-1-(3-(N-methyl-carboxymethylamino)phenyl)methylene]-6-chloro-2-indolinone
(515) 3-Z-[1-(4-dimethylaminomethylanilino)-1-(4-carboxymethoxyphenyl)-methylene]-6-bromo-2-indolinone
(516) 3-Z-[1-(4-dimethylaminomethylanilino)-1-(3 carboxymethoxyphenyl)-methylene]-6-bromo-2-indolinone
(517) 3-Z-[1-(4-dimethylaminomethylanilino)-1-(4-carboxymethylaminophenyl)-methylene]-6-bromo-2-indolinone
(518) 3-Z-[1-(4-dimethylaminomethylanilino)-1-(3-carboxymethylaminophenyl)-methylene]-6-bromo-2-indolinone
(519) 3-Z-[1-(4-dimethylaminomethylanilino)-1-(4-(N-methyl-carboxymethylamino)phenyl)methylene]-6-bromo-2-indolinone
(520) 3-Z-[1-(4-dimethylaminomethylanilino)-1-(3-(N-methyl-carboxymethylamino)phenyl)methylene]-6-bromo-2-indolinone,
as well as their tautomers, their stereoisomers or the physiologically acceptable salts thereof.

The compounds of general formula I, their tautomers, their stereoisomers or the physiologically acceptable salts thereof are thus suitable for the prevention or treatment of a specific fibrotic disease selected from the group consisting of:

Fibrosis and remodeling of lung tissue in chronic obstructive pulmonary disease (COPD), chronic bronchitis, and emphysema;

Lung fibrosis and pulmonary diseases with a fibrotic component including but not limited to idiopathic pulmonary fibrosis (IPF) , giant cell interstitial pneumonia (GIP) , sarcodosis, cystic fibrosis, respiratory distress syndrome (ARDS), granulomatosis, silicosis, drug-induced lung fibrosis (for example, induced by drugs such as bleomycin, bis-chloronitrosourea, cyclophosphamide, amiodarone, procainamide, penicillamine, gold or nitrofurantoin), silicosis, asbestosis, systemic scleroderma;

Fibrosis and remodeling in asthma;

In a preferred embodiment in accordance with the present invention, the compounds of general formula I, their tautomers, their stereoisomers or the physiologically acceptable salts thereof are especially suitable for the prevention or treatment of idiopathic pulmonary fibrosis.

### BIOLOGICAL ACTIVITY

The following experimental results illustrate the present invention without representing a limitation of its scope.

### Abreviations

DEPC (diethylpyrocarbonate)
dNTP (deoxyribonucleotide triphosphates)
CT (Cycle at which amplification reaches a set Threshold)
DNA (deoxyribonucleic acid)
cDNA (complementary DNA)
RNA (ribonucleic acid)
mRNA (messenger RNA)
PCR (polymerase chain reaction)

### Example B1:

In the following experiments of Example B1, Example A denotes the compound 3-Z-[1-(4-dimethylaminomethylanilino)-1-(4-(2-carboxyethyl)phenyl)methylene]-6-fluoro-2-indolinone, which is compound (134) of the list of compounds and compound (b) of the list of preferred compounds.

### (A) Effect of a representative compound on lung morphology following bleomycin-induced pulmonary fibrosis.

### Materials and Methods

Bleomycin sulfate (Bleomycin HEXAL^{™}) was purchased from a local pharmacy.

Bleomycin administration and treatment protocols All experiments were performed in accordance with German guidelines for animal welfare, performed by persons certified to work with animals and approved by the responsible authorities. Male Wistar rats were intratracheally injected with Bleomycin sulfate (10U/kg body weight in 300µl saline) or saline alone (saline control) using a catheter (0,5mm internal diameter, 1.0mm external diameter) through the nasal passage, following exposure to the anaesthetic Isofluorane for 5 minutes. The following day, the rats were orally treated with Example A (compound (134)) or saline suspended in 1ml 0.1% Natrosol. Control rats were administered 1ml 0.1% Natrosol (vehicle control).

A total of 30 rats were investigated and were grouped and treated as shown in Table 1.

**Table 1**

| Intratracheal instillation | No. of animals | Compound | Treatment Schedule |
|---|---|---|---|
| | | | |
| Bleomycin 10U /kg | 10 | Example A (Compound (134)) | Days 1-21 |
| Bleomycin 10U /kg | 10 | Vehicle only | Days 1-21 |
| Saline (300µl) | 10 | Vehicle only | Days 1-21 |

21 days following bleomycin instillation, the rats were killed with a lethal intraperitoneal injection of Narcoren^{™} (Pentobarbital Sodium, Rhone Merieux). The lungs were then removed, blotted dry and half was snap frozen in liquid nitrogen and stored at -80°C. The other half was fixed in 4% formalin for subsequent paraffin embedding and histology.

### Histology

The lung tissues fixed in 4% formalin were embedded into paraffin and 5µm sections were cut using a microtome (Leica SM200R) and placed on poly-L-lysine coated slides. The sections were then dried onto the slides (60°C 2 hours) and then left to cool at room temperature. Collagen deposition was assessed using Masson's Trichrome staining.

### Results

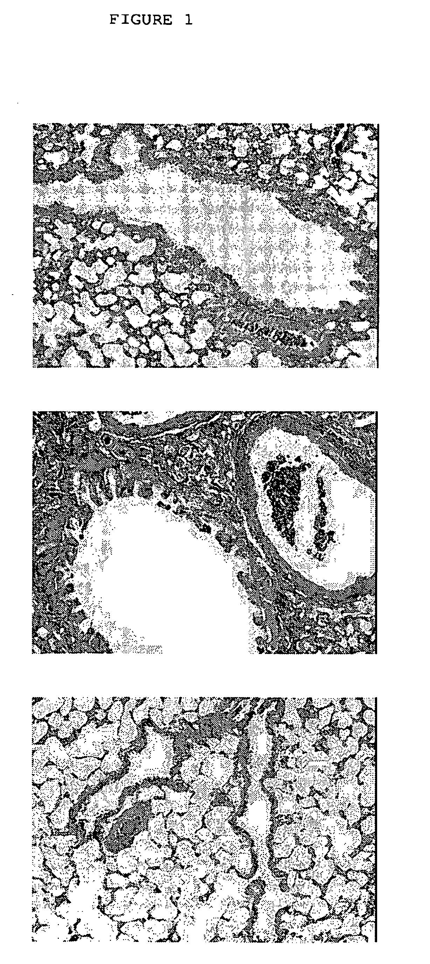

Figure 1A shows the result obtained with the control group, which received saline and the vehicle instead of bleomycin intratracheally.

Rats treated intratracheally with bleomycin and the vehicle developed severe lung fibrosis, as seen in Figure 1B. The alveoli have been largely replaced by fibroblasts and extracellular matrix and the normal lung structure is nearly obliterated.

Daily treatment of bleomycin-treated rats with 50 mg/kg of Example A (compound (134)) showed a consistent, nearly complete reversal of lung fibrosis in this model. A typical example is shown in Figure 1C. Alveoli are intact and little or no fibroblast infiltration or extracellular matrix deposition has occurred. Normal lung structure has been maintained, which is evidenced by a comparison of Figure 1C with Figure 1A.

### (B) Effect of a representative compound on expression of fibrotic marker genes following bleomycin-induced pulmonary fibrosis.

### mRNA extractions and synthesis of cDNA

One part of the frozen lung tissue dedicated to investigation of gene expression was cut into small pieces using a sterile scalpel blade. Approximately 100mg of tissue was then placed into a 2ml Eppendorf tube and 1.5ml of Trizol (Invitrogen) was added. A sterile tungsten carbide bead (Qiagen) was then added to the tube and the tube was placed in a Retsch MM300 Tissue disruptor (Qiagen) at a frequency of 30.0Hz for 8 minutes. After this time, the bead was removed and the sample centrifuged at 12000rpm for 10 minutes to remove tissue debris. The RNA was extracted using a modified version of the manufacturer's protocol supplied with Trizol. Briefly, 0.3ml chloroform was added to the tube and the tube shaken vigorously and then left to incubate at room temperature for 5 minutes, after which the tube was centrifuged for 15 minutes at 12000 rpm at 4°C. The upper colorless aqueous phase was then collected and added to 750µl isopropanol. This was then shaken vigorously and stored at -80°C overnight. The samples were then incubated at room temperature for 15 minutes, after which they were centrifuged for 40 minutes at 12000 rpm at 4°C. The supernatant was then removed and 500µl of 70% ethanol was added to wash the pellet then the sample was centrifuged for 10 minutes at 12000 rpm an 4°C, this wash step was repeated twice, after which the pellet was left to dry for 10 - 15 minutes. Finally the pellet was resuspended in 20µl RNase free water and stored at -80°C. The concentration of each sample was then measured using a spectrophotometer.

Using the Superscript^{™} III (Invitrogen, Paisley, UK) RT-first strand synthesis kit, 2µg of each mRNA sample was reversed transcribed using a modified version of the manufacturer's protocol. Briefly, a mixture of 2µg RNA, 1µl random hexamer primers (50ng/µl), 1µl dNTP mix (10mM) was made up to 10µl with DEPC-treated water and incubated at 65°C for 5 minutes, after which it was placed on ice for 5 minutes. Following this, to each reaction, 2µl RT buffer (10X), 4µl MgCl₂ (25mM), 2µl DTT (0.1M), 1µl RNaseOUT^{™} (40U/µl) and 1µl SuperScript™ III enzyme (200U/µl) was added and the mixture placed in a thermal cycler (Applied Biosystems) under the following conditions: 25° C for 10 minutes, 50°C for 50 minutes and 85°C for 5 minutes, after which 1µl of RNase H was added and incubated at 37°C for 20 minutes. The synthesized cDNA was diluted to 5ng/µl using the assumption that the RT reaction fully transcribed all of the mRNA to cDNA and was a concentration of 100ng/µl.

### Investigation of gene expression using real time PCR

Gene expression was investigated in each of the samples using the Applied Biosystems 7700 sequence detection system. Primers for the 18S endogenous control were purchased as pre-developed assay reagent kits, whereas primers and probes (see Table 2 below) for pro-collagen I and fibronectin were designed using PrimerExpress™ (Applied Biosystems), ensuring that at least one of the primers or probes in each set overlapped an intron / exon junction, thus eliminating the possibility of amplifying any contaminating genomic DNA in the cDNA sample. The purchased PDARs also amplified only cDNA.

**Table 2**

| Target | | Sequence |
|---|---|---|
| | | |
| Fibronectin | Forward | 5'-GAT GCC GAT CAG AAG TTT GGA-3' |
| | Reverse | 5'-TCG TTG GTC GTG CAG ATC TC-3' |
| | Probe | 5′-FAM-CTG CCC AAT GGC TGC CCA TGA- TAMRA-3′ |
| | | |
| Pro-Collagen I | Forward | 5′-CAG ACT GGC AAC CTG AAG AAG TC- 3′ |
| | Reverse | 5'-TCG CCC CTG AGC TCG AT-3' |
| | Probe | 5′-FAM-CTG CTC CTC CAG GGC TCC AAC GA-TAMRA3′ |

Real Time PCR was carried out in 25µl reactions, using 25ng (5µl) of cDNA per reaction. A quantitative PCR core kit was purchased (Eurogentec) and a master-mix was made up as follows for 100 reactions: 500µl 10X reaction buffer, 500µl MgCl₂ (50mM) , 200µl dNTP mix solution (5mM) , 25µl Hot Goldstar enzyme, 75µl 18S PDAR, 22.5µl forward primer, 22.5µl reverse primer, 15µl probe and 640µl DEPC treated water. 20µl of this master-mix was then added to 25ng (5µl) target cDNA. Each analysis was carried out in triplicate.

In order to quantify the gene expression, a standard curve was constructed for each primer set and was included on each plate. The standards were made up of a mix of all the cDNA's under investigation; this mix of cDNA's was serially diluted 10, 20, 50, 100, 100 times. A standard curve was constructed of the obtained C_{T} (Cycle at which amplification reaches a set Threshold) against the LOG₁₀ of the dilution factor. Curves were drawn for the target gene and the 18S rRNA endogenous control. The C_{T} value for both targets for each of the samples was then converted to a fold dilution using the standard curve and the target gene value was normalized to the 18S gene value.

### Statistics

All statistical analyses were carried out using GraphPad Prism V 4.02 software. Comparisons were made using a non-parametric T-test (Mann-Whitney U test) and a significant value was considered to be p = 0.05.

### Results

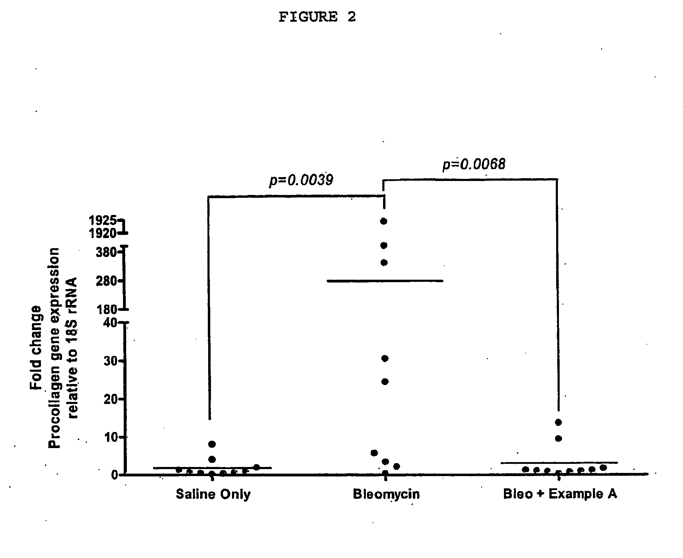

The results are shown in Figures 2 (procollagen I) and 3 (fibronectin). Each data point represents RNA isolated from the lung of a single rat.

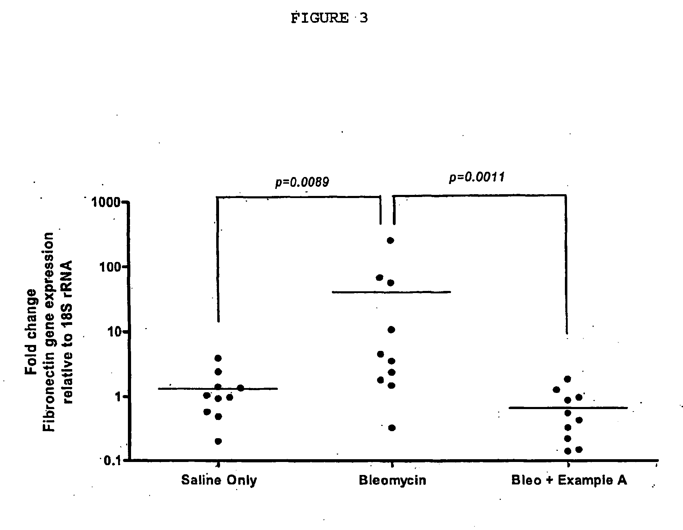

Intratracheal administration of bleomycin and subsequent treatment with vehicle only showed large increases in procollagen I and fibronectin gene expression in the lung, as seen in Figures 2 and 3, consistent with the histologically apparent lung fibrosis seen in Figure 1B.

Daily treatment of Bleomycin-treated rats with 50 mg/kg of Example A (compound (134)) showed a significant (p ≤ 0.0001) inhibition of expression of fibrotic marker genes in this model, as seen in Figures 2 and 3.

This experiment thus demonstrates that expression of fibrotic markers, and therefore deposition of extracellular matrix, may be dramatically reduced by treatment with Example A (compound (134)).

Thus, expression of fibrotic markers, and therefore deposition of extracellular matrix, may be dramatically reduced by treatment with the compounds in accordance with the present invention.

By reason of their biological properties the compounds according to the invention may be used in monotherapy or in conjunction with other pharmacologically active compounds. Such pharmacologically active compounds may be compounds which are, for example, also pharmacologically active in the treatment of fibrosis. Such pharmacologically active compounds may also be substances with a secretolytic, broncholytic and/or anti-inflammatory activity.

In a preferred embodiment in accordance with the present invention, such pharmacologically active compounds are preferably selected from the group consisting of anticholinergic agents, beta-2 mimetics, steroids, PDE-IV inhibitors, p38 MAP kinase inhibitors, NK₁ antagonists, LTD4 antagonists, EGFR inhibitors and endothelin-antagonists.

Anticholinergic agents may preferably be selected from the group consisting of the tiotropium salts, oxitropium salts, flutropium salts, ipratropium salts, glycopyrronium salts and trospium salts.

Beta-2 mimetics may preferably be selected from the beta-2 mimetics disclosed, for example, in US 4,460,581, which is incorporated herein by reference.

PDE-IV inhibitors may preferably be selected from the group consisting of enprofyllin, theophyllin, roflumilast, ariflo (cilomilast), CP-325,366, BY343, D-4396 (Sch-351591), AWD-12-281 (GW-842470), N-(3,5-dichloro-1-oxo-pyridin-4-yl)-4-difluoromethoxy-3-cyclopropylmethoxybenzamide, NCS-613, pumafentine, (-)p-[(4*a*R*,10*b*S*)-9-ethoxy-1,2,3,4,4a,10b-hexahydro-8-methoxy-2-methylbenzo[s][1,6]naphthyridin-6-yl]-N,N-diisopropylbenzamide, (R)-(+)-1-(4-bromobenzyl)-4-[(3-cyclopentyloxy)-4-methoxyphenyl]-2-pyrrolidone, 3-(cyclopentyloxy-4-methoxyphenyl)-1-(4-N'-[N-2-cyano-S-methyl-isothioureido]benzyl)-2-pyrrolidone, cis[4-cyano-4-(3-cyclopentyloxy-4-methoxyphenyl)cyclohexane-1-carbonic acid], 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], (R)-(+)-ethyl [4-(3-cyclopentyloxy-4-methoxyphenyl)pyrrolidin-2-yliden]acetate, (S)-(-)-ethyl[4-(3-cyclopentyloxy-4-methoxyphenyl)pyrrolidin-2-yliden]acetate, CDP840, Bay-198004, D-4418, PD-168787, T-440, T-2585, arofyllin, atizoram, V-11294A, C1-1018, CDC-801, CDC-3052, D-22888, YM-58997, Z-15370, 9-cyclopentyl-5,6-dihydro-7-ethyl-3-(2-thienyl)-9*H*-pyrazolo[3,9-c]-1,2,4-triazolo[4,3-a]pyridine and 9-cyclopentyl-5,6-dihydro-7-ethyl-3-(*tert*-butyl)-9*H*-pyrazolo[3,4-c]-1,2,4-triazolo[4,3-a]pyridine. These compounds may be used, as available, in the form of their racemates, enantiomers or diastereoisomers, or in the form of pharamacologically acceptable acid addition salts thereof, or in the form of their solvates and/or hydrates.

Steroids may preferably be selected from the group consisting of prednisolone, prednisone, butixocortpropionate, RPR-106541, flunisolid, beclomethasone, triamcinolone, budesonid, fluticasone, mometasone, ciclesonid, rofleponid, ST-126, dexamethasone, 6α,9α-difluoro-17α-[ (2-furanylcarbonyl) oxy]-11β-hydroxy-16α-methyl-3-oxo-androsta-1,4-dien-17β-carbothionic acid (S)-fluoromethylester, and 6α,9α-difluoro-11β-hydroxy-16α-methyl-3-oxo-17α-propionyloxy-androsta-1,4-diene-17β-carbothionic acid (S)-(2-oxo-tetrahydro-furan-3S-yl)ester. These compounds may be used, as available, in the form of their racemates, enantiomers or diastereoisomers, or in the form of pharamacologically acceptable acid addition salts thereof, or in the form of their solvates and/or hydrates.

p38 MAP kinase inhibitors may preferably be selected from the group consisting of the p38 Kinase inhibitors that are disclosed for instance in US Patents 5,716,972, US 5,686,455, US 5,656,644, US 5,593,992, US 5,593,991, US 5,663,334, US 5,670,527, US 5,559,137, 5,658,903, US 5,739,143, US 5,756,499, US 6,277,989, US 6,340,685, and US 5,716,955 and PCT applications WO 92/12154, WO 94/19350, WO 95/09853, WO 95/09851, WO 95/09847, WO 95/09852, WO 97/25048, WO 97/25047, WO 97/33883, WO 97/35856, WO 97/35855, WO 97/36587, WO 97/47618, WO 97/16442, WO 97/16441, WO 97/12876, WO 98/25619, WO 98/06715, WO 98/07425, WO 98/28292, WO 98/56377, WO 98/07966, WO 98/56377, WO 98/22109, WO 98/24782, WO 98/24780, WO 98/22457, WO 98/52558, WO 98/52559, WO 98/52941, WO 98/52937, WO 98/52940, WO 98/56788, WO 98/27098, WO 98/47892, WO 98/47899, WO 98/50356, WO 98/32733, WO 99/58523, WO 99/01452, WO 99/01131, WO 99/01130, WO 99/01136, WO 99/17776, WO 99/32121, WO 99/58502, WO 99/58523, WO 99/57101, WO 99/61426, WO 99/59960, WO 99/59959, WO 99/00357, WO 99/03837, WO 99/01441, WO 99/01449, WO 99/03484, WO 99/15164, WO 99/32110, WO 99/32111, WO 99/32463, WO 99/64400, WO 99/43680, WO 99/17204, WO 99/25717, WO 99/50238, WO 99/61437, WO 99/61440, WO 00/26209, WO 00/18738, WO 00/17175, WO 00/20402, WO 00/01688, WO 00/07980, WO 00/07991, WO 00/06563, WO 00/12074, WO 00/12497, WO 00/31072, WO 00/31063, WO 00/23072, WO 00/31065, WO 00/35911, WO 00/39116, WO 00/43384, WO 00/41698, WO 00/69848, WO 00/26209, WO 00/63204, WO 00/07985, WO 00/59904, WO 00/71535, WO 00/10563, WO 00/25791, WO 00/55152, WO 00/55139, WO 00/17204, WO 00/36096, WO 00/55120, WO 00/55153, WO 00/56738, WO 01/21591, WO 01/29041, WO 01/29042, WO 01/62731, WO 01/05744, WO 01/05745, WO 01/05746, WO 01/05749, WO 01/05751, WO 01/27315, WO 01/42189, WO 01/00208, WO 01/42241, WO 01/34605, WO 01/47897, WO 01/64676, WO 01/37837, WO 01/38312, WO 01/38313, WO 01/36403, WO 01/38314, WO 01/47921, WO 01/27089, DE 19842833, and JP 2000 86657 whose disclosures are all incorporated herein by reference in their entirety. Of particular interest for the combinations according to the invention are those p38 inhibitors disclosed in US 6,277,989, US 6,340,685, WO 00/12074, WO 00/12497, WO 00/59904, WO 00/71535, WO 01/64676, WO 99/61426, WO 00/10563, WO 00/25791, WO 01/37837, WO 01/38312, WO 01/38313, WO 01/38314, WO 01/47921, WO 99/61437, WO 99/61440, WO 00/17175, WO 00/17204, WO 00/36096, WO 98/27098, WO 99/00357, WO 99/58502, WO 99/64400, WO 99/01131, WO 00/43384, WO 00/55152, WO 00/55139, and WO 01/36403. In a preferred embodiment the p38 kinase inhibitor is selected from the compounds of following formula (I) as disclosed in WO 99/01131 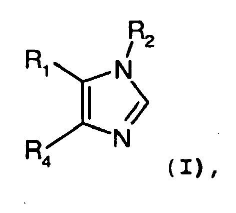 wherein
R₁ is 4-pyridyl, pyrimidinyl, 4-pyridazinyl, 1,2,4-triazin-5-yl, quinolyl, isoquinolinyl, or quinazolin-4-yl ring, which ring is substituted with Y-Rₐ and optionally with an additional independent substituent selected from C₁₋₄ alkyl, halogen, hydroxyl, C₁₋₄ alkoxy, C₁₋₄ akylthio, C₁₋₄ aklylsulfinyl, CH₂OR₁₂, amino, mono and di- C₁₋₆ alkyl substituted amino, an N-heterocyclyl ring which ring has from 5 to 7 members and optionally contains an additional heteroatom selected from oxygen, sulfur or NR₁₅, N(R₁₀)C(O)R_{b} or NHRₐ;
Y is oxygen or sulfur;
R₄ is phenyl, naphth-1-yl or naphthyl, or a heteroaryl, which is optionally substituted by one or two substituents, each of which is independently selected, and which, for a 4-phenyl, 4naphth-1-yl, 5-naphth-2-yl or 6-naphth-2-yl substituent, is halogen, cyano, nitro, C(Z)NR₇R₁₇, C(Z)OR₁₆, (CR₁₀R₂₀)ᵥCOR₁₂, SR₅, SOR₅, OR₁₂, halo-substituted-C₁₋₄ alkyl, C₁₋₄ alkyl, ZC(Z)R₁₂, NR₁₀C(Z)R₁₆, or (CR₁₀R₂₀)ᵥNR₁₀R₂₀ and which, for other positions of substitution, is halogen, cyano, C(Z)NR₁₃R₁₄, C(Z)OR₃, (CR₁₀R₂₀)ₘ"COR₃, S(O)ₘR₃, OR₃, halo-substituted-C₁₋₄ alkyl, C₁₋₄ alkyl, (CR₁₀R₂₀)_{m"}R₁₀C(Z)R₃, NR₁₀S(O)_{m'}R₈, NR₁₀S(O)_{m'}R₇R₁₇, ZC(Z)R₃ or (CR₁₀R₂₀)_{m''} NR₁₃R₁₄;
Z is oxygen or sulfur;
n is an integer having a value of 1 to 10;
m is 0, or integer 1 or 2;
m' is an integer having a value of 1 or 2;
m" is 0, or an integer having a value of 1 to 5;
v is 0, or an integer having a value of 1 to 2;
R₂ is -C (H) (A) (R₂₂);
A is optionally substituted aryl, heterocyclyl, or heteroaryl ring, or A is substituted C₁₋₁₀ alkyl;
R₂₂ is an optionally substituted C₁₋₁₀ alkyl;
Rₐ is aryl, arylC₁₋₆ alkyl, heterocyclic, heterocyclylC₁₋₆ alkyl, heteroaryl, heteroarylC₁₋₆alkyl, wherein each of these moieties may be optionally substituted;
R_{b} is hydrogen, C₁₋₆ alkyl, C₃₋₇ cycloalkyl, aryl, aryl C₁₋₄ alkyl, heteroaryl, heteroarylC₁₋₄ alkyl, heterocyclyl, or heterocyclylC₁₋₄ alkyl, wherein each of these moieties may be optionally substituted;
R₃ is heterocyclyl, heterocyclyl C₁₋₁₀ alkyl or R₈;
R₅ is hydrogen, C₁₋₄ alkyl, C₂₋₉ alkenyl, C₂₋₉ alkynyl or NR₇R₁₇, excluding the moieties SR₅ being SNR₇R₁₇ and SOR₅ being SOH;
R₆ is hydrogen, a pharmaceutically acceptable cation, C₁-₁₀ alkyl, C₃₋₇ cycloalkyl, aryl, aryl C₁₋₄ alkyl, heteroaryl, heteroaryl C₁₋₄ alkyl, heterocyclyl, aryl, or C₁₋₁₀ alkanoyl; R₇ and R₁₇ is each independently selected from hydrogen or
C₁₋₄ alkyl or R₇ and R₁₇ together with the nitrogen to which they are attached form a heterocyclic ring of 5 to 7 members which ring optionally contains an additional heteroatom selected from oxygen, sulfur or NR₁₅;
R₈ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, aryl C₁₋₁₀ alkyl, heteroaryl, heteroaryl C₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄; wherein the aryl, arylalkyl, heteroaryl, heteroaryl alkyl may be optionally substituted;
R₉ is hydrogen, C(Z) R₁₁ or optionally substituted C₁₋₁₀ alkyl, S(O)₂R₁₈, optionally substituted aryl or optionally substituted aryl C₁₋₄ alkyl;
R₁₀ and R₂₀ is each independently selected from hydrogen or C₁₋₄ alkyl;
R₁₁ is hydrogen, C₁₋₁₀ alkyl, C₃₋₇ cycloalkyl, heterocyclyl, heterocyclyl C₁₋₁₀ alkyl, aryl, arylC₁₋₁₀ alkyl, heteroaryl or heteroaryl C₁₋₁₀ alkyl, wherein these moieties may be optionally substituted;
R₁₂ is hydrogen or R₁₆;
R₁₃ an R₁₄ is each independently selected from hydrogen or optionally substituted
C₁₋₄ alkyl, optionally substituted aryl or optionally substituted arylC₁₋₄ alkyl, or together with the nitrogen which they are attached form a heterocyclic ring of 5 to 7 members which ring optionally contains an additional heteroatom selected from oxygen, sulfur or NR₉;
R₁₅ is R₁₀ or C(Z)-C₁₋₄ alkyl;
R₁₆ is C₁₋₄ alkyl, halo-substituted-C₁₋₄ alkyl, or C₃₋₇ cycloalkyl;
R₁₈ is C₁₋₁₀ alkyl, C₃₋₇ cycloalkyl, heterocyclyl, aryl, aryl₁₋₁₀ alkyl, heterocyclyl, heterocyclyl- C₁₋₁₀alkyl, heteroaryl or heteroaryl₁₋₁₀ alkyl;
or a pharmaceutically acceptable salt thereof.

NK₁ antagonists may preferably be selected from the group consisting of N-[2-(3,5-bis-trifluoromethyl-phenyl)-ethyl]-2-{4-cyclopropylmethyl-piperazin-1-yl}-N-methyl-2-phenyl-acetamide (BIIF 1149), CP-122721, FK-888, NKP 608C, NKP 608A, CGP 60829, SR 48968 (Saredutant), SR 140333 (Nolpitantium besilate/chloride), LY 303 870 (Lanepitant), MEN-11420 (Nepadutant), SB 223412, MDL-105172A, MDL-103896, MEN-11149, MEN-11467, DNK 333A, SR-144190, YM-49244, YM-44778, ZM-274773, MEN-10930, S-19752, Neuronorm, YM-35375, DA-5018, Aprepitant (MK-869) , L-754030, CJ-11974, L-758298, DNK-33A, 6b-I, CJ-11974, TAK-637, GR 205171 and the arylglycine amide derivates of general formula (VIII) 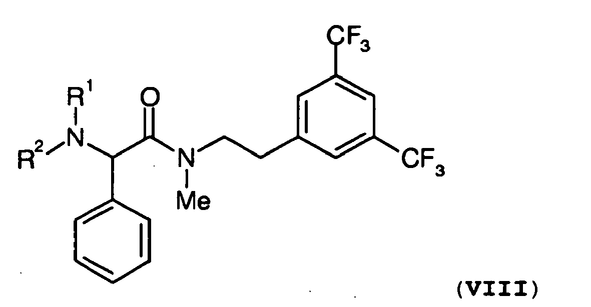 wherein
R¹ and R² together with the N-atom they are bound to form a ring of formula 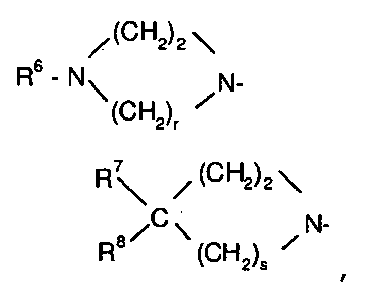 or
wherein r and s independently denote the number 2 or 3;
R⁶ denotes H, -C₁-C₅-alkyl, C₃-C₅-alkenyl, propinyl, hydroxy (C₂-C₄)alkyl, methoxy (C₂-C₉) alkyl, di (C₁-C₃) alkylamino (C₂-C₄) alkyl, amino (C₂-C₄) alkyl, amino, di (C₁-C₃)alkylamino, monofluoro- up to perfluoro(C₁-C₂)alkyl, N-methylpiperidinyl, pyridyl, pyrimidinyl, pyrazinyl or pyridazinyl,
R⁷ denotes any of the groups defined under (a) to (d):
(a) hydroxy
(b) 4-piperidinopiperidyl,
(c) 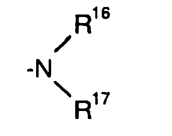 wherein R¹⁶ and R¹⁷ independently denote H, (C₁-C₄)alkyl, (C₃-C₆)cycloalkyl, hydroxy(C₂-C₄)alkyl, dihydroxy(C₂-C₄)alkyl, (C₁-C₃)alkoxy(C₂-C₄)alkyl, phenyl(C₁-C₄)alkyl or di(C₁-C₃)alkylamino(C₂-C₄)alkyl, and
   R⁸ denotes H,
   optionally in the form of enantiomers, mixtures of enantiomers or the racemates.

The compounds of formula (VIII) mentioned hereinbefore are described in WO 96/32386, WO 97/32865 and WO 02/32865. The disclosure of these international patent applications is incorporated herein by reference in its entirety.

LTD4 antagonists may preferably be selected from the group consisting of montelukast, 1-(((R)-(3-(2-(6,7-difluoro-2-quinolinyl)ethenyl)phenyl)-3-(2-(2- hydroxy-2-propyl)phenyl)thio)methylcyclopropane-acetate, 1-(((1(R)-3(3-(2-(2,3-dichlorothieno[3,2-b]pyridin-5-yl)-(E)-ethenyl)phenyl)-3-(2-(1-hydroxy-1-methylethyl)phenyl)propyl)thio)methyl)cyclopropane-acetate, pranlukast, zafirlukast, [2-[[2-(4-tert-butyl-2-thiazolyl)-5-benzofuranyl]oxymethyl]phenyl]acetate, MCC-847 (ZD-3523), MN-001, MEN-91507 (LM-1507), VUF-5078, VUF-K-8707 and L-733321. These compounds may be used, as available, in the form of their racemates, enantiomers or diastereoisomers, or in the form of pharamacologically acceptable acid addition salts thereof, or in the form of their solvates and/or hydrates.

EGFR inhibitorsmay preferably be selected from the group consisting of 4-[(3-chlor-4-fluorphenyl)amino]-6-{(4-(morpholin-4-yl)-1-oxo-2-buten-1-yl]amino}-7-cyclopropylmethoxy-chinazoline, 4-[(3-chlor-4-fluorphenyl)amino]-6-{[4-(N,N-diethylamino)-1-oxo-2-buten-1-yl]amino}-7-cyclopropylmethoxy-chinazoline, 4-[(3-chlor-4-fluorphenyl)amino]-6-{[4-(N,N-dimethylamino)-1-oxo-2-buten-1-yl]amino}-7-cyclopropylmethoxy-chinazoline, 4-[(*R*)-(1-phenyl-ethyl)amino]-6-{[4-(morpholin-4-yl)-1-oxo-2-buten-1-yl]amino}-7-cyclopentyloxy-chinazoline, 4-[(3-chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazoline, 4-[(3-chlor-4-fluor-phenyl)amino]-6-{[4-((*R*)-6-methyl-2-oxo-morpholin-4-yl)-1-oxo-2-buten-1-yl]amino}-7-[(*S*)-(tetrahydrofuran-3-yl)oxy]-chinazoline, 4-[(3-chlor-4-fluor-phenyl) amino]-6-{[4-((*R*)-2-methoxymethyl-6-oxo-morpholin-4-yl)-1-oxo-2-buten-1-yl]amino}-7-cyclopropylmethoxy-chinazoline, 4-[(3-chlor-4-fluor-phenyl)amino]-6-[2-((*S*)-6-methyl-2-oxo-morpholin-4-yl)-ethoxy]-7-methoxy-chinazoline, 4-[(3-chlor-4-fluorphenyl)amino]-6-({4-[N-(2-methoxy-ethyl)-N-methylamino]-1-oxo-2-buten-1-yl}amino)-7-cyclopropylmethoxy-chinazoline, 4-[(3-chlor-4-fluorphenyl)amino]-6-{[4-(N,N-dimethylamino)-1-oxo-2-buten-1-yl]amino}-7-cyclopentyloxy-chinazoline, 4-[(*R*)-(1-phenyl-ethyl) amino]-6-{[4-(N,N-bis-(2-methoxy-ethyl)-amino)-1-oxo-2-buten-1-yl]amino}-7-cyclopropylmethoxy-chinazoline, 4-[(*R)*-(1-phenylethyl)amino]-6-({4-[N-(2-methoxy-ethyl)-N-ethyl-amino]-1-oxo-2-buten-1-yl}amino)-7-cyclopropylmethoxy-chinazoline, 4-[(*R*)-(1-phenyl-ethyl)amino]-6-({4-[N-(2-methoxy-ethyl)-N-methyl-amino]-1-oxo-2-buten-1-yl}amino)-7-cyclopropylmethoxy-chinazoline, 4-[(*R*)-(1-phenylethyl)amino]-6-({4-[N-(tetrahydropyran-4-yl)-N-methylamino]-1-oxo-2-buten-1-yl}amino)-7-cyclopropylmethoxy-chinazoline, 4-[(3-chlor-4-fluorphenyl)amino]-6-{[4-(N,N-dimethylamino)-1-oxo-2-buten-1-yl]amino}-7-((*R*)-tetrahydrofuran-3-yloxy)-chinazoline, 4-[(3-chlor-4-fluorphenyl)amino]-6-{[4-(N,N-dimethylamino)-1-oxo-2-buten-1-yl]aminol-7-((*S*)-tetrahydrofuran-3-yloxy)-chinazoline, 4-[(3-chlor-4-fluorphenyl)amino]-6-({4-[N-(2-methoxy-ethyl)-N-methyl-amino]-1-oxo-2-buten-1-yl}amino)-7-cyclopentyloxy-chinazoline, 4-[(3-chlor-4-fluorphenyl)amino]-6-{[4-(N-cyclopropyl-N-methyl-amino)-1-oxo-2-buten-1-yl]amino}-7-cyclopentyloxy-chinazoline, 4-[(3-chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazoline, 4-[(3-chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazoline, 4-[(3-ethinyl-phenyl)amino]-6,7-bis-(2-methoxy-ethoxy)-chinazoline, 4-[(3-chlor-4-fluorphenyl)amino]-7-[3-(morpholin-4-yl)-propyloxy]-6-[(vinylcarbonyl)amino]-chinazoline, 4-[(*R*)-(1-phenyl-ethyl)amino]-6-(4-hydroxy-phenyl)-7H-pyrrolo[2,3-d]pyrimidine, 3-cyano-4-[(3-chlor-4-fluorphenyl)amino]-6-{[4-(N,N-dimethylamino)-1-oxo-2-buten-1-yl]amino}-7-ethoxy-chinoline, 4-{[3-chlor-4-(3-fluor-benzyloxy)-phenyl]amino}-6-(5-{[(2-methansulfonyl-ethyl)amino]methyl}-furan-2-yl)chinazoline, 4-[(R)-(1-phenyl-ethyl)amino]-6-{[4-((R)-6-methyl-2-oxo-morpholin-4-yl)-1-oxo-2-buten-1-yl]amino}-7-methoxy-chinazoline, 4-[(3-chlor-4-fluorphenyl)amino]-6-{[4-(morpholin-4-yl)-1-oxo-2-buten-1-yl}amino}-7-[(tetrahydrofuran-2-yl)methoxy]-chinazoline, 4-[(3-chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazoline, 4-[(3-ethinyl-phenyl)amino]-6-{[4-(5,5-dimethyl-2-oxo-morpholin-4-yl)-1-oxo-2-buten-1-yl]amino}-chinazoline, 4-[(3-chlor-4-fluor-phenyl)amino]-6-[2-(2,2-dimethyl-6-oxo-morpholin-4-yl)-ethoxy]-7-methoxy-chinazoline, 4-[(3-chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazoline, 4-[(3-chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazoline, 4-[(3-chlor-4-fluor-phenyl)amino]-6-{2-[4-(2-oxo-morpholin-4-yl)-piperidin-1-yl]-ethoxy}-7-methoxy-chinazoline, 4-[(3-chlor-4-fluor-phenyl)amino]-6-[1-(tert.-butyloxycarbonyl)-piperidin-4-yloxy]-7-methoxy-chinazoline, 4-[(3-chlor-4-fluor-phenyl)amino]-6-(trans-4-amino-cyclohexan-1-yloxy)-7-methoxy-chinazoline, 4-[(3-chlor-4-fluor-phenyl)amino]-6-(trans-4-methansulfonylamino-cyclohexan-1-yloxy)-7-methoxy-chinazoline, 4-[(3-chlor-4-fluor-phenyl)amino]-6-(tetrahydropyran-3-yloxy)-7-methoxy-chinazoline, 4-[(3-chlor-4-fluor-phenyl)amino]-6-(1-methyl-piperidin-4-yloxy)-7-methoxy-chinazoline, 4-[(3-chlor-4-fluor-phenyl)amino]-6-{1-[(morpholin-4-yl)carbonyl]-piperidin-4-yloxyl-7-methoxy-chinazoline, 4-[(3-chlor-4-fluor-phenyl)amino]-6-{1-[(methoxymethyl)carbonyl]-piperidin-4-yloxyl-7-methoxy-chinazoline, 4-[(3-chlor-4-fluor-phenyl)amino]-6-(piperidin-3-yloxy)-7-methoxy-chinazoline, 4-[(3-chlor-4-fluor-phenyl)amino]-6-[1-(2-acetylamino-ethyl)-piperidin-4-yloxy]-7-methoxy-chinazoline, 4-[(3-chlor-4-fludr-phenyl)amino]-6-(tetrahydropyran-4-yloxy)-7-ethoxy-chinazoline, 4-[(3-chlor-4-fluor-phenyl)amino]-6-((S)-tetrahydrofuran-3-yloxy)-7-hydroxy-chinazoline, 4-[(3-chlor-4-fluor-phenyl)amino]-6-(tetrahydropyran-4-yloxy)-7-(2-methoxy-ethoxy)-chinazoline, 4-[(3-chlor-4-fluor-phenyl)amino]-6-{trans-4-[(dimethylamino)sulfonylamino]-cyclohexan-1-yloxy}-7-methoxy-chinazoline, 4-[(3-chlor-4-fluor-phenyl)amino]-6-{trans-4-[(morpholin-4-yl)carbonylamino]-cyclohexan-1-yloxyl-7-methoxy-chinazoline, 4-[(3-chlor-4-fluor-phenyl)amino]-6-{trans-4-[(morpholin-4-yl)sulfonylamino]-cyclohexan-1-yloxy}-7-methoxy-chinazoline, 4-[(3-chlor-4-fluor-phenyl)amino]-6-(tetrahydropyran-4-yloxy)-7-(2-acetylamino-ethoxy)-chinazoline, 4-[(3-chlor-4-fluor-phenyl)amino]-6-(tetrahydropyran-4-yloxy)-7-(2-methansulfonylamino-ethoxy)-chinazoline, 4-[(3-chlor-4-fluor-phenyl)amino]-6-{1-[(piperidin-1-yl)carbonyl]-piperidin-4-yloxy}-7-methoxy-chinazoline, 4-[(3-chlor-4-fluor-phenyl)amino]-6-(1-aminocarbonylmethyl-piperidin-4-yloxy)-7-methoxy-chinazoline, 4-[(3-chlor-4-fluor-phenyl)amino]-6-(cis-4-{N-[(tetrahydropyran-4-yl)carbonyl]-N-methyl-amino}-cyclohexan-1-yloxy)-7-methoxy-chinazoline, 4-[(3-chlor-4-fluor-phenyl)amino]-6-(cis-4-{N-[(morpholin-4-yl)carbonyl]-N-methyl-amino}-cyclohexan-1-yloxy)-7-methoxy-chinazoline, 4-[(3-chlor-4-fluor-phenyl)amino]-6-(cis-4-{N-[(morpholin-4-yl)sulfonyl]-N-methyl-amino}-cyclohexan-1-yloxy)-7-methoxy- chinazoline, 4-[(3-chlor-4-fluor-phenyl)amino]-6-(trans-4-ethansulfonylamino-cyclohexan-1-yloxy)-7-methoxy-chinazoline, 4-[(3-chlor-4-fluor-phenyl)amino]-6-(1-methansulfonyl-piperidin-4-yloxy)-7-ethoxy-chinazoline, 4-[(3-chlor-4-fluor-phenyl)amino]-6-(1-methansulfonyl-piperidin-4-yloxy)-7-(2-methoxy-ethoxy)-chinazoline, 4-[(3-chlor-4-fluor-phenyl)amino]-6-[1-(2-methoxy-acetyl)-piperidin-4-yloxy]-7-(2-methoxy-ethoxy)-chinazoline, 4-[(3-chlor-4-fluor-phenyl)amino]-6-(cis-4-acetylamino-cyclohexan-1-yloxy)-7-methoxy-chinazoline, 4-[(3-Ethinyl-phenyl)amino]-6-[1-(tert.-butyloxycarbonyl)-piperidin-4-yloxy]-7-methoxy-chinazoline, 4-[(3-Ethinyl-phenyl)amino]-6-(tetrahydropyran-4-yloxy]-7-methoxy-chinazoline, 4-[(3-chlor-4-fluor-phenyl)amino]-6-(cis-4-{N-[(piperidin-1-yl)carbonyl]-N-methyl-amino}-cyclohexan-1-yloxy)-7-methoxy-chinazoline, 4-[(3-chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazoline, 4-[(3-chlor-4-fluor-phenyl)amino]-6-{cis-4-[(morpholin-4-yl)carbonylamino]-cyclohexan-1-yloxy}-7-methoxy-chinazoline, 4-[(3-chlor-4-fluor-phenyl)amino]-6-{1-[2-(2-oxopyrrolidin-1-yl)ethyl]-piperidin-4-yloxy}-7-methoxy-chinazoline, 4-[(3-chlor-4-fluor-phenyl)amino]-6-{1-[(morpholin-4-yl)carbonyl]-piperidin-4-yloxy}-7-(2-methoxy-ethoxy)-chinazoline, 4-[(3-Ethinyl-phenyl)amino]-6-(1-acetyl-piperidin-4-yloxy)-7-methoxy-chinazoline, 4-[(3-Ethinyl-phenyl)amino]-6-(1-methyl-piperidin-4-yloxy)-7-methoxy-chinazoline, 4-[(3-Ethinyl-phenyl)amino]-6-(1-methansulfonyl-piperidin-4-yloxy)-7-methoxy-chinazoline, 4-[(3-chlor-4-fluor-phenyl)amino]-6-(1-methyl-piperidin-4-yloxy)-7(2-methoxy-ethoxy)-chinazoline, 4-[(3-chlor-4-fluor-phenyl)amino]-6-(1-isopropyloxycarbonyl-piperidin-4-yloxy)-7-methoxy-chinazoline, 4-[(3-chlor-4-fluor-phenyl)amino]-6-(cis-4-methylamino-cyclohexan-1-yloxy)-7-methoxy-chinazoline, 4-[(3-chlor-4-fluor-phenyl)amino]-6-{cis-4-[N-(2-methoxy-acetyl)-N-methyl-amino]-cyclohexan-1-yloxy}-7-methoxy-chinazoline, 4-[(3-Ethinyl-phenyl)amino]-6-(piperidin-4-yloxy)-7-methoxy-chinazoline, 4-[(3-Ethinyl-phenyl)amino]-6-[1-(2-methoxy-acetyl)-piperidin-4-yloxy]-7-methoxy-chinazoline, 4-[(3-Ethinyl-phenyl)amino]-6-{1-[(morpholin-4-yl)carbonyl]-piperidin-4-yloxy}-7-methoxy-chinazoline, 4-[(3-chlor-4-fluor-phenyl)amino]-6-{1-[(cis-2,6-dimethyl-morpholin-4-yl)carbonyl]-piperidin-4-yloxy}-7-methoxy-chinazoline, 4-[(3-chlor-4-fluor-phenyl)amino]-6-{1-[(2-methyl-morpholin-4-yl)carbonyl]-piperidin-4-yloxy}-7-methoxy-chinazoline, 4-[(3-chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazoline, 4-[(3-chlor-4-fluor-phenyl)amino]-6-{1-[(N-methyl-N-2-methoxyethyl-amino)carbonyl]-piperidin-4-yloxy}-7-methoxy-chinazoline, 4-[(3-chlor-4-fluor-phenyl)amino]-6-(1-ethyl-piperidin-4-yloxy)-7-methoxy-chinazoline, 4-[(3-chlor-4-fluor-phenyl)amino]-6-{1-[(2-methoxyethyl)carbonyl]-piperidin-4-yloxy-7-methoxy-chinazoline, 4-[(3-chlor-4-fluor-phenyl)amino]-6-{1-[(3-methoxypropyl-amino)-carbonyl]-piperidin-4-yloxy}-7-methoxy-chinazoline, 4-[(3-chlor-4-fluor-phenyl)amino]-6-[cis-4-(N-methansulfonyl-N-methylamino)-cyclohexan-1-yloxy]-7-methoxy-chinazoline, 9-[(3-chlor-4-fluor-phenyl)amino]-6-[cis-4-(N-acetyl-N-methylamino)-cyclohexan-1-yloxyl-7-methoxy-chinazoline, 4-[(3-chlor-4-fluor-phenyl)amino]-6-(trans-4-methylamino-cyclohexan-1-yloxy)-7-methoxy-chinazoline, 4-[(3-chlor-4-fluor-phenyl)amino]-6-[trans-4-(N-methansulfonyl-N-methylamino)-cyclohexan-1-yloxy]-7-methoxy-chinazoline , 4-[(3-chlor-4-fluor-phenyl)amino]-6-(trans-4-dimethylamino-cyclohexan-1-yloxy)-7-methoxy-chinazoline , 4-[(3-chlor-4-fluor-phenyl)amino]-6-(trans-4-{N-[(morpholin-4-yl)carbonyl]-N-methyl-amino}-cyclohexan-1-yloxy)-7-methoxy-chinazoline, 4-[(3-chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazoline, 4-[(3-chlor-4-fluor-phenyl)amino]-6-(1-methansulfonyl-piperidin-4-yloxy)-7-methoxy-chinazoline, 4-[(3-chlor-4-fluor-phenyl)amino]-6-(1-cyano-piperidin-4-yloxy)-7-methoxy-chinazoline, Cetuximab, Trastuzumab, ABX-EGF and Mab ICR-62. These compounds may be used, as available, in the form of their racemates, enantiomers or diastereoisomers, or in the form of pharamacologically acceptable acid addition salts thereof, or in the form of their solvates and/or hydrates. These compounds are disclosed in the prior art, e.g. in WO 96/30347, WO 97/02266, WO 99/35146, WO 00/31048, WO 00/78735, WO 01/34574, WO 01/61816, WO 01/77104, WO02/18351, WO 02/18372, WO 02/18373, WO 02/18376, WO 02/50043, WO 03/082290, Cancer Research 2004, 64:11 (3958-3965), Am J Health-Syst Pharm 2000, 57(15), 2063-2076, Clinical Therapeutics 1999, 21(2), 309-318, WO 98/50433, and WO 95/20045.

Endothelin-antagonists may preferably be selected from the group consisting of tezosentan, bosentan, enrasentan, sixtasentan, T-0201, BMS-193884, K-8794, PD-156123, PD-156707, PD-160874, PD-180988, S-0139 and ZD-1611. Any reference to endothelin-antagonists within the scope of the present invention includes a reference to the salts, preferably pharmacologically acceptable acid addition salts, or derivatives which may be formed from the endothelin-antagonists.

These combinations may be administered either simultaneously or sequentially.

For pharmaceutical use the compounds according to the invention are preferably used for warm-blooded vertebrates, particularly humans, in doses of 0.0001-100 mg/kg of body weight.

These compounds may be administered either on their own or in conjunction with other active substances by intravenous, subcutaneous, intramuscular, intraperitoneal or intranasal route, by inhalation, or transdermally, or orally, whilst aerosol formulations are particularly suitable for inhalation.

For administration they are formulated with one or more conventional inert solid, semisolid or liquid carriers e.g. with starch, different types of cellulose, lactose, mannitol, sorbitol, glucose, calcium phosphate, hard fat, fatty alcohols, glycerol, medium chained triglycerides and related esters, polyethylene glycol, refined specialty oils, water, water/ethanol, water/glycerol, water/sorbitol, water/polyethylene glycol, propylene glycol,and/or functional excipients, e.g. with polyvinylpyrrolidone, hydroxypropylmethylcellulose, sodium carboxymethylcellulose, sodium starch glycolate, silicon dioxide, polysorbates, poloxamers, gelucires, magnesium stearate, citric acid, tartaric acid, or suitable mixtures thereof in conventional galenic preparations such as plain or coated tablets, capsules, powders, injectable solutions, ampoules, suspensions, solutions, sprays or suppositories.

The following examples of formulations illustrate the present invention without representing a limitation of its scope.

### Example F1: Coated tablet containing 75 mg of active substance

### Composition

### 1 tablet core contains:

| | |
|---|---|
| active substance | 75.0 mg |
| calcium phosphate | 131.0 mg |
| polyvinylpyrrolidone | 10.0 mg |
| carboxymethylcellulose sodium | 10.0 mg |
| silicon dioxide | 2.5 mg |
| magnesium stearate | 1.5 mg |
| | 230.0 mg |

### Preparation (direct compression)

The active substance is mixed with all components, sieved and compressed in a tablet-making machine to form tablets of the desired shape.

| | |
|---|---|
| Weight of core: | 230 mg |
| Appearance of core: | 9 mm, biconvex |

The tablet cores thus produced are coated with a film consisting essentially of hydroxypropylmethylcellulose.

| | |
|---|---|
| Weight of coated tablet: | 240 mg. |

### Example F2: Tablet containing 100 mg of active substance

### Composition

### 1 tablet contains:

| | |
|---|---|
| active substance | 100.0 mg |
| lactose | 80.0 mg |
| corn starch | 34.0 mg |
| hydroxypropylmethylcellulose | 4.0 mg |
| magnesium stearate | 2.0 mg |
| | 220.0 mg |

### Preparation (wet granulation)

The active substance, lactose and starch are mixed together and uniformly moistened with an aqueous solution of the hydroxypropylmethylcellulose. After the moist composition has been screened (2.0 mm mesh size) and dried in a rack-type drier at 50°C it is screened again (1.5 mm mesh size) and the lubricant is added. The finished mixture is compressed to form tablets.

| | |
|---|---|
| Weight of tablet: | 220 mg |
| Appearance of tablet: | 10 mm, flat faced |

with bevelled edges and breaking notch on one side.

### Example F3: Tablet containing 150 mg of active substance

### Composition

### 1 tablet contains:

| | |
|---|---|
| active substance | 150.0 mg |
| lactose | 85.0 mg |
| microcrystalline cellulose | 40.0 mg |
| polyvinylpyrrolidone | 10.0 mg |
| silicon dioxide | 10.0 mg |
| magnesium stearate | 5.0 mg |
| | 300.0 mg |

### Preparation (dry granulation)

The active substance mixed with lactose, polyvinylpyrrolidone,and parts of the microcrystalline cellulose, magnesium stearate is compacted e.g. on a roller compactor. The ribbons are broken up in fine granules through a screen with a mesh size of 0.8 mm. After subsequent sieving through a screen with a mesh size of 0.5 mm and blending with the remaining components, tablets are pressed from the mixture.

| | |
|---|---|
| Weight of tablet: | 300 mg |
| Appearance of tablet: | 10 mm, flat |

### Example F4: Hard gelatine capsule containing 150 mg of active substance

### Composition

### 1 capsule contains:

| | |
|---|---|
| active substance | 150.0 mg |
| lactose | 85.0 mg |
| microcrystalline cellulose | 40.0 mg |
| polyvinylpyrrolidone | 10.0 mg |
| silicon dioxide | 10.0 mg |
| magnesium stearate | 5.0 mg |
| | 300.0 mg |

### Preparation

The active substance mixed with lactose, polyvinylpyrrolidone,and parts of the microcrystalline cellulose, magnesium stearate is compacted e.g. on a roller compactor. The ribbons are broken up in fine granules through a screen with a mesh size of 0.8 mm. After subsequent sieving through a screen with a mesh size of 0.5 mm and blending with the remaining components, the finished mixture is packed into size 1 hard gelatine capsules.

| | |
|---|---|
| Capsule-filling: | approx. 300 mg |
| Capsule shell: | size 1 hard gelatine capsule. |

### Example F5: Suppository containing 150 mg of active substance

### 1 suppository contains:

| | |
|---|---|
| active substance | 150.0 mg |
| polyethyleneglycol 1500 | 800.0 mg |
| polyethyleneglycol 6000 | 850.0 mg |
| polyoxyl 40 hydrogenated castor oil | 200.0 mg |
| | 2,000.0 mg |

### Preparation

After the suppository mass has been melted the active substance is homogeneously distributed therein and the melt is poured into chilled moulds.

### Example F6: Suspension containing 50 mg of active substance 100 ml of suspension contains

| | |
|---|---|
| active substance | 1.00 g |
| carboxymethylcellulose sodium | 0.10 g |
| methyl p-hydroxybenzoate | 0.05 g |
| propyl p-hydroxybenzoate | 0.01 g |
| glucose | 10.00 g |
| glycerol | 5.00 g |
| 70% sorbitol solution | 20.00 g |
| flavouring | 0.30 g |
| dist. water ad | 100 ml |

### Preparation

The distilled water is heated to 70°C. The methyl and propyl p-hydroxybenzoates together with the glycerol and sodium salt of carboxymethylcellulose are dissolved therein with stirring. The solution is cooled to ambient temperature and the active substance is added and homogeneously dispersed therein with stirring. After the sugar, the sorbitol solution and the flavouring have been added and dissolved, the suspension is evacuated with stirring to eliminate air.

Thus, 5 ml of suspension contains 50 mg of active substance.

### Example F7: Ampoule containing 10 mg active substance

### Composition

| | |
|---|---|
| active substance | 10.0 mg |
| 0.01 N hydrochloric acid | q.s. |
| double-distilled water ad | 2.0 ml |

### Preparation

The active substance is dissolved in the necessary amount of 0.01 N HCl, made isotonic with sodium chloride, filtered sterile and transferred into a 2 ml ampoule.

### Example F8: Ampoule containing 50 mg of active substance

### Composition

| | |
|---|---|
| active substance | 50.0 mg |
| 0.01 N hydrochloric acid | q.s. |
| double-distilled water ad | 10.0 ml |

### Preparation

The active substance is dissolved in the necessary amount of 0.01 N HC1, made isotonic with sodium chloride, filtered sterile and transferred into a 10 ml ampoule.

Example F9: Capsule for powder inhalation containing 5 mg of active substance

### 1 capsule contains

| | |
|---|---|
| active substance | 5.0 mg |
| lactose for inhalation | 15.0 mg |
| | 20.0 mg |

### Preparation

The active substance is mixed with lactose for inhalation. The mixture is packed into capsules in a capsule-making machine (weight of the empty capsule approx. 50 mg).
weight of capsule: 70.0 mg
size of capsule = size 3

### Example F10: Solution for inhalation for a hand-held nebuliser containing 2.5 mg active substance

### 1 spray contains

| | |
|---|---|
| active substance | 2.500 mg |
| benzalkonium chloride | 0.001 mg |
| 1N hydrochloric acid q.s. | |
| ethanol/water (50/50) ad | 15.000 mg |

### Preparation

The active substance and benzalkonium chloride are dissolved in ethanol/water (50/50). The pH of the solution is adjusted with 1N hydrochloric acid. The resulting solution is filtered and transferred into suitable containers for use in hand-held nebulisers (cartridges).
Contents of the container: 4.5 g

## Claims

1. Use of the indolinones of general formula 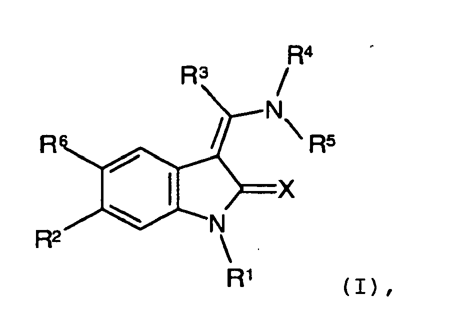 in which
X is an oxygen atom,
R¹ is a hydrogen atom,
R² is a fluorine, chlorine or bromine atom or a cyano group,
R³ is a phenyl group or a phenyl group which is monosubstituted by a fluorine, chlorine, bromine or iodine atom or by a C₁₋₃-alkoxy group, where the abovementioned unsubstituted and the monosubstituted phenyl groups may additionally be substituted in the 3- or 4-position
by a fluorine, chlorine or bromine atom,
by a cyano group,
by a C₁₋₃-alkoxy or C₁₋₂-alkyl-carbonyl-amino group,
by a cyano-C₁₋₃-alkyl, carboxy-C₁₋₃-alkyl, carboxy-C₁₋₄-alkoxy, carboxy-C₁₋₃-alkylamino, carboxy-C₁₋₃-alkyl-N-(C₁₋₃-alkyl)-amino, C₁₋₄-alkoxy-carbonyl-C₁₋₃-alkyl, C₁₋₄-alkoxy-carbonyl-C₁₋₃-alkoxy, C₁₋₄-alkoxy-carbonyl-C₁₋₃-alkylamino, C₁₋₄-alkoxy-carbonyl-C₁₋₃-alkyl-N-(C₁₋₃-alkyl)-amino, amino-C₁₋₃-alkyl, aminocarbonyl-C₁₋₃-alkyl, (C₁₋₂-alkylamino)-carbonyl-C₁₋₃-alkyl, di-(C₁₋₂-alkyl)-aminocarbonyl-C₁₋₃-alkyl, (C₁₋₂-alkyl-carbonyl)-amino-C₁₋₃-alkyl, (C₁₋₄-alkoxy-carbonyl)-amino-C₁₋₃-alkyl, (C₃₋₆-alkyl-carbonyl)-amino-C₁₋₃-alkyl, (phenyl-carbonyl) -amino-C₁₋₃-alkyl, (C₃₋₆-cycloalkyl-carbonyl)-amino-C₁₋₃-alkyl, (C₃₋₆-cycloalkyl-C₁₋₃-alkyl-carbonyl)-amino-C₁₋₃-alkyl, (thiophen-2-yl-carbonyl)-amino-C₁₋₃-alkyl, (furan-2-yl-carbonyl)-amino-C₁₋₃-alkyl, (phenyl-C₁₋₃-alkyl-carbonyl)-amino-C₁₋₃-alkyl, (2-(C₁₋₄-alkoxy)-benzoyl-carbonyl)-amino-C₁₋₃-alkyl, (pyridin-2-yl-carbonyl)-amino-C₁₋₃-alkyl, (pyridin-3-yl-carbonyl)-amino-C₁₋₃-alkyl-, (pyridin-4-yl-carbonyl)-amino-C₁₋₃-alkyl- or C₁₋₃-alkyl-piperazin-1-yl-carbonyl-C₁₋₃-alkyl group,
by a carboxy-C₂₋₃-alkenyl, aminocarbonyl-C₂₋₃-alkenyl, (C₁₋₃-alkylamino)-carbonyl-C₂₋₃-alkenyl, di-(C₁₋₃-alkyl)-amino-carbonyl-C₂₋₃-alkenyl or C₁₋₄-alkoxy-carbonyl-C₂₋₃-alkenyl group,
where the substituents may be identical or different,
R⁴ is a phenyl group or a phenyl group which is monosubstituted
by a C₁₋₃-alkyl group which is terminally substituted by an amino, guanidino, mono- or di-(C₁₋₂-alkyl)-amino-, N-[ω-di-(C₁₋₃-alkyl)-amino-C₂₋₃-alkyl]-N-(C₁₋₃-alkyl)-amino, N-methyl-(C₃₋₄-alkyl)-amino, N-(C₁₋₃-alkyl)-N-benzylamino, N-(C₁₋₄-alkoxycarbonyl)-amino, N-(C₁₋₄-alkoxycarbonyl)-C₁₋₄-alkylamino, 4-(C₁₋₃-alkyl)-piperazin-1-yl, imidazol-1-yl, pyrrolidin-1-yl, azetidin-1-yl, morpholin-4-yl, piperazin-1-yl, thiomorpholin-4-yl group,
by a di-(C₁₋₃-alkyl)-amino-(C₁₋₃-alkyl)-sulphonyl, 2-[di-(C₁₋₃-alkyl)-amino]-ethoxy, 4-(C₁₋₃-alkyl)-piperazin-1-yl-carbonyl, {ω-[di-(C₁₋₃-alkyl)-amino]-(C₂₋₃-alkyl))-N-(C₁₋₃-alkyl)-amino-carbonyl, 1-(C₁₋₃-alkyl) imidazol-2-yl, (C₁₋₃-alkyl)-sulphonyl group, or
by a group of the formula 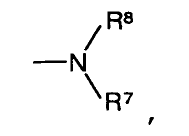 in which
R⁷ is a C₁₋₂-alkyl, C₁₋₂-alkyl-carbonyl, di-(C₁₋₂-alkyl)-amino-carbonyl-C₁₋₃-alkyl or C₁₋₃-alkylsulphonyl group and
R⁸ is C₁₋₃-alkyl, ω-(di-(C₁₋₂-alkyl)-amino]-C₂₋₃-alkyl, ω-[mono-(C₁₋₂-alkyl) -amino] -C₂₋₃-alkyl group, or
a (C₁₋₃-alkyl)-carbonyl, (C₄₋₆-alkyl)-carbonyl or carbonyl-(C₁₋₃-alkyl) group which is terminally substituted by a di-(C₁₋₂-alkyl)-amino, piperazin-1-yl or 4-(C₁₋₃-alkyl)-piperazin-1-yl group,
where all dialkylamino groups present in the radical R⁴ may also be present in quaternized form, for example as an N-methyl-(N,N-dialkyl)-ammonium group, where the counterion is preferably selected from the group consisting of iodide, chloride, bromide, methylsulphonate, para-toluenesulphonate and trifluoroacetate,
R⁵ is a hydrogen atom and
R⁶ is a hydrogen atom,
where the abovementioned alkyl groups include linear and branched alkyl groups in which additionally one to 3 hydrogen atoms may be replaced by fluorine atoms,
where additionally a carboxyl, amino or imino group present may be substituted by an in vivo cleavable radical or may be present in the form of a prodrug radical, for example in the form of a group which can be converted in vivo into a carboxyl group or in the form of a group which can be converted in vivo into an imino or amino group,
the tautomers, the diastereomers, the enantiomers, the mixtures thereof and the salts thereof,
for the preparation of a medicament for the prevention or treatment of a fibrotic disease selected from the group consisting of fibrosis and remodeling of lung tissue in chronic obstructive pulmonary disease, fibrosis and remodeling of lung tissue in chronic bronchitis, fibrosis and remodeling of lung tissue in emphysema, lung fibrosis and pulmonary diseases with a fibrotic component, fibrosis and remodeling in asthma.

2. Use in accordance with claim 1 wherein the substituted indolinone of general formula I is selected from the group consisting of:
(a) 3-Z-[1-(4-dimethylaminomethylanilino)-1-(3-(2-carboxyethyl)phenyl)methylene]-6-chloro-2-indolinone
(b) 3-Z-[1-(4-dimethylaminomethylanilino)-1-(4-(2-carboxyethyl)phenyl)methylene]-6-fluoro-2-indolinone
(c) 3-Z-[1-(4-dimethylaminomethylanilino)-1-(3-(2-carboxyethyl)phenyl)methylene]-6-fluoro-2-indolinone
(d) 3-Z-[1-(4-(N-(4-methylpiperazin-1-ylmethylcarbonyl)-N-methylamino)anilino)-1-(4-(2-carboxyethyl)phenyl)methylene]-6-fluoro-2-indolinone
(e) 3-Z-[1-(4-(N-(2-dimethylaminoethyl)-N-methylsulphonylamino)anilino)-1-(4-(2-carboxyethyl)phenyl)methylene]-6-fluoro-2-indolinone
(f) 3-Z-[1-(4-(N-(3-dimethylaminopropyl)-N-acetylamino)anilino)-1-(4-(2-carboxyethyl)phenyl)methylene]-6-fluoro-2-indolinone
(g) 3-Z-[1-(4-(1-methylimidazol-2-yl)anilino)-1-(4-(2-carboxyethyl)phenyl)methylene]-6-fluoro-2-indolinone
(h) 3-Z-[1-(4-(N-(dimethylaminomethylcarbonyl)-N-methylamino)anilino)-1-(4-(2-carboxyethyl)phenyl)methylene]-6-fluoro-2-indolinone
(i) 3-Z-[1-(4-(N-(2-dimethylaminoethylcarbonyl)-N-methylamino)anilino)-1-(4-(2-carboxyethyl)phenyl)methylene]-6-fluoro-2-indolinone
(j) 3-Z-[1-(4-(pyrrolidin-1-ylmethyl) anilino)-1-(4-(2-carboxyethyl)phenyl)methylene]-6-fluoro-2-indolinone
(k) 3-Z-[1-(4-(diethylaminomethyl)anilino)-1-(4-(2-carboxyethyl)phenyl)methylene]-6-fluoro-2-indolinone
(l) 3-Z-[1-(4-(2-dimethylaminoethyl)anilino)-1-(4-(2-carboxyethyl)phenyl)methylene]-6-chloro-2-indolinone
(m) 3-Z-[1-(4-dimethylaminomethylanilino)-1-(4-(2-carboxyethyl)phenyl)methylene]-6-chloro-2-indolinone
(n) 3-Z-[1-(4-(pyrrolidin-1-ylmethyl)anilino)-1-(4-(2-carboxyethyl)phenyl)methylene]-6-chloro-2-indolinone
(o) 3-Z-[1-(4-(pyrrolidin-1-ylmethyl)anilino)-1-(4-(2-carboxyethyl)phenyl)methylene]-6-bromo-2-indolinone
(p) 3-Z-[1-(4-(dimethylaminomethyl)anilino)-1-(4-(2-carboxyethyl)phenyl)methylene]-6-bromo-2-indolinone, and
(q) 3-Z-[1-(4-(diethylaminomethyl)anilino)-1-(4-(2-carboxyethyl)-methylene]-6-bromo-2-indolinone,
the tautomers, the diastereomers, the enantiomers, the mixtures thereof and the salts thereof.

3. Use in accordance with any one of claims 1 or 2 wherein the disease is selected from the group consisting of the lung fibrosis and pulmonary diseases with a fibrotic component selected from idiopathic pulmonary fibrosis, giant cell interstitial pneumonia, sarcodosis, cystic fibrosis, respiratory distress syndrome, drug-induced lung fibrosis, granulomatosis, silicosis, asbestosis, systemic scleroderma.

4. Use in accordance with any one of claims 1 to 3 wherein the disease is idiopathic pulmonary fibrosis.

5. Use in accordance with any one of claims 1 to 4 wherein the treatment is a combined treatment with a further pharmacologically active substance selected from the group consisting of anticholinergic agents, beta-2 mimetics, steroids, PDE-IV inhibitors, p38 MAP kinase inhibitors, NK₁ antagonists, LTD4 antagonists, EGFR inhibitors and endothelin-antagonists.

6. A pharmaceutical composition comprising a substituted indolinone of formula I as defined in any one of claims 1 or 2, optionally together with one or more pharmaceutically acceptable carriers or excipients, for the prevention or treatment of a fibrotic disease selected from the group consisting of fibrosis and remodeling of lung tissue in chronic obstructive pulmonary disease, fibrosis and remodeling of lung tissue in chronic bronchitis, fibrosis and remodeling of lung tissue in emphysema, lung fibrosis and pulmonary diseases with a fibrotic component, fibrosis and remodeling in asthma, radiation-induced fibrosis,

7. The pharmaceutical composition in accordance with claim 6, wherein the disease is selected from the group consisting of the lung fibrosis and pulmonary diseases with a fibrotic component selected from idiopathic pulmonary fibrosis, giant cell interstitial pneumonia, sarcodosis, cystic fibrosis, respiratory distress syndrome, drug-induced lung fibrosis, granulomatosis, silicosis, asbestosis, systemic scleroderma.

8. The pharmaceutical composition in accordance with claim 7 wherein the disease is idiopathic pulmonary fibrosis.

9. The pharmaceutical composition in accordance with any one of claims 6 to 8, wherein the treatment is a combined treatment with a further pharmacologically active substance selected from the group consisting of anticholinergic agents, beta-2 mimetics, steroids, PDE-IV inhibitors, p38 MAP kinase inhibitors, NK₁ antagonists, LTD4 antagonists, EGFR inhibitors and endothelin-antagonists.said further pharmacologically active substance being administered either simultaneously or sequentially.

10. Pharmaceutical composition containing a substituted indolinone of formula I as defined in any one of claims 1 or 2 in combination with a further pharmacologically active substance selected from the group consisting of anticholinergic agents, beta-2 mimetics, PDE-IV inhibitors, p38 MAP kinase inhibitors, NK₁ antagonists, LTD4 antagonists and endothelin-antagonists, optionally together with one or more pharmaceutically acceptable carriers or excipients.

## Patentansprüche

1. Verwendung der Indolinone der allgemeinen Formel 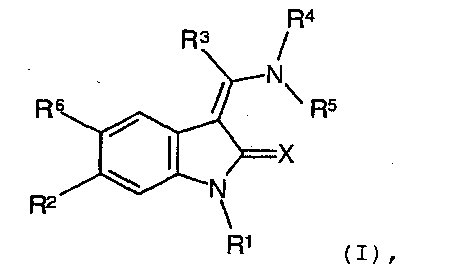 worin
X ein Sauerstoffatom darstellt,
R¹ ein Wasserstoffatom darstellt,
R² ein Fluor-, Chlor- oder Bromatom oder eine Cyanogruppe darstellt,
R³ eine Phenylgruppe darstellt oder eine Phenylgruppe, die monosubstituiert ist mit einem Fluor-, Chlor-, Brom- oder Iodatom oder mit einer C₁₋₃-Alkoxygruppe, wobei die zuvor erwähnten unsubstituierten und die monosubstituierten Phenylgruppen zusätzlich in der 3- oder 4-Position substituiert sein können
mit einem Fluor-, Chlor- oder Bromatom,
mit einer Cyanogruppe,
mit einer C₁₋₃-Alkoxy- oder C₁₋₂-Alkylcarbonylaminogruppe,
mit einer Cyano-C₁₋₃-alkyl-, Carboxy-C₁₋₃-alkyl-, Carboxy-C₁₋₄-alkoxy-, Carboxy-C₁₋₃-alkylamino-, Carboxy-C₁₋₃-alkyl-N(C₁₋₃-alkyl)amino-, C₁₋₄-Alkoxycarbonyl-C₁₋₃-alkyl-, C₁₋₄-Alkoxycarbonyl-C₁₋₃-alkoxy-, C₁₋₄-Alkoxycarbonyl-C₁₋₃-alkylamino-, C₁₋₄-Alkoxycarbonyl-C₁₋₃-alkyl-N-(C₁₋₃-alkyl)amino-, Amino-C₁₋₃-alkyl-, Aminocarbonyl-C₁₋₃-alkyl-, (C₁₋₂-Alkylamino)carbonyl-C₁₋₃-alkyl-, Di-(C₁₋₂-alkyl)aminocarbonyl-C₁₋₃-alkyl-, (C₁₋₂-Alkylcarbonyl)amino-C₁₋₃-alkyl-, (C₁₋₄-Alkoxycarbonyl)amino-C₁₋₃-alkyl-, (C₃₋₆-Alkylcarbonyl)amino-C₁₋₃-alkyl-, (Phenylcarbonyl)amino-C₁₋₃-alkyl-, (C₃₋₆-Cycloalkylcarbonyl)amino-C₁₋₃-alkyl-, (C₃₋₆-Cycloalkyl-C₁₋₃-alkylcarbonyl)amino-C₁₋₃-alkyl-, (Thiophen-2-yl-carbonyl)amino-C₁₋₃-alkyl-, (Furan-2-yl-carbonyl)amino-C₁₋₃-alkyl-, (Phenyl-C₁₋₃-alkylcarbonyl)amino-C₁₋₃-alkyl-, (2-(C₁₋₄-alkoxy)benzoylcarbonyl)amino-C₁₋₃-alkyl-, (Pyridin-2-yl-carbonyl)amino-C₁₋₃-alkyl-, (Pyridin-3-yl-carbonyl)amino-C₁₋₃-alkyl-, (Pyridin-4-yl-carbonyl)amino-C₁₋₃-alkyl- oder C₁₋₃-Alkylpiperazin-1-yl-carbonyl-C₁₋₃-alkyl-Gruppe,
mit einer Carboxy-C₂₋₃-alkenyl-, Aminocarbonyl-C₂₋₃-alkenyl-, (C₁₋₃-Alkylamino)carbonyl-C₂₋₃-alkenyl-, Di-(C₁₋₃-Alkyl)amino-carbonyl-C₂₋₃-alkenyl oder C₁₋₄-Alkoxycarbonyl-C₂-₃-alkenyl-Gruppe, wobei die Substituenten identisch oder verschieden sein können,
R⁴ eine Phenylgruppe darstellt oder eine Phenylgruppe, die monosubstituiert ist mit einer C₁₋₃-Alkylgruppe, die endständig substituiert ist mit einer Amino-, Guanidino-, Mono- oder Di-(C₁₋₂-alkyl)amino-, N-[ω-Di-(C₁₋₃-alkyl)amino-C₂₋₃-alkyl]-N-(C₁₋₃-alkyl)amino-, N-Methyl-(C₃₋₄-alkyl)amino-, N-(C₁₋₃-alkyl)-N-benzylamino-, N-(C₁₋₄-Alkoxycarbonyl)amino-, N-(C₁₋₄-Alkoxycarbonyl)-C₁₋₄-alkylamino-, 4-(C₁₋₃-Alkyl)piperazin-1-yl-, Imidazol-1-yl-, Pyrrolidin-1-yl-, Azetidin-1-yl-, Morpholin-4-yl-, Piperazin-1-yl-, Thiomorpholin-4-yl-Gruppe,
mit einer Di-(C₁₋₃-alkyl)amino-(C₁₋₃-alkyl)sulfonyl-, 2-[Di-(C₁₋₃-Alkyl)amino]ethoxy-, 4-(C₁₋₃-Alkyl)piperazin-1-yl-carbonyl-, {ω-[Di-(C₁₋₃-Alkyl)-amino]-(C₂₋₃-alkyl)}-N-(C₁₋₃-alkyl)aminocarbonyl-, 1-(C₁₋₃-Alkyl)imidazol-2-yl-, (C₁₋₃-Alkyl)sulfonyl-Gruppe oder
mit einer Gruppe der Formel 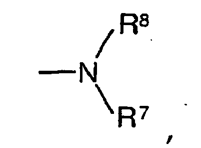 worin
R⁷ eine C₁₋₂-Alkyl-, C₁₋₂-Alkylcarbonyl-, Di-(C₁₋₂-alkyl)aminocarbonyl-C₁₋₃-alkyl- oder C₁₋₃-Alkylsulfonyl-Gruppe darstellt und
R⁸ eine C₁₋₃-Alkyl-, ω-[Di-(C₁₋₂-alkyl)amino]-C₂₋₃-alkyl-, ω-[Mono-(C₁₋₂-alkyl)amino]-C₂₋₃-alkyl-Gruppe darstellt oder
eine (C₁₋₃-Alkyl)carbonyl-, (C₄₋₆-Alkyl)carbonyl- oder Carbonyl-(C₁₋₃-alkyl)-Gruppe, die endständig substituiert ist mit einer Di-(C₁₋₂-alkyl)amino-, Piperazin-1-yl- oder 4-(C₁₋₃-Alkyl)piperazin-1-yl-Gruppe,
worin sämtliche vorliegenden Dialkylaminogruppen im Rest R⁴ ebenfalls in quatemisierter Form vorliegen können, beispielsweise als eine N-Methyl-(N,N-dialkyl)ammoniumgruppe, wobei das Gegenion bevorzugt ausgewählt ist aus der Gruppe, bestehend aus Iodid, Chlorid, Bromid, Methylsulfonat, para-Toluolsulfonat und Trifluoracetat,
R⁵ ein Wasserstoffatom ist und
R⁶ ein Wasserstoffatom ist,
wobei die zuvor erwähnten Alkylgruppen lineare und verzweigte Alkylgruppen umfassen, in denen zusätzlich 1 bis 3 Wasserstoffatome durch Fluoratome ersetzt sein können,
wobei zusätzlich eine vorliegende Carboxyl-, Amino- oder Iminogruppe mit einem in vivo spaltbaren Rest substituiert sein kann, oder in Form eines Propharmakonrests vorliegen kann, beispielsweise in Form einer Gruppe, die in vivo in eine Carboxylgruppe umgewandelt werden kann, oder in Form einer Gruppe, die in vivo in eine Imino- oder Aminogruppe umgewandelt werden kann,
den Tautomeren, den Diastereomeren, den Enantiomeren, den Mischungen hiervon und den Salzen hiervon,
zur Herstellung eines Medikaments zur Vorbeugung oder Behandlung einer fibrotischen Erkrankung, ausgewählt aus der Gruppe, bestehend aus Fibrose und Umbildung von Lungengewebe bei chronisch obstruktiver pulmonaler Erkrankung, Fibrose und Umbildung von Lungengewebe bei chronischer Bronchitis, Fibrose und Umbildung von Lungengewebe bei einem Emphysem, Lungenfibrose und pulmonale Erkrankungen mit einer fibrotischen Komponente, Fibrose und Umbildung bei Asthma.

2. Verwendung nach Anspruch 1, wobei das substituierte Indolinon der allgemeinen Formel I ausgewählt ist aus der Gruppe, bestehend aus:
(a) 3-Z-[1-(4-Dimethylaminomethylanilino)-1-(3-(2-carboxyethyl)phenyl)methylen]-6-chlor-2-indolinon,
(b) 3-Z-[1-(4-Dimethylaminomethylanilino)-1-(4-(2-carboxyethyl)phenyl)-methylen]-6-fluor-2-indolinon,
(c) 3-Z-[1-(4-Dimethylaminomethylanilino)-1-(3-(2-carboxyethyl)phenyl)-methylen]-6-fluor-2-indolinon,
(d) 3-Z-[1-(4-(N-(4-Methylpiperazin-1-yl-methylcarbonyl)-N-methylamino)-anilino)-1-(4-(2-carboxyethyl)phenyl)methylen]-6-fluor-2-indolinon,
(e) 3-Z-[1-(4-(N-(2-dimethylaminoethyl)-N-methylsulfonylamino)anilino)-1-(4-(2-carboxyethyl)phenyl)methylen]-6-fluor-2-indolinon,
(f) 3-Z-[1-(4-(N-(3-dimethylaminopropyl)-N-acetylamino)anilino)-1-(4-(2-carboxyethyl)phenyl)methylen]-6-fluor-2-indolinon,
(g) 3-Z-[1-(4-(1-Methylimidazol-2-yl)anilino)-1-(4-(2-carboxyethyl)phenyl)-methylen]-6-fluor-2-indolinon,
(h) 3-Z-[1-(4-(N-(Dimethylaminomethylcarbonyl)-N-methylamino)anilino)-1-(4-(2-carboxyethyl)phenyl)methylen]-6-fluor-2-indolinon,
(i) 3-Z-[1-(4-(N-(2-Dimethylaminoethylcarbonyl)-N-methylamino)anilino)-1-(4-(2-carboxyethyl)phenyl)methylen]-6-fluor-2-indolinon,
(j) 3-Z-[1-(4-(Pyrrolidin-1-ylmethyl)anilino)-1-(4-(2-carboxyethyl)-phenyl)methylen]-6-fluor-2-indolinon,
(k) 3-Z-[1-(4-(Diethylaminomethyl)anilino)-1-(4-(2-carboxyethyl)-phenyl)methylen]-6-fluor-2-indolinon,
(l) 3-Z-[1-(4-(2-Dimethylaminoethyl)anilino)-1-(4-(2-carboxyethyl)-phenyl)methylen]-6-chlor-2-indolinon,
(m) 3-Z-[1-(4-(Dimethylaminomethylanilino)-1-(4-(2-carboxyethyl)-phenyl)methylen]-6-chlor-2-indolinon,
(n) 3-Z-[1-(4-(Pyrrolidin-1-yl-methyl)anilino)-1-(4-(2-carboxyethyl)-phenyl)methylen]-6-chlor-2-indolinon,
(o) 3-Z-[1-(4-(Pyrrolidin-1-yl-methyl)anilino)-1-(4-(2-carboxyethyl)-phenyl)methylen]-6-brom-2-indolinon,
(p) 3-Z-[1-(4-(Dimethylaminomethyl)anilino)-1-(4-(2-carboxyethyl)phenylmethylen]-6-brom-2-indolinon und
(q) 3-Z-[1-(4-(Diethylaminomethyl)anilino)-1-(4-(2-carboxyethyl)methylen]-6-brom-2-indolinon,
den Tautomeren, den Diastereomeren, den Enantiomeren, den Mischungen hiervon und den Salzen hiervon.

3. Verwendung nach irgendeinem der Ansprüche 1 oder 2, wobei die Krankheit ausgewählt ist aus der Gruppe, bestehend aus Lungenfibrose und pulmonaler Erkrankungen mit einer fibrotischen Komponente, ausgewählt aus idiopathischer pulmonaler Fibrose, interstitieller Riesenzellenpneumonie, Sarcodose, cystischer Fibrose, respiratory distress-Syndrom, Arzneimittel-induzierter Lungenfibrose, Granulomatose, Silicose, Asbestose, systemischer Sklerodermie.

4. Verwendung nach einem der Ansprüche 1 bis 3, wobei die Erkrankung idiopathische pulmonale Fibrose darstellt.

5. Verwendung nach einem der Ansprüche 1 bis 4, wobei die Behandlung eine kombinierte Behandlung mit einer weiteren pharmakologisch aktiven Substanz darstellt, ausgewählt aus der Gruppe, bestehend aus anticholinergen Mitteln, β-2-Mimetika, Steroiden, PDE-IV-Inhibitoren, p38-MAP-Kinase-Inhibitoren, NK₁-Antagonisten, LTD4-Antagonisten, EGFR-Inhibitoren und Endothelin-Antagonisten.

6. Pharmazeutische Zusammensetzung, umfassend ein substituiertes Indolinon der Formel I, wie in irgendeinem der Ansprüche 1 oder 2 definiert, gegebenenfalls zusammen mit einem oder mehreren pharmazeutisch akzeptablen Trägern oder Hilfsstoffen zur Vorbeugung oder Behandlung einer fibrotischen Erkrankung, ausgewählt aus der Gruppe, bestehend aus Fibrose und Umbildung von Lungengewebe bei chronisch obstruktiver pulmonaler Erkrankung, Fibrose und Umbildung von Lungengewebe bei chronischer Bronchitis, Fibrose und Umbildung von Lungengewebe bei einem Emphysem, Lungenfibrose und pulmonalen Erkrankungen mit einer fibrotischen Komponente, Fibrose und Umbildung bei Asthma, strahlungsinduzierter Fibrose.

7. Pharmazeutische Zusammensetzung nach Anspruch 6, wobei die Krankheit ausgewählt ist aus der Gruppe, bestehend aus Lungenfibrose und pulmonalen Erkrankungen mit einer fibrotischen Komponente, ausgewählt aus idiopathischer pulmonaler Fibrose, interstitieller Riesenzellenpneumonie, Sarcodose, cystischer Fibrose, respiratory distress-Syndrom, Arzneimittel-induzierter Lungenfibrose, Granulomatose, Silicose, Asbestose, systemischer Sklerodermie.

8. Pharmazeutische Zusammensetzung nach Anspruch 7, wobei die Erkrankung idiopathische pulmonale Fibrose darstellt.

9. Pharmazeutische Zusammensetzung nach irgendeinem der Ansprüche 6 bis 8, wobei die Behandlung eine kombinierte Behandlung mit einer weiteren pharmakologisch aktiven Substanz darstellt, ausgewählt aus der Gruppe, bestehend aus anticholinergen Mitteln, β-2-Mimetika, Steroiden, PDE-IV-Inhibitoren, p38-MAP-Kinase-Inhibitoren, NK₁-Antagonisten, LTD4-Antagonisten, EGFR-Inhibitoren und Endothelin-Antagonisten, wobei eine weitere pharmakologisch aktive Substanz entweder simultan oder darauf folgend verabreicht wird.

10. Pharmazeutische Zusammensetzung, enthaltend ein substituiertes Indolinon der Formel I, wie in irgendeinem der Ansprüche 1 oder 2 definiert, in Kombination mit einer weiteren pharmakologisch aktiven Substanz, ausgewählt aus der Gruppe, bestehend aus anticholinergen Mitteln, β-2-Mimetika, PDE-IV-Inhibitoren, p38-MAP-Kinase-Inhibitoren, NK₁-Antagonisten, LTD4-Antagonisten und Endothelin-Antagonisten, gegebenenfalls zusammen mit einem oder mehreren pharmakologisch akzeptablen Trägem oder Hilfsstoffen.

## Revendications

1. Utilisation des indolinones de formule générale 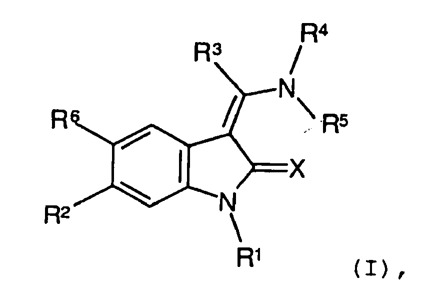 dans laquelle
X est un atome d'oxygène,
R¹ est un atome d'hydrogène,
R² est un atome de fluor, de chlore ou de brome ou un groupe cyano,
R³ est un groupe phényle ou un groupe phényle qui est monosubstitué par un atome de fluor, de chlore, de brome ou d'iode ou par un groupe alcoxy en C₁-C₃, les groupes phényle non substitué et monosubstitué précités pouvant en outre être substitués en position 3 ou 4
par un atome de fluor, de chlore ou de brome,
par un groupe cyano,
par un groupe alcoxy en C₁-C₃ ou (alkyl en C₁-C₂)-carbonyl-amino,
par un groupe cyano-(alkyle en C₁-C₃), carboxy-(alkyle en C_{I}-C₃), carboxy-(alcoxy en C₁-C₄), carboxy-(alkylamino en C₁-C₃), carboxy-(alkyl en C₁-C₃)-N-(alkyl en C₁-C₃)-amino, (alcoxy en C₁-C₄)-carbonyl-(alkyle en C₁-C₃), (alcoxy en C₁-C₄)-carbonyl-(alcoxy en C₁-C₃), (alcoxy en C₁-C₄)-carbonyl-(alkylamino en C₁-C₃), (alcoxy en C₁-C₄)-carbonyl-(alkyl en C₁-C₃)-N-(alkyl en C₁-C₃)-amino, amino-alkyle en C₁-C₃, aminocarbonyl-(alkyle en C₁-C₃), (alkylamino en C₁-C₂)-carbonyl-(alkyle en C₁-C₃), di(alkyl en C₁-C₂)-aminocarbonyl-(alkyle en C₁-C₃), (alkyl en C₁-C₂-carbonyl)-amino-(alkyle en C₁-C₃), [(alcoxy en C₁-C₄)-carbonyl]-amino-(alkyle en C₁-C₃), [(alkyl en C₃-C₆)-carbonyl]-amino-(alkyle en C₁-C₃), (phényl-carbonyl)-amino-(alkyle en C₁-C₃), ((cycloalkyl en C₃-C₆)-carbonyl)-amino-(alkyle en C₁-C₃), ((cycloalkyl en C₃-C₆)-(alkyl en C₁-C₃)-carbonyl)-amino-(alkyle en C₁-C₃), (thiophén-2-ylcarbonyl)-amino-(alkyle en C₁-C₃), (furan-2-ylcarbonyl)-amino-(alkyle en C₁-C₃), [phényl-(alkyl en C₁-C₃)-carbonyl]amino-(alkyle en C₁-C₃), [2-(alcoxy en C₁-C₄)-benzoylcarbonyl]amino-(alkyle en C₁-C₃), (pyridin-2-ylcarbonyl)-amino-(alkyle en C₁-C₃), (pyridin-3-ylcarbonyl)-amino-(alkyle en C₁-C₃), (pyridin-4-ylcarbonyl)-amino-(alkyle en C₁-C₃) ou (alkyl en C₁-C₃)-pipérazin-1-ylcarbonyl-(alkyle en C₁-C₃),
par un groupe carboxy-(alcényle en C₂-C₃), aminocarbonyl-(alcényle en C₂-C₃), (alkylamino en C₁-C₃)-carbonyl-(alcényle en C₂-C₃), di(alkyl en C₁-C₃)aminocarbonyl-(alcényle en C₂-C₃) ou (alcoxy en C₁-C₄)carbonyl-(alcényle en C₂-C₃),
les substituants pouvant être identiques ou différents,
R⁴ est un groupe phényle ou un groupe phényle qui est monosubstitué
par un groupe alkyle en C₁-C₃ qui est substitué en position terminale par un groupe amino, guanidino, mono- ou di(alkyl en C₁-C₂)-amino, N-[ω-di(alkyl en C₁-C₃)-amino-(alkyle en C₂-C₃)]-N-(alkyl en C₁-C₃)-amino, N-méthyl-(alkyl en C₃-C₄)-amino, N-(alkyl en C₁-C₃)-N-benzylamino, N-(alcoxycarbonyl en C₁-C₄)-amino, N-(alcoxycarbonyl en C₁-C₄)-(alkylamino en C₁-C₄), 4-(alkyl en C₁-C₃)-pipérazin-1-yle, imidazol-1-yle, pyrrolidin-1-yle, azétidin-1-yle, morpholin-4-yle, pipérazin-1-yle, thiomorpholin-4-yle,
par un groupe di(alkyl en C₁-C₃)-amino-(alkyl en C₁-C₃)-sulfonyle, 2-[di(alkyl en C₁-C₃)-amino]-éthoxy, 4-(alkyl en C₁-C₃)-pipérazin-1-ylcarbonyle, {ω-[di-(alkyl en C₁-C₃)-amino]-(alkyl en C₂-C₃)}-N-(alkyl en C₁-C₃)-aminocarbonyle, 1-(alkyl en C₁-C₃)imidazol-2-yle, alkylsulfonyle en C₁-C₃, ou
par un groupe de formule 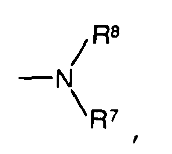 dans lequel
R⁷ est un groupe alkyle en C₁-C₂, (alkyl en C₁-C₂)-carbonyle, di(alkyl en C₁-C₂)-aminocarbonyl-(alkyle en C₁-C₃) ou alkylsulfonyle en C₁-C₃, et
R⁸ est un groupe alkyle en C₁-C₃, ω-[di(alkyl en C₁-C₂)amino]-(alkyle en C₂-C₃), ou
un groupe (alkyl en C₁-C₃)-carbonyle, (alkyl en C₄-C₆)-carbonyle ou carbonyl-(alkyle en C₁-C₃) qui est substitué en position terminale par un groupe di-(alkyl en C₁-C₂)-amino, pipérazin-1-yle ou 4-(alkyl en C₁-C₃)-pipérazin-1-yle;
tous les groupes dialkylamino présents dans le radical R⁴ pouvant aussi être présents sous forme quaternaire, par exemple sous forme d'un groupe N-méthyl-(N,N-dialkyl)ammonium, le contre-ion étant de préférence choisi dans le groupe constitué par les ions iodure, chlorure, bromure, méthylsulfonate, p-toluènesulfonate et trifluoroacétate,
R⁵ est un atome d'hydrogène et
R⁶ est un atome d'hydrogène,
où les groupes alkyle précités comprennent des groupes alkyle linéaires et ramifiés dans lesquels, de plus, 1 à 3 atomes d'hydrogène peuvent être remplacés par des atomes de fluor,
et où en outre un groupe carboxyle, amino ou imino présent peut être substitué par un radical dissociable *in vivo* ou peut être présent sous la forme d'un radical de promédicament, par exemple sous forme d'un groupe qui peut être converti *in vivo* en un groupe carboxyle ou sous forme d'un groupe qui peut être converti *in vivo* en un groupe imino ou amino,
de leurs tautomères, diastéréoisomères, énantiomères, leurs mélanges et leurs sels,
pour la préparation d'un médicament destiné à la prévention ou au traitement d'une maladie fibrogène choisie dans le groupe constitué par la fibrose et le remodelage du tissu pulmonaire dans la bronchopneumopathie chronique obstructive, la fibrose et le remodelage du tissu pulmonaire dans la bronchite chronique, la fibrose et le remodelage du tissu pulmonaire dans l'emphysème, la fibrose pulmonaire et les maladies pulmonaires ayant une composante fibrogène, la fibrose et le remodelage dans l'asthme.

2. Utilisation selon la revendication 1, dans laquelle l'indolinone substituée de formule générale 1 est choisie dans le groupe constitué par:
(a) la 3-Z-[1-(4-diméthylaminométhylanilino)-1-(3-(2-carboxyéthyl)phényl)méthylène]-6-chloro-2-indolinone
(b) la 3-Z-[1-(4-diméthylaminométhylanilino)-1-(4-(2-carboxyéthyl)phényl)méthylène]-6-fluoro-2-indolinone
(c) la 3-Z-[1-(4-diméthylaminométhylanilino)-1-(3-(2-carboxyéthyl)phényl)méthylène]-6-fluoro-2-indolinone
(d) la 3-Z-[1-(4-(N-(4-méthylpipérazin-1-ylméthylcarbonyl)-N-méthylamino)anilino)-1-(4-(2-carboxyéthyl)phényl)méthylène]-6-fluoro-2-indolinone
(e) la 3-Z-[1-(4-(N-(2-diméthylaminoéthyl)-N-méthylsulfonylamino)anilino)-1-(4-(2-carboxyéthyl)phényl)méthylène]-6-fluoro-2-indolinone
(f) la 3-Z-[1-(4-(N-(3-diméthylaminopropyl)-N-acétylamino)anilino)-1-(4-(2-carboxyéthyl)phényl)méthylène]-6-fluoro-2-indolinone
(g) la 3-Z-[1-(4-(1-méthylimidazol-2-yl)anilino)-1-(4-(2-carboxyéthyl)phényl)méthylène]-6-fluoro-2-indolinone
(h) la 3-Z-[1-(4-(N-(diméthylaminométhylcarbonyl)-N-méthylamino)anilino)-1-(4-(2-carboxyéthyl)phényl)méthylène]-6-fluoro-2-indolinone
(i) la 3-Z-[1-(4-(N-(2-diméthylaminoéthylcarbonyl)-N-méthylamino)anilino)-1-(4-(2-carboxyéthyl)phényl)méthylène]-6-fluoro-2-indolinone
(j) la 3-Z-[1-(4-pyrrolidin-1-ylméthyl)anilino)-1-(4-(2-carboxyéthyl)phényl)méthylène]-6-fluoro-2-indolinone
(k) la 3-Z-[1-(4-diéthylaminométhyl)anilino)-1-(4-(2-carboxyéthyl)phényl)méthylène]-6-fluoro-2-indolinone
(l) la 3-Z-[1-(4-(2-diméthylaminoéthyl)anilino)-1-(4-(2-carboxyéthyl)phényl)méthylène]-6-chloro-2-indolinone
(m) la 3-Z-[1-(4-diméthylaminométhylanilino)-1-(4-(2-carboxyéthyl)phényl)méthylène]-6-chloro-2-indolinone
(n) la 3-Z-[1-(4-pyrrolidin-1-ylméthyl)anilino)-1-(4-(2-carboxyéthyl)phényl)méthylène]-6-chloro-2-indolinone
(o) la 3-Z-[1-(4-pyrrolidin-1-ylméthyl)anilino)-1-(4-(2-carboxyéthyl)phényl)méthylène]-6-bromo-2-indolinone
(p) la 3-Z-[1-(4-(diméthylaminométhyl)anilino)-1-(4-(2-carboxyéthyl)phényl)méthylène]-6-bromo-2-indolinone, et
(q) la 3-Z-[1-(4-(diéthylaminométhyl)anilino)-1-(4-(2-carboxyéthyl)-méthylène]-6-bromo-2-indolinone,
leurs tautomères, diastéréoisomères, énantiomères, mélanges et sels.

3. Utilisation selon l'une quelconque des revendications 1 ou 2, dans laquelle la maladie est choisie dans le groupe constitué par la fibrose pulmonaire et les maladies pulmonaires ayant une composante fibrogène choisies parmi la fibrose pulmonaire idiopathique, la pneumonie interstitielle à cellules géantes, la sarcoïdose, la mucoviscidose, le syndrome de détresse respiratoire, la fibrose pulmonaire induite par des médicaments, la granulomatose, la silicose, l'amiantose, la sclérodermie généralisée.

4. Utilisation selon l'une quelconque des revendications 1 à 3, dans laquelle la maladie est la fibrose pulmonaire idiopathique.

5. Utilisation selon l'une quelconque des revendications 1 à 4, dans laquelle le traitement est un traitement combiné avec une autre substance pharmacologiquement active choisie dans le groupe constitué par les agents anticholinergiques, les β-2-mimétiques, les stéroïdes, les inhibiteurs de PDE-N, les inhibiteurs de la MAP-kinase p38, les antagonistes de NK₁, les antagonistes de LTD4, les inhibiteurs d'EGFR et les antagonistes de l'endothéline.

6. Composition pharmaceutique comprenant une indolinone substituée de formule I telle que définie dans l'une quelconque des revendications 1 ou 2, éventuellement avec un ou plusieurs vecteurs ou excipients pharmaceutiquement acceptables, pour la prévention ou le traitement d'une maladie fibrogène choisie dans le groupe constitué par la fibrose et le remodelage du tissu pulmonaire dans la bronchopneumopathie chronique obstructive, la fibrose et le remodelage du tissu pulmonaire dans la bronchite chronique, la fibrose et le remodelage du tissu pulmonaire dans l'emphysème, la fibrose pulmonaire et les maladies pulmonaires ayant une composante fibrogène, la fibrose et le remodelage dans l'asthme, le fibrose induite par des rayonnements.

7. Composition pharmaceutique selon la revendication 6, dans laquelle la maladie est choisie dans le groupe constitué par la fibrose pulmonaire et les maladies pulmonaires ayant une composante fibrogène choisies parmi la fibrose pulmonaire idiopathique, la pneumonie interstitielle à cellules géantes, la sarcoïdose, la mucoviscidose, le syndrome de détresse respiratoire, la fibrose pulmonaire induite par des médicaments, la granulomatose, la silicose, l'amiantose, la sclérodermie généralisée.

8. Composition pharmaceutique selon la revendication 7, dans laquelle la maladie est la fibrose pulmonaire idiopathique.

9. Composition pharmaceutique selon l'une quelconque des revendications 6 à 8, dans laquelle le traitement est un traitement combiné avec une autre substance pharmacologiquement active choisie dans le groupe constitué par les agents anticholinergiques, les β-2-mimétiques, les stéroïdes, les inhibiteurs de PDE-IV, les inhibiteurs de la MAP-kinase p38, les antagonistes de NK₁, les antagonistes de LTD4, les inhibiteurs d'EGFR et les antagonistes de l'endothéline, ladite autre substance pharmacologiquement active étant administrée simultanément ou successivement.

10. Composition pharmaceutique contenant une indolinone substituée de formule 1 telle que définie dans l'une quelconque des revendications 1 ou 2 en combinaison avec une autre substance pharmacologiquement active choisie dans le groupe constitué par les agents anticholinergiques, les β-2-mimétiques, les inhibiteurs de PDE-IV, les inhibiteurs de la MAP-kinase p38, les antagonistes de NK₁, les antagonistes de LTD4 et les antagonistes de l'endothéline, éventuellement avec un ou plusieurs vecteurs ou excipients pharmaceutiquement acceptables.
